(19) Europäisches Patentamt
European Patent Office
Office européen des brevets

(11) EP 1 753 758 B1

(12) EUROPEAN PATENT SPECIFICATION

(45) Date of publication and mention of the grant of the patent:
**11.03.2009 Bulletin 2009/11**

(21) Application number: **05755042.8**

(22) Date of filing: **24.05.2005**

(51) Int Cl.:
*C07D 417/06* (2006.01) *C07D 417/04* (2006.01)
*C07D 233/84* (2006.01) *C07D 233/90* (2006.01)
*C07D 409/04* (2006.01) *C07D 413/04* (2006.01)
*C07D 403/04* (2006.01) *C07D 401/04* (2006.01)
*C07C 211/29* (2006.01) *A61K 31/4439* (2006.01)
*A61K 31/501* (2006.01) *A61K 31/502* (2006.01)
*A61K 31/427* (2006.01) *A61K 31/422* (2006.01)
*A61K 31/4245* (2006.01)

(86) International application number:
**PCT/EP2005/052369**

(87) International publication number:
**WO 2005/118578 (15.12.2005 Gazette 2005/50)**

(54) **MERCAPTOIMIDAZOLES AS CCR2 RECEPTOR ANTAGONISTS**

MERCAPTOIMIDAZOLE ALS CCR2-REZEPTORANTAGONISTEN

MERCAPTOIMIDAZOLES EN TANT QU'ANTAGONISTES DU R CEPTEUR CCR2

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **26.05.2004 PCT/EP2004/050933**

(43) Date of publication of application:
**21.02.2007 Bulletin 2007/08**

(73) Proprietor: **Janssen Pharmaceutica NV
2340 Beerse (BE)**

(72) Inventors:
- **BOECKX, Gustaaf Maria
Janssen Pharmaceutica N.V.
B-2340 Beerse (BE)**
- **VAN LOMMEN, Guy R.E.
Janssen Pharmaceutica N.V.
B-2340 Beerse (BE)**
- **DOYON, Julien G.P.-O.
Janssen Pharmaceutica N.V.
B-2340 Beerse (BE)**
- **COESEMANS, Erwin
Janssen Pharmaceutica N.V.
B-2340 Beerse (BE)**

(74) Representative: **Vervoort, Liesbeth et al
Janssen Pharmaceutica N.V.
Turnhoutseweg 30
2340 Beerse (BE)**

(56) References cited:
**WO-A-02/066458 WO-A-03/097633
WO-A-20/04069809 WO-A-20/04069810
US-A1- 2003 149 081**

- **BULLETIN DES SOCIETES CHIMIQUES BELGES, vol. 73, no. 3-4, 1964, pages 181-188, XP009042783**
- **ARCHIV DER PHARMAZIE, vol. 305, no. 12, 1972, pages 891-901, XP009042782 WEINHEIM, GERMANY**
- **ANNALES PHARMACEUTIQUES FRANCAISES, vol. 29, no. 1, 1971, pages 63-70, XP009042723**
- **DATABASE CHEMCATS CHEMICAL ABSTRACTS SERVICE, COLUMBUS, OHIO,US; XP002314406 & 19 January 2004 (2004-01-19), CHEMBRIDGE CORPORATION, 16981 VIA TAZON, SUITE G, SAN DIEGO, CA, 92127 USA**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 753 758 B1

Printed by Jouve, 75001 PARIS (FR)

## Description

The present invention concerns mercaptoimidazole derivatives having CCR2 receptor antagonistic properties. The invention further relates to methods for their preparation and pharmaceutical compositions comprising them. The invention also relates to the use of said compounds for the manufacture of a medicament for the prevention or the treatment of diseases mediated through activation of the CCR2 receptor, in particular the CCR2B receptor.

WO 02/066458 describes 2-thio-substituted imidazole derivatives having immunomodulating and/or inhibiting activity on the release of cytokines, especially TNF-$\alpha$ and IL-$\beta$.
FR 1,487,326 relates to thio-imidazole derivatives useful as analgetic and for its vasodilatation activity.
FR 6,751 M describes thio-imidazole derivatives as sedatives and analgesics.
US 3,850,944 describes 2-mercapto-5-(3-pyridyl)-imidazole derivatives having antiinflammatory activity.
Bull. Soc. Chim.Belg., 73, pp181-188 (1964) describes the synthesis and properties of $\alpha$-arylalkylamines.
Archiv der Pharmazie, 305 (12), pp891-901 (1972) describes the synthesis and pharmacology of N-chloracyl-benzylamines.
Annales pharmaceutiques francaises, 29(1), pp 63-70 (1971) describes the synthesis of imidazole derivatives.
WO 2004/069809 and WO 2004/069810 describe mercaptoimidazole derivatives having CCR2 receptor antagonistic properties.

The compounds of the invention differ from the prior art compounds in structure, in their pharmacological activity and/or pharmacological potency.

One aspect of the present invention relates to a compound of formula

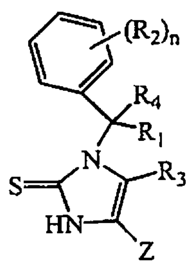

(I)

,

a *N*-oxide, a pharmaceutically acceptable addition salt, a quaternary amine, a polymorphic form or a stereochemically isomeric form thereof, wherein

$R_1$ represents $C_{1-6}$alkyl or $C_{1-6}$alkyloxy$C_{1-6}$alkyl; each $R_2$ independently represents halo;

$R_3$ represents hydrogen, cyano, $C_{1-6}$alkyl optionally substituted with hydroxy or $C_{1-6}$alkyloxy, C(=O)-O-$R_5$, C(=O)-$NR_{6a}R_{6b}$, C(=S)-$NR_{6a}R_{6b}$, $S(=O)_2$-$NR_{6a}R_{6b}$ or C(=O)-$R_7$;

$R_4$ represents hydrogen;

$R_5$ represents hydrogen, $C_{1-6}$alkyl, hydroxy$C_{1-6}$alkyl, $C_{2-6}$alkenyl, $C_{2-6}$alkynyl, polyhalo$C_{1-6}$alkyl, $C_{1-6}$alkyloxy$C_{1-6}$alkyl, amino$C_{1-6}$alkyl, mono-or di($C_{1-4}$alkyl)amino$C_{1-6}$alkyl, aminocarbonyl$C_{1-6}$alkyl, mono-or di($C_{1-4}$alkyl)aminocarbonyl$C_{1-6}$alkyl or aryl;

$R_{6a}$ and $R_{6b}$ each independently represent hydrogen, $C_{1-6}$alkyl, amino, mono-or di($C_{1-4}$alkyl)amino, arylNH-, amino$C_{1-6}$alkyl, mono-or di($C_{1-4}$alkyl)amino-$C_{1-6}$alkyl, $C_{1-6}$alkylcarbonylamino, aminocarbonylamino, $C_{1-6}$alkyloxy, carbonylamino or hydroxy$C_{1-6}$alkyl; or

$R_{6a}$ and $R_{6b}$ taken together with the nitrogen to which they are attached form pyrrolidinyl, imidazolidinyl, pyrazolidinyl, piperidinyl, piperazinyl, morpholinyl, thiomorpholinyl or piperazinyl substituted with $C_{1-6}$alkyl;

$R_7$ represents hydrogen, $C_{1-6}$alkyl, hydroxy$C_{1-6}$alkyl, $C_{2-6}$alkenyl, $C_{2-6}$alkynyl, polyhalo$C_{1-6}$alkyl, $C_{1-6}$alkyloxy$C_{1-6}$alkyl, amino$C_{1-6}$alkyl, mono-or di($C_{1-4}$alkyl)amino$C_{1-6}$alkyl, aminocarbonyl$C_{1-6}$alkyl, mono-or di($C_{1-4}$alkyl)aminocarbonyl$C_{1-6}$alkyl, aryl or heteroaryl;

Z represents a cyclic ring system selected from

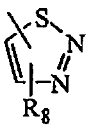 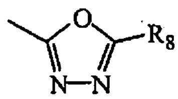 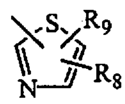 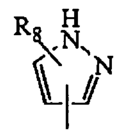 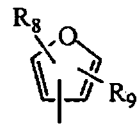 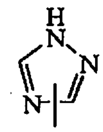

(a-1) (a-2) (a-3) (a-4) (a-5) (a-6)

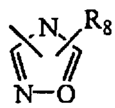 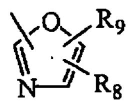 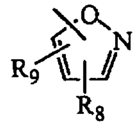 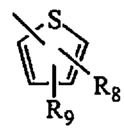 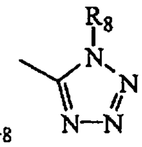 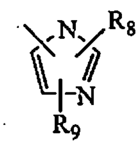

(a-7) (a-8) (a-9) (a-10) (a-11) (a-12)

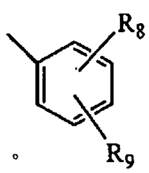 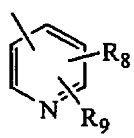 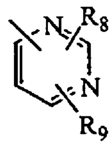 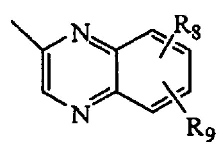 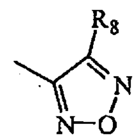

(a-13) (a-14) (a-15) (a-16) (a-17)

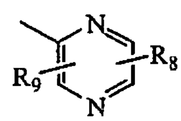

(a-18)

each $R_8$ independently represents hydrogen, halo, $C_{1-6}$alkyl, $C_{1-6}$alkyloxy, polyhalo$C_{1-6}$alkyl, polyhalo$C_{1-6}$alkyloxy, cyano, aminocarbonyl, mono-or di($C_{1-4}$alkyl)aminocarbonyl, amino, mono-or di ($C_{1-4}$alkyl)amino,
hydroxy$C_{1-6}$alkylamino, aryl, aryloxy, piperidinyl, piperidinylamino, morpholinyl, piperazinyl or nitro;
each $R_9$ independently represents hydrogen, halo or $C_{1-6}$alkyl;

n is 1, 2, 3, 4 or 5;

aryl represents phenyl or phenyl substituted with one, two, three, four or five substituents each independently selected from halo, $C_{1-6}$alkyl, $C_{1-6}$alkyloxy, polyhalo$C_{1-6}$alkyl, polyhalo$C_{1-6}$alkyloxy, cyano, aminocarbonyl, mono-or di($C_{1-4}$alkyl)aminocarbonyl, amino, mono-or di($C_{1-4}$alkyl)amino, phenyloxy or nitro;

heteroaryl represents furanyl, thienyl, pyrrolyl, oxazolyl, thiazolyl, imidazolyl, pyrazolyl, isoxazolyl, isothiazolyl, oxadiazolyl, triazolyl, thiadiazolyl, pyridyl, pyridazinyl, pyrimidinyl, pyrazinyl, each of said heterocycles optionally being substituted with one or two substituents each independently selected from halo, $C_{1-6}$alkyl, $C_{1-6}$alkyloxy, polyhalo$C_{1-6}$alkyl, polyhalo$C_{1-6}$alkyloxy, cyano, aminocarbonyl, mono-or di($C_{1-4}$alkyl)aminocarbonyl, amino, mono-or di($C_{1-4}$alkyl)amino or nitro.

The present invention also relates to the use of a compound for the manufacture of a medicament for preventing or treating diseases mediated through activation of the CCR2 receptor, in particular for preventing or treating inflammatory diseases, wherein said compound is a compound of formula (I)

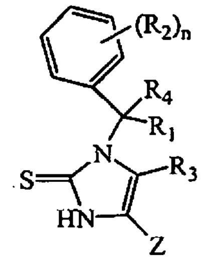

(I)

,

a *N*-oxide, a pharmaceutically acceptable addition salt, a quaternary amine, a polymorphic form or a stereochemically isomeric form thereof, wherein

| | |
|---|---|
| $R_1$ | represents $C_{1-6}$alkyl or $C_{1-6}$alkyloxy$C_{1-6}$alkyl; |
| each $R_2$ | independently represents halo; |
| $R_3$ | represents hydrogen, cyano, $C_{1-6}$alkyl optionally substituted with hydroxy or $C_{1-6}$alkyloxy, C(=O)-O-$R_5$, C(=O)-$NR_{6a}R_{6b}$, C(=S)-$NR_{6a}R_{6b}$, $S(=O)_2$-$NR_{6a}R_{6b}$ or C(=O)-$R_7$; |
| $R_4$ | represents hydrogen; |
| $R_5$ | represents hydrogen, $C_{1-6}$alkyl, hydroxy$C_{1-6}$alkyl, $C_{2-6}$alkenyl, $C_{2-6}$alkynyl, polyhalo$C_{1-6}$alkyl, $C_{1-6}$alkyloxy$C_{1-6}$alkyl, amino$C_{1-6}$alkyl, mono-or di($C_{1-4}$alkyl)amino$C_{1-6}$alkyl, aminocarbonyl$C_{1-6}$alkyl, mono-or di($C_{1-4}$alkyl)aminocarbonyl$C_{1-6}$alkyl or aryl; |
| $R_{6a}$ and $R_{6b}$ | each independently represent hydrogen, $C_{1-6}$alkyl, amino, mono-or di($C_{1-4}$alkyl)amino, arylNH-, amino$C_{1-6}$alkyl, mono-or di($C_{1-4}$alkyl)amino $C_{1-6}$alkyl, $C_{1-6}$alkylcarbonylamino, aminocarbonylamino, $C_{1-6}$alkyloxy, carbonylamino or hydroxy$C_{1-6}$alkyl; or |
| $R_{6a}$ and $R_{6b}$ | taken together with the nitrogen to which they are attached form pyrrolidinyl, imidazolidinyl, pyrazolidinyl, piperidinyl, piperazinyl, morpholinyl, thiomorpholinyl or piperazinyl substituted with $C_{1-6}$alkyl; |
| $R_7$ | represents hydrogen, $C_{1-6}$alkyl, hydroxy$C_{1-6}$alkyl, $C_{2-6}$alkenyl, $C_{2-6}$alkynyl, polyhalo$C_{1-6}$alkyl, $C_{1-6}$alkyloxy$C_{1-6}$alkyl, amino$C_{1-6}$alkyl, mono-or di($C_{1-4}$alkyl)amino$C_{1-6}$alkyl, aminocarbonyl$C_{1-6}$alkyl, mono-or di($C_{1-4}$alkyl)aminocarbonyl$C_{1-6}$alkyl, aryl or heteroaryl; |
| Z | represents a cyclic ring system selected from |

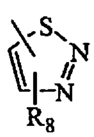
(a-1)

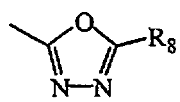
(a-2)

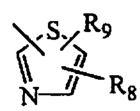
(a-3)

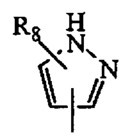
(a-4)

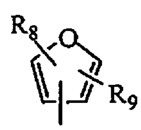
(a-5)

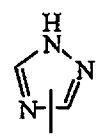
(a-6)

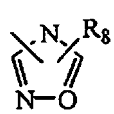
(a-7)

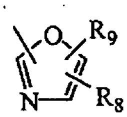
(a-8)

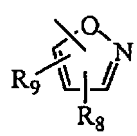
(a-9)

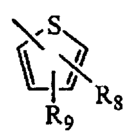
(a-10)

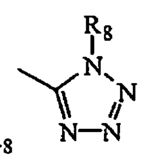
(a-11)

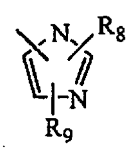
(a-12)

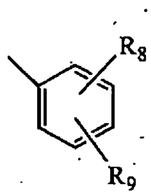

(a-13)

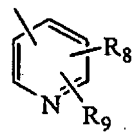

(a-14)

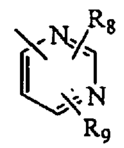

(a-15)

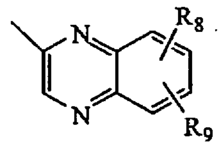

(a-16)

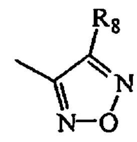

(a-17)

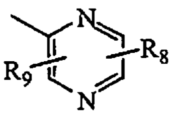

(a-18)

each $R_8$ independently represents hydrogen, halo, $C_{1-6}$alkyl, $C_{1-6}$alkyloxy, polyhalo$C_{1-6}$alkyl, polyhalo$C_{1-6}$alkyloxy, cyano, aminocarbonyl, mono-or di($C_{1-4}$alkyl)aminocarbonyl, amino, mono-or di ($C_{1-4}$alkyl)amino,
hydroxy$C_{1-6}$alkylamino, aryl, aryloxy, piperidinyl, piperidinylamino, morpholinyl, piperazinyl or nitro;
each $R_9$ independently represents hydrogen, halo or $C_{1-6}$alkyl;

n is 1, 2, 3, 4 or 5;

aryl represents phenyl or phenyl substituted with one, two, three, four or five substituents each independently selected from halo, $C_{1-6}$alkyl, $C_{1-6}$alkyloxy, polyhalo$C_{1-6}$alkyl, polyhalo$C_{1-6}$alkyloxy, cyano, aminocarbonyl, mono-or di($C_{1-4}$alkyl)aminocarbonyl, amino, mono-or di($C_{1-4}$alkyl)amino, phenyloxy or nitro;

heteroaryl represents furanyl, thienyl, pyrrolyl, oxazolyl, thiazolyl, imidazolyl, pyrazolyl, isoxazolyl, isothiazolyl, oxadiazolyl, triazolyl, thiadiazolyl, pyridyl, pyridazinyl, pyrimidinyl, pyrazinyl, each of said heterocycles optionally being substituted with one or two substituents each independently selected from halo, $C_{1-6}$alkyl, $C_{1-6}$alkyloxy, polyhalo$C_{1-6}$alkyl, polyhalo$C_{1-6}$alkyloxy, cyano, aminocarbonyl, mono-or di($C_{1-4}$alkyl)aminocarbonyl, amino, mono-or di($C_{1-4}$alkyl)amino or nitro.

As used hereinbefore or hereinafter $C_{1-4}$alkyl as a group or part of a group defines straight or branched chain saturated hydrocarbon radicals having from 1 to 4 carbon atoms such as methyl, ethyl, propyl, 1-methylethyl, butyl; $C_{1-6}$alkyl as a group or part of a group defines straight or branched chain saturated hydrocarbon radicals having from 1 to 6 carbon atoms such as the group defined for $C_{1-4}$alkyl and pentyl, hexyl, 2-methylbutyl and the like; $C_{3-7}$cycloalkyl is generic to cyclopropyl, cyclobutyl, cyclopentyl, cyclohexyl and cycloheptyl; $C_{2-6}$alkenyl defines straight and branched chain hydrocarbon radicals having from 2 to 6 carbon atoms containing a double bond such as ethenyl, propenyl, butenyl, pentenyl, hexenyl and the like; $C_{2-6}$alkynyl defines straight and branched chain hydrocarbon radicals having from 2 to 6 carbon atoms containing a triple bond such as ethynyl, propynyl, butynyl, pentynyl, hexynyl and the like.

As used hereinbefore, the term (=O) forms a carbonyl moiety when attached to a carbon atom, a sulfoxide moiety when attached to a sulfur atom and a sulfonyl moiety when two of said terms are attached to a sulfur atom.

The term halo is generic to fluoro, chloro, bromo and iodo. As used in the foregoing or hereinafter, polyhalomethyl as a group or part of a group is defined as mono- or polyhalosubstituted methyl, in particular methyl with one or more fluoro atoms, for example, difluoromethyl or trifluoromethyl; polyhalo$C_{1-6}$alkyl as a group or part of a group is defined as mono- or polyhalosubstituted $C_{1-6}$alkyl, for example, the groups defined in polyhalomethyl, 1,1-difluoro-ethyl and the like. In case more than one halogen atoms are attached to an alkyl group within the definition of polyhalomethyl or polyhalo$C_{1-6}$alkyl, they may be the same or different.

The term heteroaryl in the definition of $R_7$ is meant to include all the possible isomeric forms of the heterocycles, for instance, pyrrolyl comprises 1*H*-pyrrolyl and 2*H*-pyrrolyl.

The aryl, heteroaryl, heterocyclic ring systems or cyclic ring systems listed in the definitions of the substituents of the compounds of formula (I) (see for instance $R_5$, $R_7$ and Z) as mentioned hereinabove or hereinafter may be attached to the remainder of the molecule of formula (I) through any ring carbon or heteroatom as appropriate, if not otherwise specified. Thus, for example, when heteroaryl is imidazolyl, it may be 1-imidazolyl, 2-imidazolyl, 4-imidazolyl and the like.

When any variable (eg. $R_{6a}$, $R_{6b}$) occurs more than one time in any constituent, each definition is independent.

Lines drawn from substituents into ring systems indicate that the bond may be attached to any of the suitable

ring atoms. When the lines are drawn into bicyclic ring systems, it indicates that the bond may be attached to any of the suitable ring atoms of any one of the two cycles of the bicyclic ring system.

For therapeutic use, salts of the compounds of formula (I) are those wherein the counterion is pharmaceutically acceptable. However, salts of acids and bases which are non-pharmaceutically acceptable may also find use, for example, in the preparation or purification of a pharmaceutically acceptable compound. All salts, whether pharmaceutically acceptable or not are included within the ambit of the present invention.

The pharmaceutically acceptable addition salts as mentioned hereinabove are meant to comprise the therapeutically active non-toxic acid addition salt forms which the compounds of formula (I) are able to form. The latter can conveniently be obtained by treating the base form with such appropriate acids as inorganic acids, for example, hydrohalic acids, e.g. hydrochloric, hydrobromic and the like; sulfuric acid; nitric acid; phosphoric acid and the like; or organic acids, for example, acetic, propanoic, hydroxyacetic, 2-hydroxypropanoic, 2-oxopropanoic, oxalic, malonic, succinic, maleic, fumaric, malic, tartaric, 2-hydroxy-1,2,3-propanetricarboxylic, methanesulfonic, ethanesulfonic, benzenesulfonic, 4-methylbenzenesulfonic, cyclobexanesulfamic, 2-hydroxybenzoic, 4-amino-2-hydroxybenzoic and the like acids. Conversely the salt form can be converted by treatment with alkali into the free base form.

The compounds of formula (I) containing acidic protons may be converted into their therapeutically active non-toxic metal or amine addition salt forms by treatment with appropriate organic and inorganic bases. Appropriate base salt forms comprise, for example, the ammonium salts, the alkali and earth alkaline metal salts, e.g. the lithium, sodium, potassium, magnesium, calcium salts and the like, salts with organic bases, e.g. primary, secondary and tertiary aliphatic and aromatic amines such as methylamine, ethylamine, propylamine, isopropylamine, the four butylamine isomers, dimethylamine, diethylamine, diethanolamine, dipropylamine, diisopropylamine, di-n-butylamine, pyrrolidine, piperidine, morpholine, trimethylamine, triethylamine, tripropylamine, quinuclidine, pyridine, quinoline and isoquinoline, the benzathine, *N*-methyl-D-glucamine, 2-amino-2-(hydroxymethyl)-1,3-propanediol, hydrabamine salts, and salts with amino acids such as, for example, arginine, lysine and the like. Conversely the salt form can be converted by treatment with acid into the free acid form.

The term addition salt also comprises the hydrates and solvent addition forms which the compounds of formula (I) are able to form. Examples of such forms are e.g. hydrates, alcoholates and the like.

The term "quaternary amine" as used hereinbefore defines the quaternary ammonium salts which the compounds of formula (I) are able to form by reaction between a basic nitrogen of a compound of formula (I) and an appropriate quaternizing agent, such as, for example, an optionally substituted alkylhalide, arylhalide or arylalkylhalide, e.g. methyliodide or benzyliodide. Other reactants with good leaving groups may also be used, such as alkyl trifluoromethanesulfonates, alkyl methanesulfonates, and alkyl *p*-toluenesulfonates. A quaternary amine has a positively charged nitrogen. Pharmaceutically acceptable counterions include chloro, bromo, iodo, trifluoroacetate and acetate. The counterion of choice can be introduced using ion exchange resins.

The *N*-oxide forms of the present compounds are meant to comprise the compounds of formula (I) wherein one or several tertiary nitrogen atoms are oxidized to the so-called *N*-oxide.

It will be appreciated that some of the compounds of formula (I) and their *N*-oxides, addition salts, quaternary amines, polymorphic forms or stereochemically isomeric forms may contain one or more centers of chirality and exist as stereochemically isomeric forms.

The term "stereochemically isomeric forms" as used hereinbefore or hereinafter defines all the possible stereoisomeric forms which the compounds of formula (I), and their *N*-oxides, addition salts, quaternary amines, polymorphic forms or physiologically functional derivatives may possess. Unless otherwise mentioned or indicated, the chemical designation of compounds denotes the mixture of all possible stereochemically isomeric forms, said mixtures containing all diastereomers and enantiomers of the basic molecular structure as well as each of the individual isomeric forms of formula (I) and their *N*-oxides, salts, solvates, quaternary amines or polymorphic forms substantially free, *i.e*. associated with less than 10%, preferably less than 5%, in particular less than 2% and most preferably less than 1% of the other isomers. Thus, when a compound of formula (I) is for instance specified as (E), this means that the compound is substantially free of the (Z) isomer.

In particular, stereogenic centers may have the R- or S-configuration; substituents on bivalent cyclic (partially) saturated radicals may have either the *cis*- or *trans*-configuration. Compounds encompassing double bonds can have an E (entgegen) or Z (zusammen) -stereochemistry at said double bond. The terms cis, trans, R, S, E and Z are well known to a person skilled in the art.

Stereochemically isomeric forms of the compounds of formula (I) are obviously intended to be embraced within the scope of this invention.

Polymorphic forms of the present compounds also fall within the ambit of the present invention. The term "polymorphic forms" as used hereinbefore or hereinafter defines all possible crystalline arrangement of a particular compound. A polymorphic form of a compound is the same chemical entity, but in a different crystalline arrangement. The term "polymorphic form" is well-known to a person skilled in the art.

Polymorphic forms of pharmaceutical compounds may be of interest to those involved in the development of

a suitable dosage form because if the polymorphic form is not held constant during clinical and stability studies, the exact dosage used or measured may not be comparable from one lot to the next. Once a pharmaceutical compound is produced for use, it is important to recognize the polymorphic form delivered in each dosage form to assure that the production process use the same form and that the same amount of drug is included in each dosage. Therefore, it is imperative to assure that either a single polymorphic form or some known combination of polymorphic forms is present. In addition, certain polymorphic forms may exhibit enhanced thermodynamic stability and may be more suitable than other polymorphic forms for inclusion in pharmaceutical formulations.

Some of the compounds of formula (1) may also exist in their tautomeric form. Such forms although not explicitly indicated in the above formula (I) are intended to be included within the scope of the present invention. For instance, it is intended that formula (I) includes the tautomeric form of

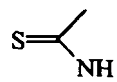

being

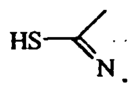

Thus, the compounds of the present invention include compounds of formula

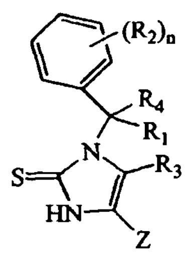

and

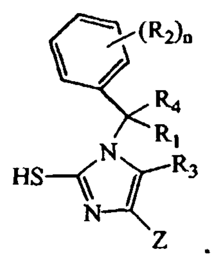

Whenever used hereinafter, the term "compounds of formula (I)" is meant to also include their *N*-oxide forms, their addition salts, their quaternary amines, their polymorphic forms or their stereochemically isomeric forms. Of special interest are those compounds of formula (I) which are stereochemically pure.

Whenever used hereinbefore or hereinafter that substituents can be selected each independently out of a list of numerous definitions, such as for example for $R_{6a}$ or $R_{6b}$, all possible combinations are intended which are chemically possible.

A first interesting embodiment of the present invention relates to a compound of formula

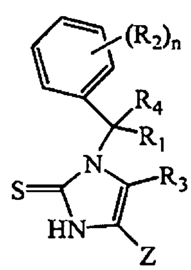

(I)

,

a *N*-oxide, a pharmaceutically acceptable addition salt, a quaternary amine or a stereochemically isomeric form thereof, wherein

| | |
|---|---|
| $R_1$ | represents $C_{1-6}$alkyl or $C_{1-6}$alkyloxy$C_{1-6}$alkyl; |
| each $R_2$ | independently represents halo; |

$R_3$ represents hydrogen, cyano, $C_{1-6}$alkyl optionally substituted with hydroxy or $C_{1-6}$alkyloxy, C(=O)-O-$R_5$, C(=O)-$NR_{6a}R_{6b}$, C(=S)-$NR_{6a}R_{6b}$, $S(=O)_2$-$NR_{6a}R_{6b}$ or C(=O)-$R_7$;

$R_4$ represents hydrogen;

$R_5$ represents hydrogen, $C_{1-6}$alkyl, hydroxy$C_{1-6}$alkyl, $C_{2-6}$alkenyl, $C_{2-6}$alkynyl, polyhalo$C_{1-6}$alkyl, $C_{1-6}$alkyloxy$C_{1-6}$alkyl, amino$C_{1-6}$alkyl, mono-or di($C_{1-4}$alkyl)amino$C_{1-6}$alkyl, aminocarbonyl$C_{1-6}$alkyl, mono-or di($C_{1-4}$alkyl)aminocarbonyl$C_{1-6}$alkyl or aryl;

$R_{6a}$ and $R_{6b}$ each independently represent hydrogen, $C_{1-6}$alkyl, amino, mono-or di($C_{1-4}$alkyl)amino, arylNH-, amino$C_{1-6}$alkyl, mono-or di($C_{1-4}$alkyl)amino-$C_{1-6}$alkyl, $C_{1-6}$alkylcarbonylamino, aminocarbonylamino, $C_{1-6}$alkyloxy, carbonylamino or hydroxy$C_{1-6}$alkyl; or

$R_{6a}$ and $R_{6b}$ taken together with the nitrogen to which they are attached form pyrrolidinyl, imidazolidinyl, pyrazolidinyl, piperidinyl, piperazinyl, morpholinyl, thiomorpholinyl or piperazinyl substituted with $C_{1-6}$alkyl;

$R_7$ represents hydrogen, $C_{1-6}$alkyl, hydroxy$C_{1-6}$alkyl, $C_{2-6}$alkenyl, $C_{2-6}$alkynyl, polyhalo$C_{1-6}$alkyl, $C_{1-6}$alkyloxy$C_{1-6}$alkyl, amino$C_{1-6}$alkyl, mono-or di($C_{1-4}$alkyl)amino$C_{1-6}$alkyl, aminocarbonyl$C_{1-6}$alkyl, mono-or di($C_{1-4}$alkyl)aminocarbonyl$C_{1-6}$alkyl, aryl or heteroaryl;

Z represents a cyclic ring system selected from

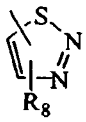
(a-1)

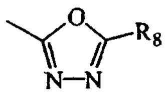
(a-2)

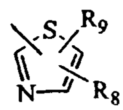
(a-3)

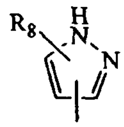
(a-4)

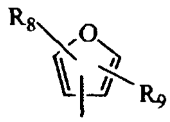
(a-5)

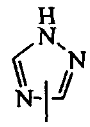
(a-6)

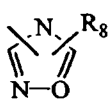
(a-7)

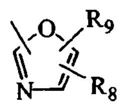
(a-8)

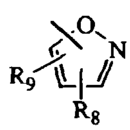
(a-9)

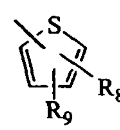
(a-10)

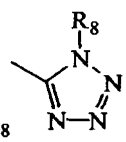
(a-11)

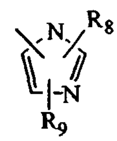
(a-12)

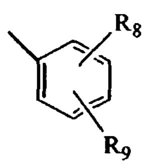
(a-13)

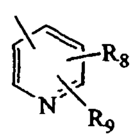
(a-14)

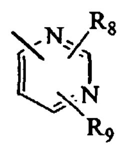
(a-15)

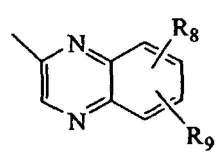
(a-16)

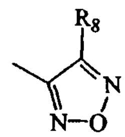
(a-17)

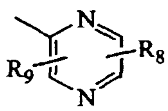
(a-18)

each $R_8$ independently represents hydrogen, halo, $C_{1-6}$alkyl, $C_{1-6}$alkyloxy, polyhalo$C_{1-6}$alkyl, polyhalo$C_{1-6}$alkyloxy, cyano, aminocarbonyl, mono-or di($C_{1-4}$alkyl)aminocarbonyl, amino, mono-or di($C_{1-4}$alkyl)amino, hydroxy$C_{1-6}$alkylamino, aryl, aryloxy, piperidinyl, piperidinylamino, morpholinyl, piperazinyl or nitro;

each $R_9$ independently represents hydrogen, halo or $C_{1-6}$alkyl;

n is 1, 2, 3, 4 or 5;

aryl represents phenyl or phenyl substituted with one, two, three, four or five substituents each independently selected from halo, $C_{1-6}$alkyl, $C_{1-6}$alkyloxy, polyhalo$C_{1-6}$alkyl, polyhalo$C_{1-6}$alkyloxy, cyano, aminocarbonyl, mono-or di($C_{1-4}$alkyl)aminocarbonyl, amino, mono-or di($C_{1-4}$alkyl)amino, phenyloxy or nitro;

heteroaryl represents furanyl, thienyl, pyrrolyl, oxazolyl, thiazolyl, imidazolyl, pyrazolyl, isoxazolyl, isothiazolyl, oxadiazolyl, triazolyl, thiadiazolyl, pyridyl, pyridazinyl, pyrimidinyl, pyrazinyl, each of said heterocycles optionally being substituted with one or two substituents each independently selected from halo, $C_{1-6}$alkyl, $C_{1-6}$alkyloxy, polyhalo$C_{1-6}$alkyl, polyhalo$C_{1-6}$alkyloxy, cyano, aminocarbonyl, mono-or di($C_{1-4}$alkyl)aminocarbonyl, amino, mono-or di($C_{1-4}$alkyl)amino or nitro.

A second interesting embodiment of the present invention relates to a compound of formula

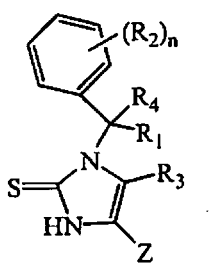 (I)

,

wherein

$R_1$ represents $C_{1-6}$alkyl or $C_{1-6}$alkyloxy$C_{1-6}$alkyl;

each $R_2$ independently represents halo;

$R_3$ represents hydrogen, cyano, $C_{1-6}$alkyl optionally substituted with hydroxy or $C_{1-6}$alkyloxy, C(=O)-O-$R_5$, C(=O)-$NR_{6a}R_{6b}$, C(=S)-$NR_{6a}R_{6b}$, $S(=O)_2$-$NR_{6a}R_{6b}$ or C(=O)-$R_7$;

$R_4$ represents hydrogen;

$R_5$ represents hydrogen, $C_{1-6}$alkyl, hydroxy$C_{1-6}$alkyl, $C_{2-6}$alkenyl, $C_{2-6}$alkynyl, polyhalo$C_{1-6}$alkyl, $C_{1-6}$alkyloxy$C_{1-6}$alkyl, amino$C_{1-6}$alkyl, mono-or di($C_{1-4}$alkyl)amino$C_{1-6}$alkyl, aminocarbonyl$C_{1-6}$alkyl, mono-or di($C_{1-4}$alkyl)aminocarbonyl$C_{1-6}$alkyl or aryl;

$R_{6a}$ and $R_{6b}$ each independently represent hydrogen, $C_{1-6}$alkyl, amino, mono-or di($C_{1-4}$alkyl)amino, arylNH-, amino$C_{1-6}$alkyl, mono-or di($C_{1-4}$alkyl)amino-$C_{1-6}$alkyl, $C_{1-6}$alkylcarbonylamino, aminocarbonylamino, $C_{1-6}$alkyloxy, carbonylamino or hydroxy$C_{1-6}$alkyl; or

$R_{6a}$ and $R_{6b}$ taken together with the nitrogen to which they are attached form pyrrolidinyl, imidazolidinyl, pyrazolidinyl, piperidinyl, piperazinyl, morpholinyl, thiomorpholinyl or piperazinyl substituted with $C_{1-6}$alkyl;

$R_7$ represents hydrogen, $C_{1-6}$alkyl, hydroxy$C_{1-6}$alkyl, $C_{2-6}$alkenyl, $C_{2-6}$alkynyl, polyhalo$C_{1-6}$alkyl, $C_{1-6}$alkyloxy$C_{1-6}$alkyl, amino$C_{1-6}$alkyl, mono-or di($C_{1-4}$alkyl)amino$C_{1-6}$alkyl, aminocarbonyl$C_{1-6}$alkyl, mono-or di($C_{1-4}$alkyl)aminocarbonyl$C_{1-6}$alkyl, aryl or heteroaryl;

Z represents a cyclic ring system selected from

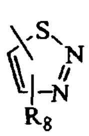
(a-1)

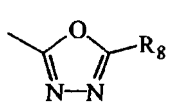
(a-2)

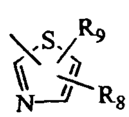
(a-3)

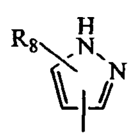
(a-4)

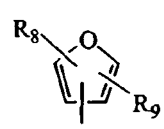
(a-5)

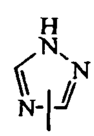
(a-6)

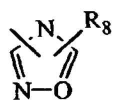

(a-7)

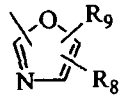

(a-8)

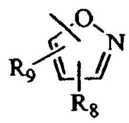

(a-9)

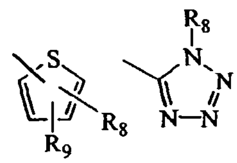

(a-10) (a-11)

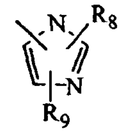

(a-12)

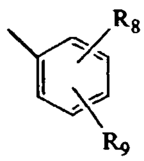

(a-13)

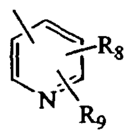

(a-14)

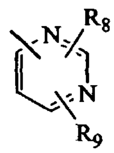

(a-15)

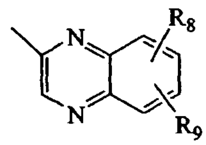

(a-16)

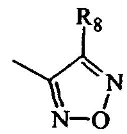

(a-17)

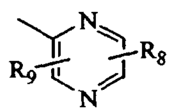

(a-18)

each $R_8$ independently represents hydrogen, halo, $C_{1-6}$alkyl, $C_{1-6}$alkyloxy, polyhalo$C_{1-6}$alkyl, polyhalo$C_{1-6}$alkyloxy, cyano, aminocarbonyl, mono-or di($C_{1-4}$alkyl)aminocarbonyl, amino, mono-or di ($C_{1-4}$alkyl)amino, hydroxy$C_{1-6}$alkylamino, aryl, aryloxy, piperidinyl, piperidinylamino, morpholinyl, piperazinyl or nitro;

each $R_9$ independently represents hydrogen, halo or $C_{1-6}$alkyl;

n is 1, 2, 3,4 or 5;

aryl represents phenyl or phenyl substituted with one, two, three, four or five substituents each independently selected from halo, $C_{1-6}$alkyl, $C_{1-6}$alkyloxy, polyhalo$C_{1-6}$alkyl, polyhalo$C_{1-6}$alkyloxy, cyano, aminocarbonyl, mono-or di($C_{1-4}$alkyl)aminocarbonyl, amino, mono-or di($C_{1-4}$alkyl)amino, phenyloxy or nitro;

heteroaryl represents furanyl, thienyl, pyrrolyl, oxazolyl, thiazolyl, imidazolyl, pyrazolyl, isoxazolyl, isothiazolyl, oxadiazolyl, triazolyl, thiadiazolyl, pyridyl, pyridazinyl, pyrimidinyl, pyrazinyl, each of said heterocycles optionally being substituted with one or two substituents each independently selected from halo, $C_{1-6}$alkyl, $C_{1-6}$alkyloxy, polyhalo$C_{1-6}$alkyl, polyhalo$C_{1-6}$alkyloxy, cyano, aminocarbonyl, mono-or di($C_{1-4}$alkyl)aminocarbonyl, amino, mono-or di($C_{1-4}$alkyl)amino or nitro.

A third interesting embodiment are the compounds of formula (I) or any subgroup thereof as mentioned hereinbefore as interesting embodiment wherein $R_3$ represents hydrogen, cyano, C(=O)-O-$R_5$, C(=O)-$NR_{6a}R_{6b}$, C(=S)-$NR_{6a}R_{6b}$, S(=O)$_2$-$NR_{6a}R_{6b}$ or C(=O)-$R_7$.

A fourth interesting embodiment are the compounds of formula (I) or any subgroup thereof as mentioned hereinbefore as interesting embodiment wherein $R_3$ represents cyano, $C_{1-6}$alkyl optionally substituted with hydroxy or $C_{1-6}$alkyloxy, C(=O)-O-$R_5$, C(=O)-$NR_{6a}R_{6b}$, C(=S)-$NR_{6a}R_{6b}$, S(=O)$_2$-$NR_{6a}R_{6b}$ or C(=O)-$R_7$. A fifth interesting embodiment are the compounds of formula (I) or any subgroup thereof as mentioned hereinbefore as interesting embodiment wherein $R_3$ represents cyano, C(=O)-O-$R_5$, C(=O)-$NR_{6a}R_{6b}$, C(=S)-$NR_{6a}R_{6b}$, S(=O)$_2$-$NR_{6a}R_{6b}$ or C(=O)-$R_7$.

A sixth interesting embodiment are the compounds of formula (I) or any subgroup thereof as mentioned hereinbefore as interesting embodiment wherein $R_3$ represents hydrogen, cyano, C(=O)-O-$R_5$, C(=O)-$NR_{6a}R_{6b}$ or C(=O)-$R_7$; preferably C(=O)-O-$R_5$; more preferably C(=O)-O-$C_{1-6}$alkyl; most preferred C(=O)-O-$CH_3$.

A seventh interesting embodiment are the compounds of formula (I) or any subgroup thereof as mentioned hereinbefore as interesting embodiment wherein Z is other than 3-pyridyl.

An eight interesting embodiment are the compounds of formula (I) or any subgroup thereof as mentioned

hereinbefore as interesting embodiment wherein Z is a cyclic ring system selected from (a-1), (a-2), (a-3), (a-4), (a-5), (a-6), (a-7), (a-9), (a-10), (a-11), (a-12), (a-13), (a-14), (a-15) or (a-16) as defined hereinabove; preferably a cyclic ring system selected from (a-1), (a-2), (a-3), (a-4), (a-9), (a-10), (a-12), (a-13), (a-14), (a-16) or (a-18); more preferably a cyclic ring system selected from (a-1), (a-2), (a-3), (a-9), (a-10), (a-12), (a-13), (a-14) or (a-16); even more preferably a cyclic ring system selected from (a-1), (a-2), (a-9), (a-10) or (a-13); most preferred a cyclic ring system of formula (a-9).

A ninth interesting embodiment are the compounds of formula (I) or any subgroup thereof as mentioned hereinbefore as interesting embodiment wherein Z is a cyclic ring system selected from (a-2), (a-7) or (a-9) or a cyclic ring system selected from (a-2), (a-8) or (a-10); preferably wherein Z is a cyclic ring system selected from (a-2), (a-7) or (a-9); more preferably wherein Z is a cyclic ring system selected from (a-2), (a-7) or (a-9) and wherein $R_3$ represents C(=O)-O-$R_5$.

A tenth interesting embodiment are the compounds of formula (I) or any subgroup thereof as mentioned hereinbefore as interesting embodiment wherein n is 2 or 3; preferably wherein n is 2.

An eleventh interesting embodiment are the compounds of formula (I) or any subgroup thereof as mentioned hereinbefore as interesting embodiment wherein n is 2 and said two $R^2$ substituents are placed in meta and para postion.

A twelfth interesting embodiment are the compounds of formula (I) or any subgroup thereof as mentioned hereinbefore as interesting embodiment wherein $R_2$ represents chloro or fluoro, more in particular fluoro.

A thirteenth interesting embodiment are the compounds of formula (I) or any subgroup thereof as mentioned hereinbefore as interesting embodiment wherein $R_1$ is methyl, ethyl, *n*-propyl, methoxymethyl; $R_1$ is $C_{1-6}$alkyl or $C_{1-6}$alkyloxy$C_{1-6}$alkyl, in particular methyl, ethyl, propyl, methoxymethyl, more in particular methyl, ethyl, *n*-propyl or methoxymethyl; more preferably $R_1$ is $C_{1-6}$alkyl, in particular methyl, ethyl and propyl, more in particular methyl, ethyl or *n*-propyl; most preferred $R_1$ is ethyl.

A fourteenth interesting embodiment are the compounds of formula (I) or any subgroup thereof as mentioned hereinbefore as interesting embodiment which are stereochemically pure.

A fifteenth interesting embodiment are the compounds of formula (I) or any subgroup thereof as mentioned hereinbefore as interesting embodiment wherein the carbon atom carrying the $R_1$ and $R_4$ substituent has the (S) configuration, i.e. a compound of formula (I')

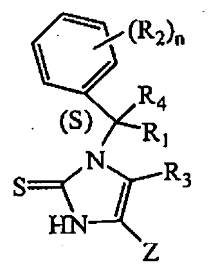

(I')

,

a *N*-oxide, a pharmaceutically acceptable addition salt, a quaternary amine, a polymorphic form or a stereochemically isomeric form thereof.

Also interesting compounds are those compounds of formula (I) wherein one or more, preferably all of the following restrictions apply:

a) $R_1$ represents $C_{1-6}$alkyl or $C_{1-6}$alkyloxy$C_{1-6}$alkyl, especially methyl, ethyl, propyl or methoxymethyl;
b) $R_2$ represents halo, e.g. chloro or fluoro;
c) $R_3$ represents hydrogen, cyano, C(=O)-O-$R_5$, C(=O)-N$R_{6a}R_{6b}$ or C(=O)-$R_7$;
d) Z represents a ring system selected from (a-1), (a-2), (a-3), (a-4), (a-5), (a-6), (a-7), (a-9), (a-10), (a-11), (a-12), (a-13), (a-14), (a-15) or (a-16);
e) $R_4$ represents hydrogen;
f) n is 2 or 3.

Also interesting compounds are those compounds of formula (I) wherein one or more of the following restrictions apply:

a) $R_1$ represents $C_{1-6}$alkyl or $C_{1-6}$alkyloxy$C_{1-6}$alkyl, especially methyl, ethyl, propyl or methoxymethyl;
b) $R_2$ represents halo, especially chloro or fluoro;
c) $R_3$ represents hydrogen; cyano; C(=O)-O-$R_5$ wherein $R_5$ is preferably hydrogen, $C_{1-6}$alkyl or $C_{1-6}$alkyloxy$C_{1-6}$alkyl; C(=O)-N$R_{6a}R_{6b}$ wherein $R_{6a}$ and $R_{6b}$ are preferably hydrogen or hydroxy$C_{1-6}$alkyl; C(=O)-$R_7$ wherein $R_7$ is preferably optionally substituted thiazolyl;

d) Z represents a ring system selected from (a-1), (a-2), (a-3), (a-4), (a-9), (a-10), (a-12), (a-13), (a-14), (a-16) or (a-18).
e) $R_4$ represents hydrogen;
f)nis2.

Further interesting compounds are those compounds of formula (I) wherein one or more of the following restrictions apply:

a) $R_1$ represents $C_{1-6}$alkyl, especially ethyl or propyl;
b) $R_2$ represents halo, especially chloro or fluoro;
c) $R_3$ represents C(=O)-O-$R_5$ or C(=O)-$NR_{6a}R_{6b}$;
d) Z represents a cyclic ring system selected from (a-1), (a-2), (a-9), (a-10) or (a-13);
e) $R_4$ represents hydrogen;
f) n is 2.

Yet further interesting compounds are those compounds of formula (I) wherein one or more of the following restrictions apply:

a) $R_1$ represents ethyl or propyl;
b) $R_2$ represents chloro or fluoro;
c) $R_3$ represents C(=O)-O-$R_5$, especially C(=O)-O-$CH_3$;
d) Z represents cyclic ring system (a-9);
e) $R_4$ represents hydrogen;
f)nis2.

Preferred compounds of formula (I) are compounds 31, 6, 27, 9, 24, 40, 25, 7, 26, 45, 48, 49, 43, 36, 16, 28, 33, 32, 34, 51, 52 or 53; a *N*-oxide, a pharmaceutically acceptable addition salt, a quaternary amine, a polymorphic form or a stereochemically isomeric form thereof.

More preferred compounds of formula (I) are compounds 26, 48, 43, 52 or 53, especially compound 26, 43 or 53; a *N*-oxide, a pharmaceutically acceptable addition salt, a quaternary amine, a polymorphic form or a stereochemically isomeric form thereof.

Most preferred is any one of the following :

a compound of formula (I) wherein the compound is (S)-3-[1-(3,4-difluoro-phenyl)-propyl]-5-isoxazol-5-yl-2-thioxo-2,3-dihydro-1*H*-imidazole-4-carboxylic acid methyl ester, a *N*-oxide, a pharmaceutically acceptable addition salt, a quaternary amine or a polymorphic form thereof;
a compound of formula (I) wherein the compound is (S)-3-[1-(3,4-difluoro-phenyl)-propyl]-5-isoxazol-5-yl-2-thioxo-2,3-dihydro-1*H*-imidazole-4-carboxylic acid methyl ester or a *N*-oxide thereof;
a compound of formula (I) wherein the compound is (S)-3-[1-(3,4-difluoro-phenyl)-propyl]-5-isoxazol-5-yl-2-thioxo-2,3-dihydro-1*H*-imidazole-4-carboxylic acid methyl ester or a pharmaceutically acceptable addition salt thereof;
a compound of formula (I) wherein the compound is (S)-3-[1-(3,4-difluoro-phenyl)-propyl]-5-isoxazol-5-yl-2-thioxo-2,3-dihydro-1*H*-imidazole-4-carboxylic acid methyl ester or a quaternary amine thereof;
a compound of formula (I) wherein the compound is (S)-3-[1-(3,4-difluoro-phenyl)-propyl]-5-isoxazol-5-yl-2-thioxo-2,3-dihydro-1*H*-imidazole-4-carboxylic acid methyl ester; or
a compound of formula (I) wherein the compound is (S)-3-[1-(3,4-difluoro-phenyl)-propyl]-5-isoxazol-5-yl-2-thioxo-2,3-dihydro-1*H*-imidazole-4-carboxylic acid methyl ester with a melting point of 128.5°C.

In general, compounds of formula (I) wherein $R_3$ represents hydrogen, said compounds being represented by formula (I-a), can be prepared by reacting an intermediate of formula (II-a) or (II-b) with KSCN in the presence of a suitable acid, such as for example hydrochloric acid and the like, and a suitable solvent, such as for example an alcohol, e.g. ethanol, optionally in the presence of water.

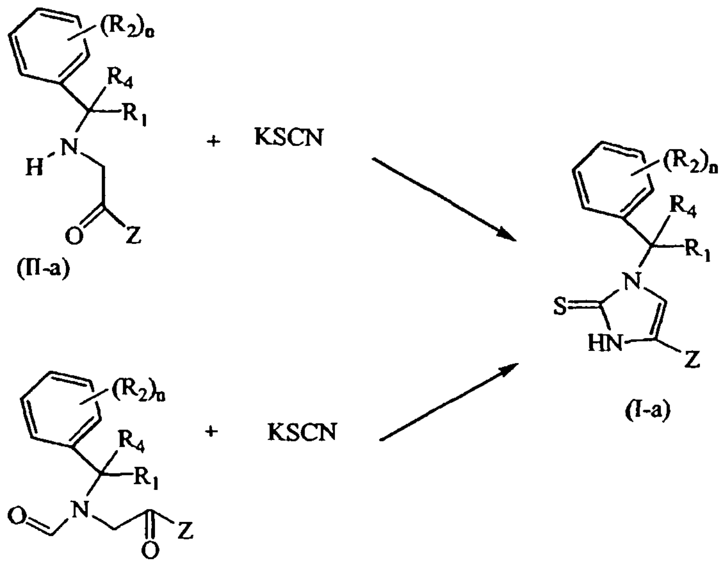


(II-a)

(I-a)

(II-b)

Compounds of formula (I) wherein $R_3$ is other than hydrogen, said $R_3$ being represented by $R_{3'}$ and said compounds being represented by formula (I-b), can be prepared by reacting an intermediate of formula (III) with an intermediate of formula (IV) wherein $W_1$ represents a suitable leaving group, such as for example $C_{1-6}$alkyloxy, e.g. methoxy or t-butyloxy, or halo, e.g. chloro and the like, in the presence of KSCN, a suitable acid, such as for example hydrochloric acid and the like, a suitable solvent, such as for example tetrahydrofuran, or a mixture of tetrahydrofuran and a suitable alcohol, e.g. methanol, and a suitable base, such as for example t-BuONa, LiN(isopropyl)$_2$ or NH[Si($CH_3$)$_3$]$_3$].

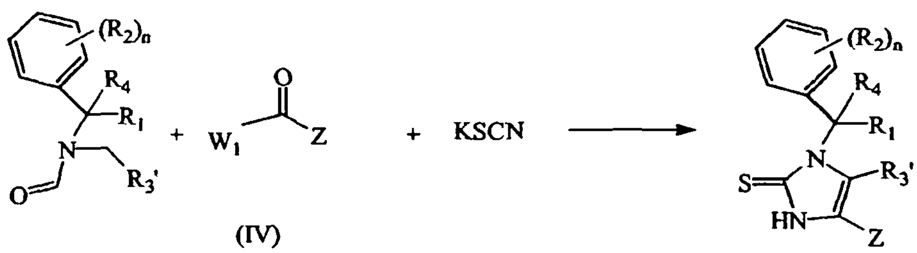


(IV)

(III)

(I-b)

Compounds of formula (I-b) can also be prepared by reacting an intermediate of formula (V) with a suitable base, such as for example sodium hydroxide and the like, in the presence of a suitable solvent, such as for example an alcohol, e.g. ethanol.

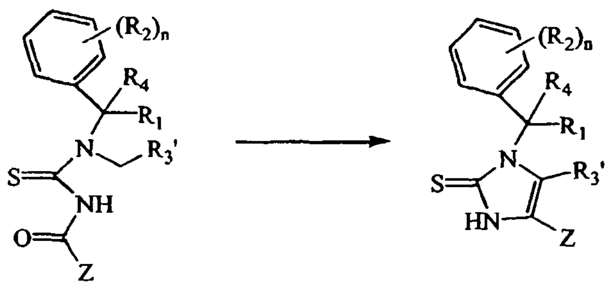

(V) (I-b)

Compounds of formula (I) wherein Z represents optionally substituted 1,3,4-oxadiazole, said compounds being represented by formula (I-c), can be prepared by reacting an intermediate of formula (VI) with phosphoric trichloride or Burgess'reagent optionally in the presence of a suitable solvent, such as for example tetrahydrofuran.

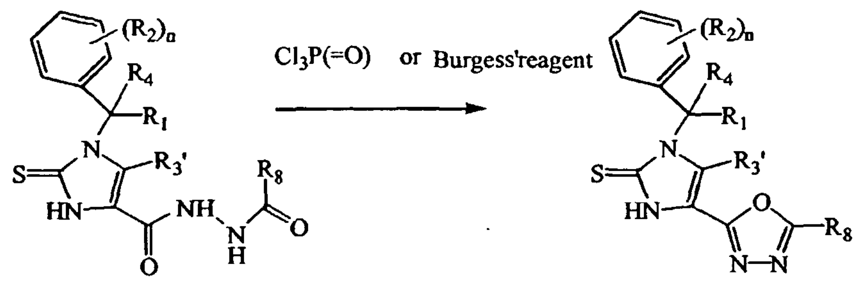


(VI) (I-c)

Compounds of formula (I) wherein $R_3$ represents C(=O)-O-$R_5$, wherein $R_{5'}$ represents $C_{1-6}$alkyl or hydroxy$C_{1-6}$alkyl, said compounds being represented by formula (I-d), can be prepared by reacting an intermediate of formula (VII), wherein $W_2$ represents a suitable leaving group, such as for example halo, e.g. chloro and the like, with an appropriate alcohol of formula HO-$R_{5'}$ in the presence of a suitable solvent, such as for example tetrahydrofuran.

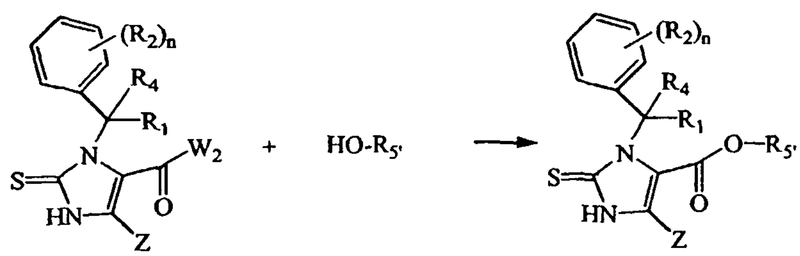


(VII) (I-d)

Compounds of formula (I) wherein $R_3$ represents C(=O)-N$R_{6a}R_{6b}$, said compounds being represented by formula (I-e), can be prepared by reacting an intermediate of formula (VII), with an intermediate of formula (VIII), such as for example $NH_3$ (or acetic acid ammonium salt), pyrrolidine and the like, in the presence of a suitable solvent, such as for example acetone, tetrahydrofuran, *N,N*-dimethylformamide and the like.

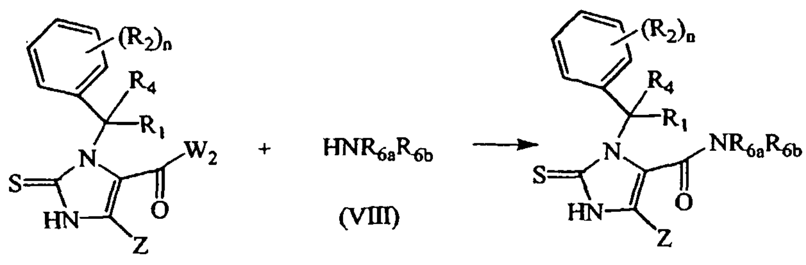

(VII) (I-e)

Compounds of formula (I) wherein $R_3$ represents $CH_2$-OH, said compounds being represented by formula (I-f), can be prepared by reacting an intermediate of formula (VII) with a suitable reducing agent, such as for example $NaBH_4$ in the presence of a suitable solvent, such as for example tetrahydrofuran.

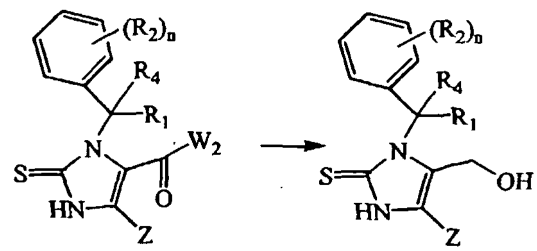

(VII) (I-f)

Compounds of formula (I') can be prepared according to the above described reactions but starting from an intermediate wherein the carbon atom carrying the $R_1$ and $R_4$ substituent has the (S) configuration.
Alternatively, compounds of formula (I) wherein the carbon atom carrying the $R_1$ and $R_4$ substituent has the (R) configuration can be prepared according to the above described reactions but starting from an intermediate wherein the carbon atom carrying the $R_1$ and $R_4$ substituent has the (R) configuration.

The compounds of formula (I) may further be prepared by converting compounds of formula (I) into each other according to art-known group transformation reactions.

The compounds of formula (I) may be converted to the corresponding *N*-oxide forms following art-known procedures for converting a trivalent nitrogen into its *N*-oxide form. Said *N*-oxidation reaction may generally be carried out by reacting the starting material of formula (I) with an appropriate organic or inorganic peroxide. Appropriate inorganic peroxides comprise, for example, hydrogen peroxide, alkali metal or earth alkaline metal peroxides, e.g. sodium peroxide, potassium peroxide; appropriate organic peroxides may comprise peroxy acids such as, for example, benzenecarboperoxoic acid or halo substituted benzenecarboperoxoic acid, e.g. 3-chlorobenzenecarboperoxoic acid, peroxoalkanoic acids, e.g. peroxoacetic acid, alkylhydroperoxides, e.g. tert.butyl hydro-peroxide. Suitable solvents are, for example, water, lower alcohols, e.g. ethanol and the like, hydrocarbons, e.g. toluene, ketones, e.g. 2-butanone, halogenated hydrocarbons, e.g. dichloromethane, and mixtures of such solvents.

Compounds of formula (I) wherein $R_3$ represents C(=O)-O-$C_{1-6}$alkyl, may be converted into a compound of formula (I) wherein $R_3$ represents $CH_2$-OH by reaction with a suitable reducing agent, such as for example $LiHBEt_3$ in the presence of a suitable solvent, such as for example tetrahydrofuran.

Compounds of formula (I) wherein $R_3$ represents C(=O)-O-$C_{1-6}$alkyl, can also be converted into a compound of formula (I) wherein $R_3$ represents C(=O)-OH by reaction with a suitable base, such as NaOH, in the presence of a suitable solvent, such as for example $H_2O$, tetrahydrofuran or an appropriate alcohol, e.g. methanol and the like.

Compounds of formula (I) wherein $R_3$ represents C(=O)-O-$C_{1-6}$alkyl, can also be converted into a compound of formula (I) wherein $R_3$ represents C(=O)-$NR_{6a}R_{6b}$, by reaction with the appropriate base of formula $NHR_{6a}R_{6b}$ in a suitable solvent, such as for example $H_2O$.

Compounds of formula (I) wherein $R_3$ represents C(=O)-O-H, can be converted into a compound of formula (I) wherein $R_3$ represents C(=O)-$NR_{6a}R_{6b}$, by reaction with the appropriate base of formula $NHR_{6a}R_{6b}$ in the presence

of *N'*-(ethylcarbonimidoyl)-*N,N*-dimethyl-1,3-propanediamine, 1-hydroxy-1*H*-benzotriazole and a suitable solvent, such as for example *N,N*-dimethylformamide.

Compounds of formula (I) wherein $R_3$ represents C(=O)-O-H, can also be converted into a compound of formula (I) wherein $R_3$ represents C(=O)-$NH_2$ by reaction with $NH_4OH$ in the presence of $SOCl_2$.

Compounds of formula (I) wherein $R_3$ represents C(=O)-O-$C_{1-6}$alkyl, can also be converted into a compound of formula (I) wherein $R_3$ represents C(=O)-O-$C_{1-6}$alkyl-O-$C_{1-6}$alkyl, by reaction with HO-$C_{1-6}$alkyl-O-$C_{1-6}$alkyl in the presence of $NaBH_4$.

Compounds of formula (I) wherein $R_3$ represents cyano or C(=O)-O-$C_{1-6}$alkyl, can be converted into a compound of formula (I) wherein $R_3$ represents aminocarbonyl by reaction with $NH_4OH$.

Compounds of formula (I) wherein $R_3$ represents cyano, can also be converted into a compound of formula (I) wherein $R_3$ represents C(=S)$NR_{6a}R_{6b}$ by reaction with hydrogen sulfide in the presence of *N*-ethyl-*N*-(1-methylethyl)-2-propanamine in a suitable solvent such as pyridine.

Compounds of formula (I) wherein $R_3$ represents C(=O)-$NR_{6a}R_{6b}$ can be converted into a compound of formula (I) wherein $R_3$ represents C(=O)-$C_{1-6}$alkyl by reaction with chloro$C_{1-6}$alkyMg in a suitable solvent such as tetrahydrofuran.

Compounds of formula (I) wherein $R_3$ represents C(=O)-$C_{1-6}$alkyl can be converted into compounds of formula (I) wherein $R_3$ represents hydroxy$C_{1-6}$alkyl by reaction with a suitable reducing agent such as $NaBH_4$, in the presence of a suitable solvent such as methanol.

Some of the compounds of formula (I) and some of the intermediates in the present invention may contain an asymmetric carbon atom. Pure stereochemically isomeric forms of said compounds and said intermediates can be obtained by the application of art-known procedures. For example, diastereoisomers can be separated by physical methods such as selective crystallization or chromatographic techniques, e.g. counter current distribution, liquid chromatography and the like methods. Enantiomers can be obtained from racemic mixtures by first converting said racemic mixtures with suitable resolving agents such as, for example, chiral acids, to mixtures of diastereomeric salts or compounds; then physically separating said mixtures of diastereomeric salts or compounds by, for example, selective crystallization or chromatographic techniques, e.g. liquid chromatography and the like methods; and finally converting said separated diastereomeric salts or compounds into the corresponding enantiomers. Pure stereochemically isomeric forms may also be obtained from the pure stereochemically isomeric forms of the appropriate intermediates and starting materials, provided that the intervening reactions occur stereospecifically.

An alternative manner of separating the enantiomeric forms of the compounds of formula (I) and intermediates involves liquid chromatography, in particular liquid chromatography using a chiral stationary phase.

Some of the intermediates and starting materials are known compounds and may be commercially available or may be prepared according to art-known procedures.

Intermediates of formula (II-a) may be prepared by reacting an intermediate of formula (VIII) wherein $W_3$ represents a suitable leaving group, such as for example halo, e.g. bromo, with an intermediate of formula (IX) in the presence of a suitable base, such as for example *N,N*-diisopropylethanamine, and a suitable solvent, such as for example tetrahydrofuran.

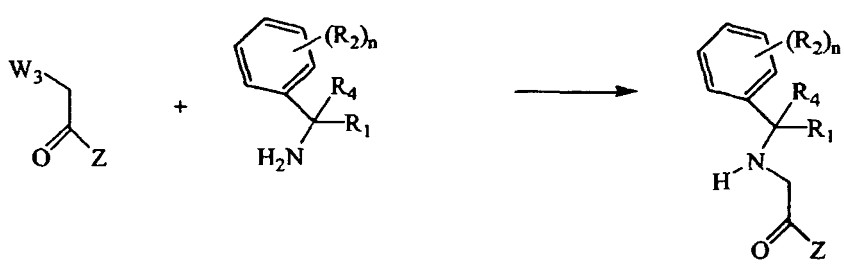

(VIII) (IX) (II-a)

Intermediates of formula (IX) wherein $R_4$ represents hydrogen, said intermediates being represented by formula (IX-a), may be prepared by reacting an intermediate of formula (X) with a suitable reducing agent, such as for example $H_2$, in the presence of a suitable catalyst, such as for example Raney Nickel, a suitable catalyst poison, such as for example a thiophene solution, and a suitable base, such as for example $NH_3$.

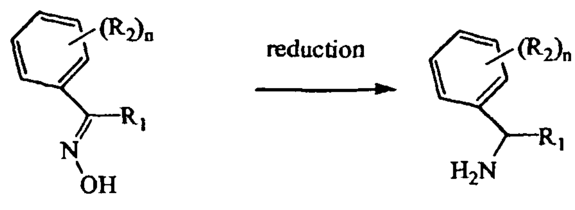


(X) (IX-a)

Intermediates of formula (X) may be prepared by reacting an intermediate of formula (XI) with HO-$NH_2$ in the presence of a suitable base, such as for example $Na_2CO_3$, and a suitable solvent, such as for example an alcohol, e.g. ethanol, and water.

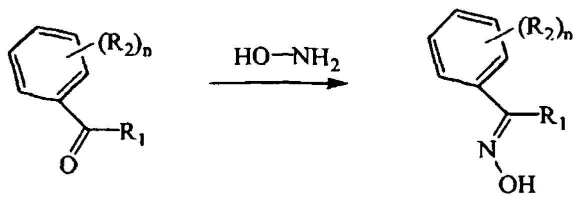


(XI) (X)

Intermediates of formula (II-b) can be prepared by reacting an intermediate of formula (VIII) with an intermediate of formula (XII) in the presence of a suitable solvent, such as for example acetonitrile.

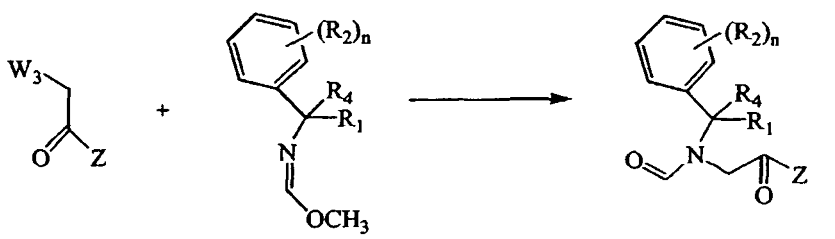

(VIII) (XII) (II-b)

Intermediates of formula (XII) can be prepared by reacting an intermediate of formula (XIII) with trimethyloxonium and tetrafluoroborate in the presence of a suitable solvent, such as for example methylenechloride.

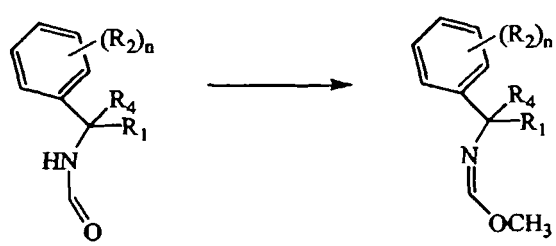

(XIII) (XII)

Intermediates of formula (XIII) wherein $R_4$ is hydrogen, said intermediates being represented by formula (XIII-a), can be prepared by reacting an intermediate of formula (XIV) with HC(=O)-$NH_2$ in the presence of a suitable acid, such as for example formic acid.

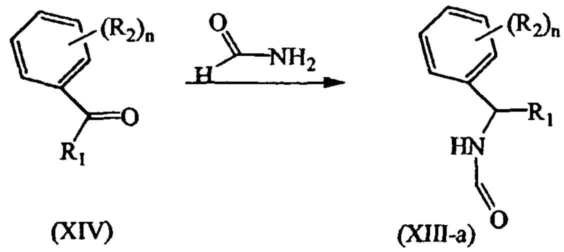

(XIV) (XIII-a)

Intermediates of formula (III) can be prepared from an intermediate of formula (XV) in the presence of formic acid or a formate, such as for example n-butylformate, and in the presence of a suitable solvent, such as for example xylene.

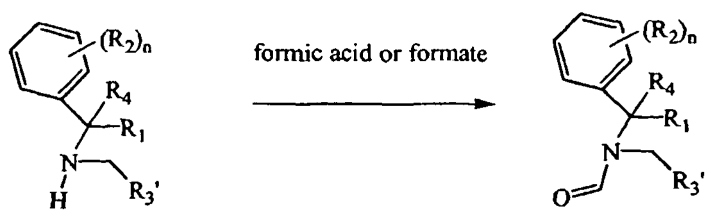


(XV) (III)

Intermediates of formula (XV) can be prepared by reacting an intermediate of formula (IX) with an intermediate of formula (XVI) wherein $W_4$ represents a suitable leaving group, such as for example halo, e.g. chloro and the like, in the presence of a suitable base, such as for example *N,N*-diethylethanamine, and a suitable solvent, such as for example *N,N*-dimethylformamide or tetrahydrofuran. The intermediates of formula (IX) may contain a chiral center at the carbon atom carrying the $R_1$ and $R_4$ substituent depending on the substituents representing $R_1$ and $R_4$. Said stereospecific intermediates of formula (IX) are represented by formula (IX-b). When the reaction is performed starting from a stereospecific intermediate of formula (IX-b), a stereospecific intermediate of formula (XV) is obtained, said intermediate being represented by formula (XV-a).

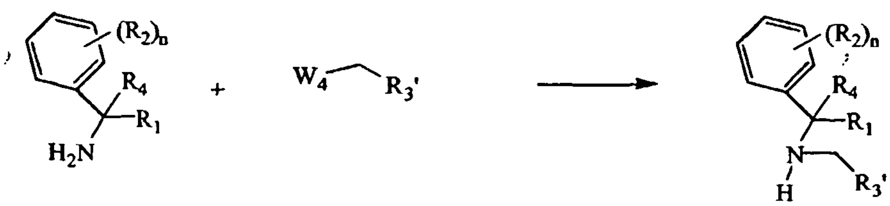

(IX) (XVI) (XV)

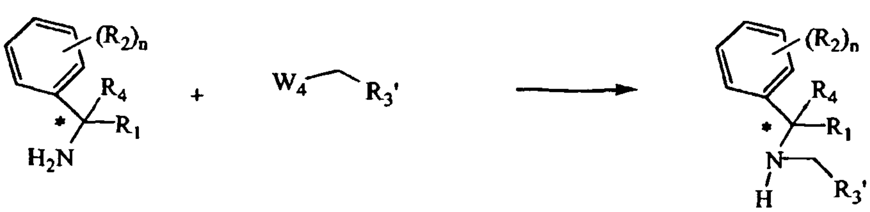

(IX-b) (XVI) (XV-a)

* indicates the chiral center and may be (R) or (S) depending on the $R_1$ and $R_4$ substituents

Stereospecific intermediates of formula (XV-a) can also be prepared by reacting an intermediate of formula (XV) with a suitable stereospecific resolution agent, such as for example [S-(R*,R*)]-2,3-bis[(4-methylbenzoyl)oxy]-butanedioic acid, in the presence of a suitable solvent, such as for example an alcohol, e.g. 2-propanol. When a stereospecific intermediate of formula (XV-a) is reacted further according to the methods described hereinabove, the resulting intermediates are also stereospecific and finally the resulting final compounds are also stereospecific.

Intermediates of formula (XV) wherein $R^4$ represents hydrogen, said intermediates being represented by formula (XV-b), can also be prepared by reacting an intermediate of formula (XXVI) with an intermediate of formula (XXVII) in the presence of a suitable reducing agent, such as for example $H_2$, a suitable catalyst, such as for example Pd on charcoal, a suitable catalyst poison, such as for example a thiophene solution, a suitable weak base, such as for example KF or potassium acetate, a suitable acid, such as for example hydrochloric acid, and a suitable solvent, such as for example an alcohol, e.g. methanol.

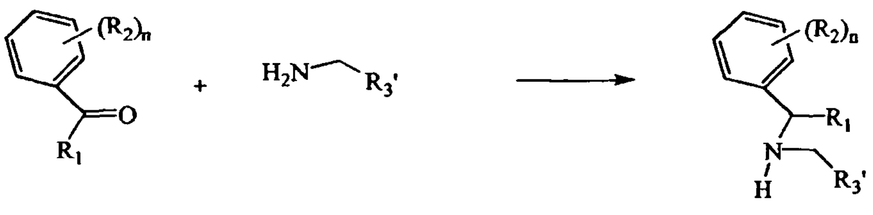

(XXVI) (XXVII) (XV-b)

Intermediates of formula (IX-b), can be prepared by reacting an intermediate of formula (XVII) with triphenylphosphine, in the presence of a suitable solvent, such as for example tetrahydrofuran and water or by reacting an intermediate of formula (XVII) with a suitable reducing agent, such as for example $H_2$, in the presence of a suitable catalyst, such as for example Pt on charcoal or Pd on charcoal, and a suitable solvent, such as for example an alcohol, e.g. methanol.

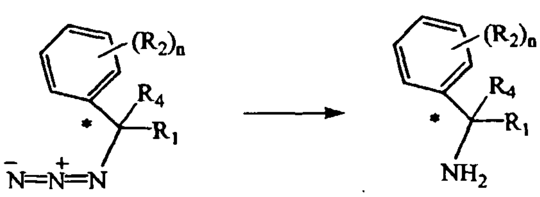

(XVII) (IX-b)

* indicates the chiral center and may be (R) or (S) depending on the $R_1$ and $R_4$ substituents

When a stereospecific intermediate of formula (IX-b) is reacted further according to the methods described hereinabove, the resulting intermediates are also stereospecific and finally the resulting final compounds are also stereospecific.

Intermediates of formula (XVII) can be prepared by reacting an intermediate of formula (XVIII) with diphenylphosphoryl azide in the presence of 2,3,4,6,7,8,9,10-octahydro-pyrimido[1,2-a]azepine and in the presence of a suitable solvent, such as for example toluene.

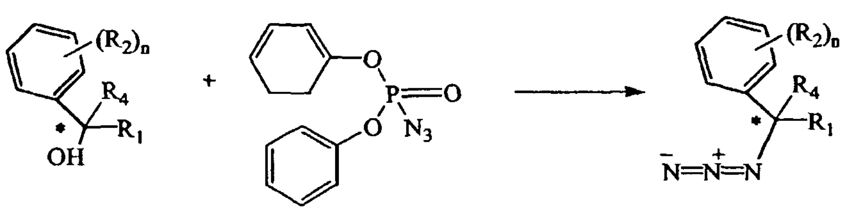

(XVIII) (XVII)

* indicates the chiral center and may be (R) or (S) depending on the $R_1$ and $R_4$ substituents Stereospecific intermediates

of formula (XVIII) wherein $R_4$ is hydrogen and $R_1$ is methyl, ethyl, or *n*-propyl, said $R_1$ being represented by Alk and said intermediates being represented by formula (XVIII-a) and (XVIII-b), can be prepared by reacting an intermediate of formula (XIX) with $ZnAlk_2$ wherein Alk represents methyl, ethyl or *n*-propyl, in the presence of a stereospecific catalyst, such as for example *N,N'*-(1R,2R)-1,2-cyclohexanediylbis[1,1,1-trifluoro]-methanesulfonamide respectively *N,N'*-(1S, 2S)-1,2-cyclohexanediylbis[1,1,1-trifluoro]-methanesulfonamide, $Ti(iPrO)_4$ and a suitable solvent, such as for example toluene.

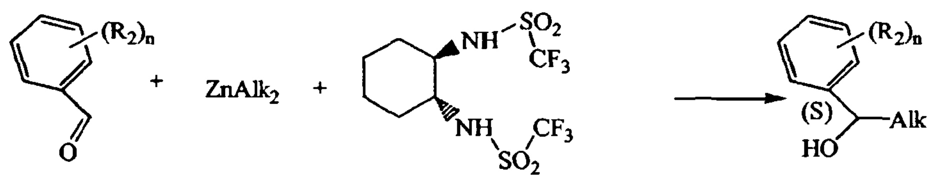

(XIX) 1R,2R (XVIII-a)

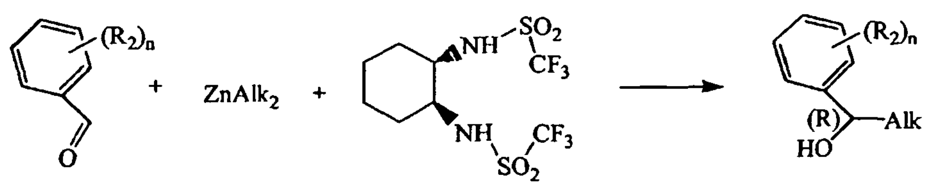

(XIX) 1S,2S (XVIII-b)

Intermediates of formula (V) can be prepared by reacting an intermediate of formula (XX) wherein $W_5$ represents a suitable leaving group, such as for example halo, e.g. chloro and the like, with an intermediate of formula (IX) and an intermediate of formula (XXI), in the presence of a suitable base, such as for example *N,N*-diisopropylethanamine, and a suitable solvent, such as for example tetrahydrofuran.

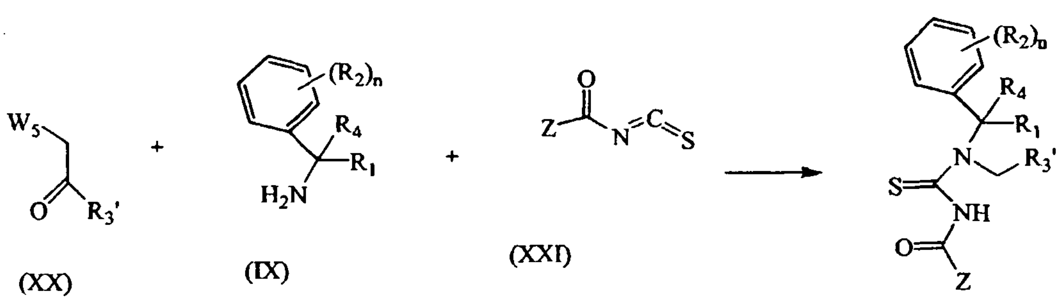

(XX) (IX) (XXI) (V)

Intermediates of formula (VI) can be prepared by reacting an intermediate of formula (XXII) wherein $W_6$ represents a suitable leaving group, such as for example halo, e.g. chloro, with an intermediate of formula (XXIII) in the presence of a suitable solvent, such as for example tetrahydrofuran, optionally in the presence of a suitable base, such as for example *N,N*-diethylethanamine.

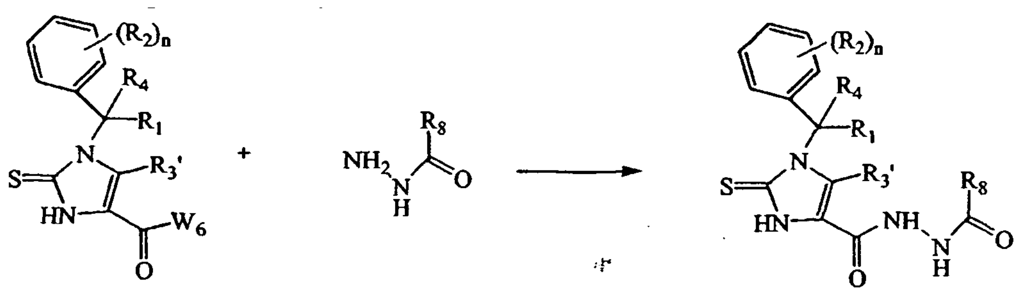

(XXII) (XXIII) (VI)

Intermediates of formula (XXII) wherein $W_6$ represents chloro, said intermediates being represented by formula (XXIII-a), can be prepared by reacting an intermediate of formula (XXIV) with $SOCl_2$ optionally in the presence of a suitable solvent, such as for example methylene chloride.

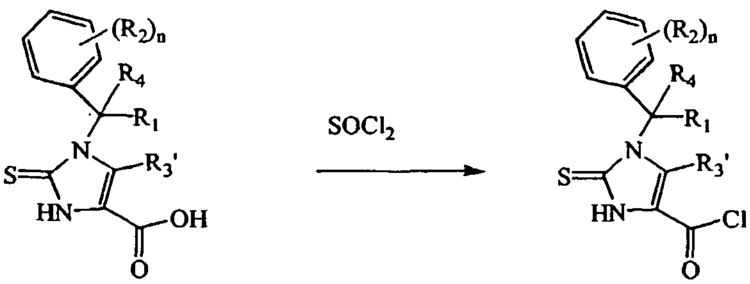


(XXIV) (XXII-a)

Intermediates of formula (XXIV) can be prepared by reacting an intermediate of formula (III) with an intermediate of formula (XXVI) in the presence of KSCN, $NaOC(CH_3)_3$, a suitable acid, such as for example hydrochloric acid, and a suitable solvent, such as for example tetrahydrofuran. This reaction also leads to the preparation of intermediates of formula (XXV).

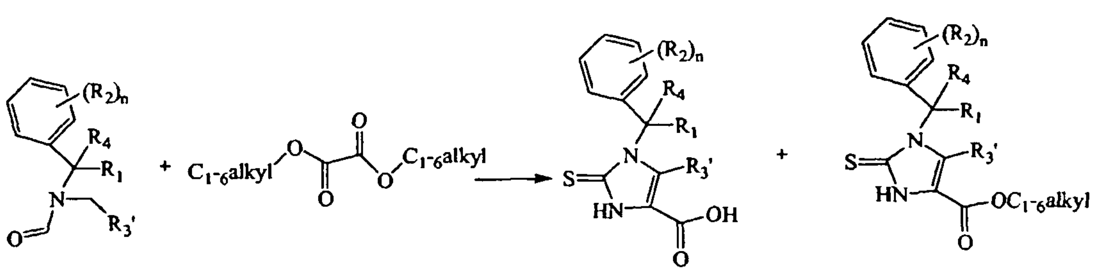

(III) (XXVI) (XXIV) (XXV)

Intermediates of formula (XXIV) can also be prepared by hydrolyzing an intermediate of formula (XXV) in the presence of a suitable base, such as for example sodium hydroxide, in the presence of a suitable solvent, such as an alcohol, e.g. methanol, and water. Intermediates of formula (XXIV) may also be prepared by hydrolysis of an intermediate of formula (XXV) in the presence of a suitable acid, such as for example trifluoroacetic acid, in the presence of a suitable solvent, such as for example methylene chloride.

In the preparation of the compounds of the present invention, interesting intermediates are intermediates of formula (IX)

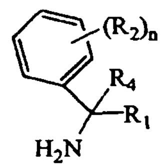

(IX) ,

a *N*-oxide, a pharmaceutically acceptable addition salt, a quaternary amine or stereochemically isomeric form thereof.

As already indicated hereinabove, the intermediates of formula (IX) may contain a chiral center at the carbon atom carrying the $R_1$ and $R_4$ substituent depending on the substituents representing $R_1$ and $R_4$. In case said carbon atom represents a chiral center, a preferred embodiment of the intermediates of formula (IX) are those intermediates wherein the intermediate is stereospecific, i.e. wherein the intermediate has the (R) or (S) configuration at the carbon atom carrying the $R_1$ and $R_4$ substituent (intermediates of formula (IX-b). Particularly preferred are those intermediates of formula (IX-b) which have the (S) configuration (intermediates of formula (IX-b-1).

Thus the present invention also relates to intermediates of formula (IX-b-1)

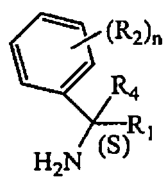

(IX-b-1) ,

a *N*-oxide, a pharmaceutically acceptable addition salt or a quaternary amine thereof.

The present invention also relates to intermediates of formula (IX-b-1) provided that when n=2 and each $R_2$ is chloro and said two chloro substituents are placed in meta and para position, then $R_1$ is other than ethyl.

Another preferred embodiment are those intermediates of formula (IX-b-1) wherein each $R_2$ is independently selected from halo, provided that when n=2 and each $R_2$ is chloro and said two chloro substituents are placed in meta and para position, then $R_1$ is other than ethyl.

A further embodiment are those intermediates of formula (IX-b-1) wherein n is 1, 2, or 3, in particular 2, provided that when n is 2 and each $R_2$ is chloro and said two chloro substituents are placed in meta and para position, then $R_1$ is other than ethyl.

Again another embodiment are those intermediates of formula (IX-b-1) wherein n is 2 and the two $R_2$ substituents are placed in meta and para position provided that when each $R_2$ is chloro, then $R_1$ is other than ethyl.

Another embodiment are those intermediates of formula (IX-b-1) as described hereinabove wherein $R_4$ is hydrogen.

Also interesting are those intermediates of formula (IX) and (IX-b-1) wherein $R_1$ is methyl, ethyl, *n*-propyl, methoxymethyl, in particular methyl, ethyl and *n*-propyl provided that when n is 2 and $R_2$ represents chloro, and said two chloro substituents are placed in meta and para position, and $R_4$ is hydrogen, then $R_1$ is other than ethyl.

Also interesting are those intermediates of formula (IX) or (IX-b-1) as described hereinabove wherein $R_2$ is chloro or fluoro, more in particular fluoro.

Particularly interesting intermediates are those intermediates of formula (IX-a) or (IX-b-1) having the following formula

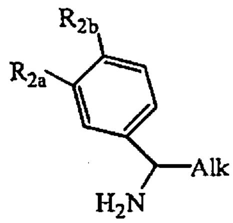

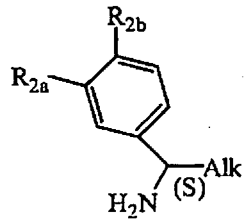

(IX-a-1) (IX-b-1-1)

a *N*-oxide, a pharmaceutically acceptable addition salt or a quaternary amine thereof, wherein Alk is defined as hereinabove, i.e. Alk represents methyl, ethyl and *n*-propyl, and each $R_{2a}$ and $R_{2b}$ independently represents chloro or fluoro.

An interesting embodiment are those intermediates of formula (IX-a-1) provided that when $R_{2a}$ and $R_{2b}$ are both chloro, then Alk is other than ethyl.

Further interesting intermediates of formula (IX-a-1) are those intermediates of formula (IX-a-1) provided that when $R_{2a}$ and $R_{2b}$ are both chloro, then Alk is other than methyl, ethyl, *n*-propyl and provided that when $R_{2a}$ and $R_{2b}$ are both fluoro then Alk is other than ethyl.

Also interesting are those intermediates of formula (IX-b-1-1) provided that when $R_{2a}$ and $R_{2b}$ are both chloro, then Alk is other than ethyl.

A particular interesting intermediate of formula (IX-b-1-1) is that intermediate wherein $R_{2a}$ and $R_{2b}$ are both fluoro and Alk represents ethyl. i.e. a compound of formula (IX-b-1-1-a).

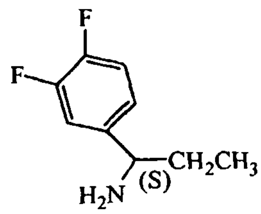

(IX-b-1-1-a)

The compounds of formula (I) and (I') show CCR2 receptor antagonistic properties.

The C - C chemokine receptor 2 (CCR2) and its ligand monocyte chemoattractant (chemotactic) protein (MCP-1; in new chemokine nomenclature also called CCL2) are recognized to be implicated in both acute and chronic inflammatory processes.

Chemokines (contraction of "chemotactic cytokines") are most important regulators of leukocyte trafficking. This biological role is exerted by interacting - on target cells - with seven-transmembrane-domain receptors that are coupled to heterodimeric G proteins. Chemokines are mainly grouped into 2 major families (C - C or C - X - C family) dependent on the presence of an amino acid (represented by X) between the two conserved cysteine residues (represented by C) near the amino terminus. In general, chemokines from the C - C family attract monocytes, macrophages, T cells and NK cells.

A chemokine, which acts through the CCR2 receptor, is MCP-1 as indicated above. Therefore, the CCR2 receptor is also known as the MCP-1 receptor.

MCP-2, MCP-3 and MCP-4 may also act, at least in part, through this receptor.

It is recognized that the CCR2 receptor and MCP-1 play a role in the pathophysiology of various inflammatory diseases. Therefore, CCR2 receptor antagonists, which block the CCR2 receptor, have potential as pharmaceutical agents to combat inflammatory conditions such as arthritis, osteoarthritis, rheumatoid arthritis, glomerulonephritis, diabetic nephropathy, lung fibrosis, idiopathic pulmonary fibrosis, sarcoidosis, vasculitis, hepatitis, nonalcoholic steatohepatitis, inflammatory conditions of the brain such as Alzheimer's disease, restenosis, alveolitis, asthma, allergic rhinitis, allergic conjunctivitis, atherosclerosis, psoriasis, delayed-type hypersensitivity reactions of the skin, inflammatory bowel disease, acute or chronic brain inflammation, e.g. multiple sclerosis, autoimmune encephalomyelitis, chronic obstructive pulmonary disease (COPD), uveitis, dermatitis, atopic dermatitis. CCR2 receptor antagonists may also be useful to treat autoimmune diseases such as diabetes or transplant rejection, stroke, reperfusion injury, ischemia, cancer, myocardial

infraction, pain, in particular neuropathic pain.

The compounds of the present invention may also be used to inhibit the entry of Human Immunodeficiency Virus (HIV) into monocytes and lymphocytes, thereby having a therapeutic role in the treatment of AIDS (Acquired Immunodeficiency Syndrome).

The CCR2 receptor exists in two isoforms, namely the CCR2A and the CCR2B receptor.

Due to their CCR2 receptor antagonistic activity, in particular their CCR2B receptor antagonistic activity, the compounds of formula (I), their *N*-oxides, pharmaceutically acceptable addition salts, quaternary amines, polymorphic forms or stereochemically isomeric forms are useful in the treatment or prevention, in particular for the treatment, of diseases or conditions mediated through the activation of the CCR2 receptor, in particular the CCR2B receptor. Diseases or conditions related to an activation of the CCR2 receptor comprise inflammatory conditions such as arthritis, osteoarthritis, rheumatoid arthritis, glomerulonephritis, diabetic nephropathy, lung fibrosis, idiopathic pulmonary fibrosis, sarcoidosis, vasculitis, hepatitis, nonalcoholic steatohepatitis, inflammatory conditions of the brain such as Alzheimer's disease, restenosis, alveolitis, asthma, allergic rhinitis, allergic conjunctivitis, atherosclerosis, psoriasis, delayed-type hypersensitivity reactions of the skin, inflammatory bowel disease, acute or chronic brain inflammation, e.g. multiple sclerosis, autoimmune encephalomyelitis, chronic obstructive pulmonary disease (COPD), uveitis, dermatitis, atopic dermatitis, autoimmune diseases such as diabetes or transplant rejection, stroke, reperfusion injury, ischemia, cancer, myocardial infraction, pain (neuropathic pain). In particular, the compounds of formula (I) are useful in the treatment or prevention of inflammatory diseases and autoimmune diseases, especially rheumatoid arthritis, atherosclerosis, multiple sclerosis, inflammatory bowel disease and chronic obstructive pulmonary disease (COPD). The compounds of formula (I) are also of particular interest in the treatment or prevention of psoriasis, asthma, rheumatoid arthritis or pain (neuropathic pain), more in particular psoriasis, asthma or rheumatoid arthritis.

In view of the above-described pharmacological properties, the compounds of formula (I), their *N*-oxides, pharmaceutically acceptable addition salts, quaternary amines and stereochemically isomeric forms, may be used as a medicine. In particular, the present compounds can be used for the manufacture of a medicament for treating or preventing diseases mediated through activation of the CCR2 receptor, in particular the CCR2B receptor. More in particular, the compounds of the invention can be used for the manufacture of a medicament for treating or preventing inflammatory diseases, especially rheumatoid arthritis, atherosclerosis, multiple sclerosis, inflammatory bowel disease and chronic obstructive pulmonary disease (COPD). The compounds of the invention can also in particular be used for the manufacture of a medicament for treating or preventing psoriasis, asthma, rheumatoid arthritis or pain (neuropathic pain), more in particular psoriasis, asthma or rheumatoid arthritis.

The blockade of the CCR2 receptor by the present compounds of formula (I) inhibits the normal function of MCP-1. Therefore, the present compounds can also be described as MCP-1 inhibitors and hence can be used to prevent or treat diseases mediated through MCP-1.

The present invention also provides compositions for preventing or treating diseases mediated through activation of the CCR2 receptor, in particular the CCR2B receptor. Said compositions comprise a therapeutically effective amount of a compound of formula (I) and a pharmaceutically acceptable carrier or diluent.

The compounds of the present invention may be formulated into various pharmaceutical forms for administration purposes. As appropriate compositions there may be cited all compositions usually employed for systemically administering drugs. To prepare the pharmaceutical compositions of this invention, an effective amount of the particular compound, optionally in addition salt form, as the active ingredient is combined in intimate admixture with a pharmaceutically acceptable carrier, which carrier may take a wide variety of forms depending on the form of preparation desired for administration. These pharmaceutical compositions are desirable in unitary dosage form suitable, particularly, for administration orally, rectally, percutaneously, or by parenteral injection. For example, in preparing the compositions in oral dosage form, any of the usual pharmaceutical media may be employed such as, for example, water, glycols, oils, alcohols and the like in the case of oral liquid preparations such as suspensions, syrups, elixirs, emulsions and solutions; or solid carriers such as starches, sugars, kaolin, diluents, lubricants, binders, disintegrating agents and the like in the case of powders, pills, capsules, and tablets. Because of their ease in administration, tablets and capsules represent the most advantageous oral dosage unit forms, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. Also included are solid form preparations, which are intended to be converted, shortly before use, to liquid form preparations. In the compositions suitable for percutaneous administration, the carrier optionally comprises a penetration enhancing agent and/or a suitable wetting agent, optionally combined with suitable additives of any nature in minor proportions, which additives do not introduce a significant deleterious effect on the skin. Said additives may facilitate the administration to the skin and/or may be helpful for preparing the desired compositions. These compositions may be administered in various ways, e.g., as a transdermal patch, as a spot-on, as an ointment.

The compounds of the present invention may also be administered via inhalation or insufflation by means of methods and formulations employed in the art for administration via this way. Thus, in general the compounds of the present invention may be administered to the lungs in the form of a solution, a suspension or a dry powder. Any system developed for the delivery of solutions, suspensions or dry powders via oral or nasal inhalation or insufflation are suitable for the administration of the present compounds.

The compounds of the present invention may also be topically administered in the form of drops, in particular eye drops. Said eye drops may be in the form of a solution or a suspension. Any system developed for the delivery of solutions or suspensions as eye drops are suitable for the administration of the present compounds.

It is especially advantageous to formulate the aforementioned pharmaceutical compositions in unit dosage form for ease of administration and uniformity of dosage.

Unit dosage form as used herein refers to physically discrete units suitable as unitary dosages, each unit containing a predetermined quantity of active ingredient calculated to produce the desired therapeutic effect in association with the required pharmaceutical carrier. Examples of such unit dosage forms are tablets (including scored or coated tablets), capsules, pills, powder packets, wafers, suppositories, injectable solutions or suspensions and the like, and segregated multiples thereof.

The exact dosage and frequency of administration depends on the particular compound of formula (I) used, the particular condition being treated, the severity of the condition being treated, the age, weight, sex, extent of disorder and general physical condition of the particular patient as well as other medication the individual may be taking, as is well known to those skilled in the art. Furthermore, it is evident that said effective daily amount may be lowered or increased depending on the response of the treated subject and/or depending on the evaluation of the physician prescribing the compounds of the instant invention.

The compounds of formula (I) may also be used in combination with other conventional anti-inflammatory or immunosuppressive agents, such as steroids, cyclooxygenase-2 inhibitors, non-steroidal-anti-inflammatory drugs, TNF-a antibodies, such as for example acetyl salicylic acid, bufexamac, diclofenac potassium, sulindac, diclofenac sodium, ketorolac trometamol, tolmetine, ibuprofen, naproxen, naproxen sodium, tiaprofen acid, flurbiprofen, mefenamic acid, nifluminic acid, meclofenamate, indomethacin, proglumetacine, ketoprofen, nabumetone, paracetamol, piroxicam, tenoxicam, nimesulide, fenylbutazon, tramadol, beclomethasone dipropionate, betamethasone, beclamethasone, budesonide, fluticasone, mometasone, dexamethasone, hydrocortisone, methylprednisolone, prednisolone, prednisone, triamcinolone, celecoxib, rofecoxib, valdecoxib, infliximab, leflunomide, etanercept, CPH 82, methotrexate, sulfasalazine, antilymphocytory immunoglobulines, antithymocytory immunoglobulines, azathioprine, cyclosporine, tacrolimus substances, ascomycin, rapamycin, muromonab-CD3.

Thus, the present invention also relates to the combination of a compound of formula (I) and another anti-inflammatory or immunosuppressive agent. Said combination may be used as a medicine. The present invention also relates to a product containing (a) a compound of formula (I), and (b) another anti-inflammatory or immunosuppressive compound, as a combined preparation for simultaneous, separate or sequential use in the treatment of diseases mediated through activation of the CCR2 receptor, in particular mediated through the CCR2B receptor. The different drugs in such products may be combined in a single preparation together with pharmaceutically acceptable carriers. Alternatively, such products may comprise, for example, a kit comprising a container with a suitable composition containing a compound of formula (I) and another container with a composition containing another anti-inflammatory or immunosuppressive compound. Such a product may have the advantage that a physician can select on the basis of the diagnosis of the patient to be treated the appropriate amounts of each component and the sequence and timing of the administration thereof.

The following examples are intended to illustrate the present invention.

## Experimental Part

Hereinafter "RT" means room temperature, "THF" means tetrahydrofuran, "DIPE" means diisopropylether, "TFA" means trifluoroacetic acid, "DBU" means 2,3,4,6,7,8,9,10-octahydropyrimido[1,2-a]azepine and "DMF" means *N*,*N*-dimethylformamide.

## A. Preparation of the intermediate compounds

### Example A1

#### a. Preparation of intermediate 1

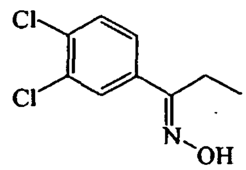

A solution of $Na_2CO_3$ (part of 0.52 mol) in $H_2O$ (150 ml) was added to a stirring mixture of 1-(3,4-dichlorophenyl)-1-propanone (0.345 mol) in ethanol, p.a. (150 ml), then the remainder of $Na_2CO_3$ was added and hydroxylamine monohydrochloride (0.345 mol) was added portionwise while stirring vigorously. The reaction mixture was heated to reflux temperature and extra $H_2O$ (75 ml) was added, then the resulting mixture was stirred and refluxed for 6 hours. Extra hydroxylamine monohydrochloride (2.4 g) was added and the mixture was refluxed further for 18 hours. Again extra hydroxylamine monohydrochloride (3 g) was added; the reaction mixture was refluxed for 24 hours and stirred for 2 days at room temperature. The solids were filtered off, washed with EtOH/$H_2O$ (1/1) and dried (vacuum, stream of air) at 56˚C. Yield: 71.8 g of intermediate 1 (95.4 %).

b. Preparation of intermediate 2 and 3

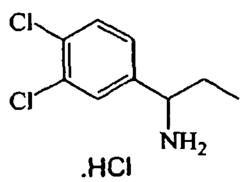

.HCl

Intermediate 2

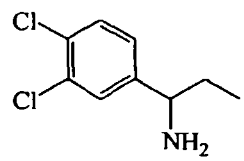

Intermediate 3

A mixture of intermediate 1 (0.3 mol) in $CH_3OH/NH_3$ (7 N) (500 ml) was hydrogenated at 14˚C with Raney Nickel (cat. quant.) as a catalyst in the presence of thiophene solution (6 ml). After uptake of $H_2$ (2 equiv.), the catalyst was filtered off and the filtrate was evaporated, then co-evaporated 2 times with toluene. The residue was stirred in boiling 2-propanol (250 ml) and the mixture was filtered off hot. The filtrate was allowed to reach room temperature and HCl/2-propanol (6N, 150 ml) was added slowly while stirring vigorously. The solvent was evaporated and the residue was stirred in DIPE, then filtered off, washed and dried (vacuum) at 60˚C. Yield: 53 g of intermediate 2 (73.4 %). A part of this fraction was converted into its free base: Intermediate 2 (18.0 g) was stirred in $CH_2Cl_2$ (200 ml) and a 15 % aqueous $K_2CO_3$ solution was added, then the resulting mixture was stirred for 1 hour and a 50 % NaOH solution was added to increase the pH. The organic layer was separated, washed with $H_2O$, dried ($MgSO_4$), filtered off and the solvent was evaporated. Yield: 51 g of intermediate 3.

Example A2

a. Preparation of intermediate 4

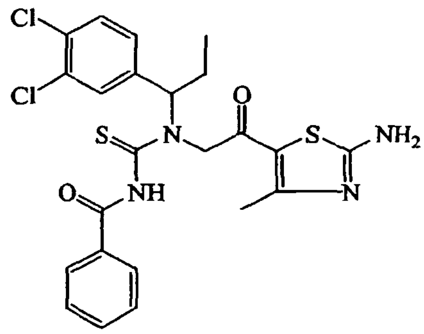

Intermediate 3 (prepared according to A1.b) (0.00208 mol) was dissolved in THF (15 ml) and *N*-ethyl-*N*-(1-methylethyl)-2-propanamine (0.0052 mol) was added, followed by 1-(2-amino-4-methyl-5-thiazolyl)-2-bromo-ethanone monohydrobromide (0.00104 mol). The reaction mixture was stirred at room temperature for 18 hours and benzoyl isothiocyanate (0.003 mol) was added, then the mixture was stirred for 2 hours at room temperature and poured out into ice-water (60 ml). The product was extracted with $CH_2Cl_2$ and then the organic layer was separated, dried ($MgSO_4$) and filtered off. Finally, the solvent was evaporated. Yield: 1.38 g of intermediate 4.

b. Preparation of intermediate 5

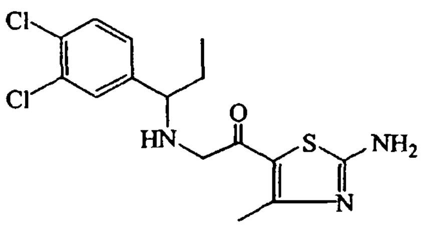

Intermediate 3 (prepared according to A1.b) (208 mg) was dissolved in THF, p.a., dried on molecular sieves, (15 ml) and then *N*-ethyl-*N*-(1-methylethyl)-2-propanamine (0.0016 mol) was added followed by 1-(2-amino-4-methyl-5-thiazolyl)-2-bromoethanone monohydrobromide (0.00095 mol). The reaction mixture was stirred for 18 hours at room temperature and the resulting precipitate was filtered off, washed with THF and dried in vacuum. Yield: 330 mg of intermediate 5.

Example A3

a. Preparation of intermediate 6

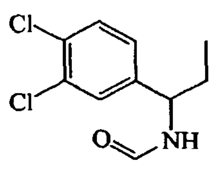

A solution of 1-(3,4-dichlorophenyl)-1-propanone (0.0748 mol) in formamide (45 ml) and formic acid (30 ml) was stirred and refluxed for 22 hours and then the reaction mixture was allowed to reach room temperature. The mixture was warmed to 50˚C, poured out into ice water (400 ml) and extracted with EtOAc. The organic layer was separated, washed with $H_2O$, with a saturated aqueous $NaHCO_3$ solution and again with $H_2O$ and with brine, then dried ($MgSO_4$)

and filtered off. The solvent was evaporated and the residual oil was left to stand overnight. The resulting solids were filtered off, stirred in DIPE, filtered off again, washed and dried (vacuum) at 50˚C. Yield: 14.0 g of intermediate 6 (80.6 %).

b. Preparation of intermediate 7

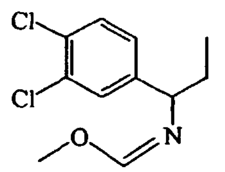

A solution of intermediate 6 (0.0032 mol) in $CH_2Cl_2$ p.a. (5 ml) was stirred at room temperature under $N_2$ and then a solution of trimethyloxonium tetrafluoroborate (0.00331 mol) in $CH_2Cl_2$ p.a. (5 ml) was added. The reaction mixture was stirred at room temperature for 24 hours and was slowly poured out into an aqueous NaOH solution with ice, then $CH_2Cl_2$ was added. The separated organic layer was dried over NaOH-pellets, filtered off and the solvent was evaporated, yielding intermediate 7 which was used in the next step.

c. Preparation of intermediate 8

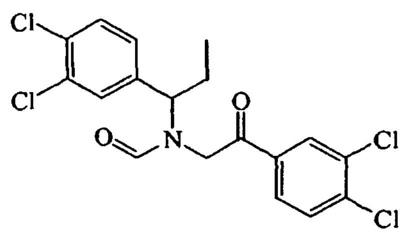

2-Bromo-1-(3,4-dichlorophenyl)ethanone (0.00284 mol) was added to a stirring solution of intermediate 7 (0.00569 mol) in dry $CH_3CN$, p.a. (35 ml) under $N_2$ and then the solution was stirred and refluxed for 24 hours. The reaction mixture was left to stand over the weekend and the solvent was evaporated. The residue was purified by column chromatography over silica gel (eluent gradient: $CH_2Cl_2/CH_3OH$ 100/0 -> 99.5/0.5 -> 99/1). The product fractions were collected and the solvent was evaporated. Yield: 1.1 g of fraction 1. A part (0.55 g) of this fraction was purified by reversed phase high-performance liquid chromatography ($CH_3CN$). The product fractions were collected and the organic solvent was evaporated. The aqueous concentrate was extracted with $CH_2Cl_2$ and the separated organic layer was evaporated. Yield: 0.12 g of intermediate 8.

Example A4

a. Preparation of intermediate 9

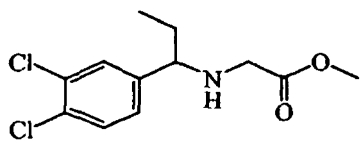

A solution of intermediate 2 (prepared according to A1.b) (0.0748 mol) and chloro acetic acid methyl ester (0.08 mol) in DMF, p.a., dried on molecular sieves, (150 ml) was stirred at room temperature under $N_2$ and $Et_3N$ (0.224 mol) was slowly added, then the reaction mixture was stirred for 20 hours at room temperature and extra chloro acetic acid methyl ester (3.3 ml) was added. The mixture was stirred for another 20 hours at room temperature and again extra chloro acetic acid methyl ester (2 ml) was added. The resulting mixture was stirred for 24 hours and then the solids were filtered off and washed with DMF. $Et_2O$ (800 ml) was added and the mixture was washed 3 times with $H_2O$ (500 ml). The organic layer was separated, dried ($MgSO_4$), filtered off and the solvent was evaporated, then co-evaporated with toluene. The residual oil (23.4 g) was filtered over silica gel (eluent: $CH_2Cl_2/CH_3OH$ 99/1). The product fractions were collected and the solvent was evaporated, finally co-evaporated with toluene. Yield: 20.6 g of intermediate 9 (99.7 %).

b. Preparation of intermediate 10

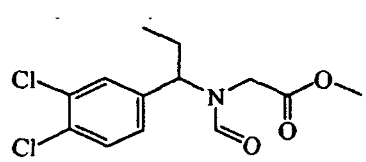

A solution of formic acid (7.5 ml) and intermediate 9 (0.0746 mol) in xylene, p.a. (225 ml) was stirred and refluxed for 4 hours and then the reaction mixture was allowed to reach room temperature. The mixture was washed 2 times with $H_2O$ (2 x 200 ml), with a saturated aqueous $NaHCO_3$ solution (200 ml) and with brine (200 ml), then the separated organic layer was dried ($MgSO_4$) and filtered off. Finally, the solvent was evaporated. Yield: 21.3 g of intermediate 10 (93.9 %)

Example A5

a. Preparation of intermediate 11 and 20

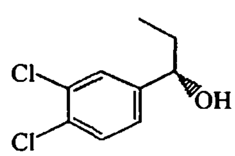

(S) (-)
Intermediate 11

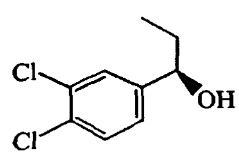

(R) (+)
Intermediate 20

A mixture of *N,N'*-(1R,2R)-1,2-cyclohexanediylbis[1,1,1-trifluoromethanesulfonamide] (0.005 mol) and Ti (*i*-PrO)$_4$ (0.030 mol) in toluene (q.s.) was degassed and placed under Ar-flow, then the reaction mixture was stirred for 20 minutes at 40°C and cooled to -78°C. $Et_2Zn$ (0.030 mol) was added dropwise and after 20 minutes, a mixture of 3,4-dichlorobenzaldehyde (0.0250 mol) in toluene (q.s.) was added dropwise. The reaction mixture was allowed to reach 0°C. The mixture was stirred overnight at room temperature, then quenched with HCl (2N). This mixture was extracted with $CH_2Cl_2$. The separated organic layer was washed, dried, filtered and the solvent evaporated. The residue was purified by column chromatography over silica gel (eluent $CH_2Cl_2$ /CH3OH 98/2). The product fractions were collected and the solvent was evaporated. Yield: 5.1 g of intermediate 11.
Intermediate 20 can be prepared by the above reaction by using *N,N'*-(1S,2S)-1,2-cyclohexanediylbis[1,1,1-trifluoromethanesulfonamide] as catalyst.

b-1). Preparation of intermediate 12

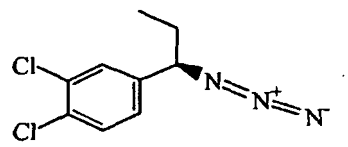

(R)

A mixture of intermediate 11 (0.025 mol; prepared according to A5.a) and diphenylphosphoryl azide (0.030 mol) in toluene (50 ml) was stirred at 0˚C and 2,3,4,6,7,8,9,10-octahydropyrimido[1,2-a]azepine (0.030 mol) was added. The reaction mixture was stirred for 2 hours at 0˚C, then stirred overnight at room temperature. The mixture was diluted with water and toluene. The organic layer was separated, washed once with water, once with 5% HCl, and the solvent was evaporated, yielding intermediate 12 (quantitative yield; used in next reaction step).

b-2). Preparation of intermediate 21

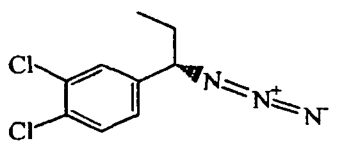

(S)

A mixture of intermediate 20 (prepared according to A5.a) (0.127 mol) and diphenylphosphoryl azide (0.153 mol) in toluene (q.s.) was stirred at 0˚C. 2,3,4,6,7,8,9,10-octahydropyrimido[1,2-a]azepine (0.153 mol) was added dropwise and the reaction mixture was stirred for 1 hour at 0˚C, then for 2 hours at room temperature, then for 3 hours at 50˚C. The reaction mixture was cooled, washed with water, with 0.5 M HCl, with water, dried, filtered and the solvent was evaporated. The residue was purified by flash column chromatography over silica gel (eluent: $CH_2Cl_2/CH_3OH$ 99.5/0.5). The product fractions were collected and the solvent was evaporated. Yield: 23.5 g of intermediate 21.

c-1. Preparation of intermediate 13

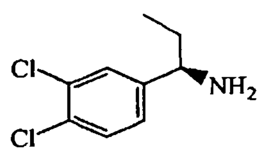

(R)

A mixture of intermediate 12 (0.025 mol; prepared according to A5.b-1), triphenylphosphine (0.027 mol) in THF (70 ml) and $H_2O$ (20 ml) was stirred overnight at room temperature. The solvent was evaporated. The residue was treated with 10% HCl. The acidic layer was washed with DIPE, then alkalized, followed by an extraction with $CH_2Cl_2$. The separated organic layer was dried, filtered and the solvent evaporated. The residue was purified by column chromatography over silica gel. The product fractions were collected and the solvent was evaporated. Yield: 1.1 g of intermediate 13.

c-2. Preparation of intermediate 22

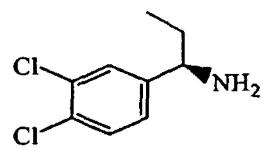

(S)

A mixture of intermediate 21 (prepared according to A5.b-2) (0.122 mol; 23.5 g) in methanol (q.s.) was hydrogenated at 50°C with Pt/C 5% (5 g) as a catalyst. After uptake of $H_2$, the catalyst was filtered off and the filtrate was evaporated. Yield: 21 g of intermediate 22.

d. Preparation of intermediate 14

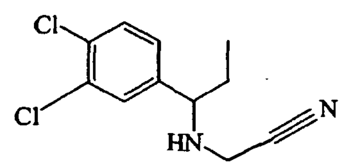

A solution of intermediate 3 (prepared according to A1.b) (0.0116 mol) in $Et_3N$ (0.013 mol) and DMF, p.a., dried on molecular sieves, (20 ml) was stirred on an ice bath. A solution of chloro acetonitrile (0.0128 mol) in DMF, p.a., dried on molecular sieves, (2.5ml) was added dropwise. The reaction mixture was stirred at room temperature for 6 hours. More chloro acetonitrile (0.0063 mol) in DMF, p.a., dried on molecular sieves, (1 ml) was added dropwise. The reaction mixture was stirred for another 24 hours. More chloro acetonitrile (0.0063 mol) in DMF, p.a., dried on molecular sieves, (1 ml) was added dropwise and the reaction mixture was stirred for another 24 hours. More $Et_3N$ (1 ml) was added, then more chloro acetonitrile (0.0079 mol) in DMF, p.a., dried on molecular sieves, (1 ml) was added dropwise. The reaction mixture was stirred for 20 hours. The precipitate was filtered off. The filtrate was poured out into $Et_2O$ (200 ml) and washed with $H_2O/NaHCO_3$ (10 %;100 ml) and $H_2O$ (2x). The separated organic layer was dried ($MgSO_4$), filtered and the solvent was evaporated and co-evaporated with toluene. The residue was purified over silica gel (eluent : $CH_2Cl_2$/MeOH 99:1). The desired fractions were collected and the solvent was evaporated and coevaporated with toluene. Yield : 2.3g of intermediate 14 (81.6%).

e. Preparation of intermediate 15

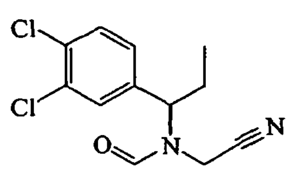

A mixture of intermediate 14 (0.00946 mol) and n-butyl formate (15 ml) was stirred and refluxed for 4 days. The solvent was evaporated, then co-evaporated with toluene. Yield: 2.68 g of intermediate 15.

Example A6

a. Preparation of

intermediate 16 and 17

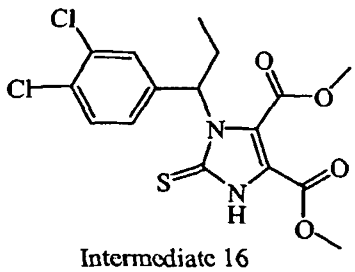

Intermediate 16

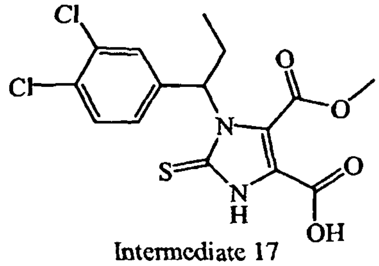

Intermediate 17

A solution of intermediate 10 (prepared according to A4.b) (0.0618 mol) and dimethyl ethanedioic acid ester (0.11 mol) in THF,p.a., dried on molecular sieves, (100 ml) was stirred under $N_2$-atmosphere, then 2-methyl-2-propanol sodium salt (0.066 mol) was added and the reaction mixture was stirred at room temperature for 18 hours and another 24 hours. Finally the mixture was stirred at 60˚C for 4 hours. Extra 2-methyl-2-propanol sodium salt (4 g) and extra dimethyl ethanedioic acid ester (6 g) were added and the reaction mixture was stirred over the weekend at room temperature. The solvent was evaporated, the residue was dissolved in $H_2O$ (250 ml) and washed 2 times with $Et_2O$. The aqueous layer was separated and $CH_3OH$ (200 ml), potassium thiocyanic acid salt (10 g) and HCl (36%, p.a.) (q.s.) were added, then the mixture was stirred for 18 hours at 60˚C. The solvent was partly evaporated and the concentrate was extracted with $CH_2Cl_2$. The organic layer was separated, dried ($MgSO_4$), filtered and the solvent was evaporated. The residue (5 g) was purified by filtration over silica gel (eluent: $CH_2Cl_2/CH_3OH$ 99/1). The product fractions were collected and the solvent was evaporated. The residue was triturated under Hexane, filtered off, washed , then dried (vacuum, 50˚C). Yield: 5.2 g of intermediate 16. The fractions containing a side-product were combined and the solvent was evaporated, then co-evaporated with hexane/DIPE. The residue was stirred in $Et_2O$/Hexane and the resulting precipitate was filtered off, washed with hexane, then dried (vacuum, 50˚C). Yield: 0.28 g of intermediate 17.

b. Preparation of intermediate 18

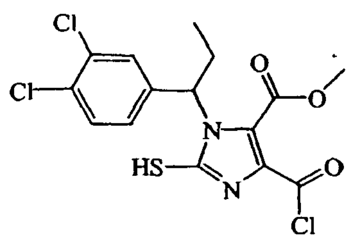

A mixture of intermediate 17 (0.000257 mol) in thionyl chloride (5 ml) was stirred overnight at room temperature, then the solvent was evaporated and co-evaporated with toluene (p.a.), yielding intermediate 18 used in the next step.

c. Preparation of intermediate 19

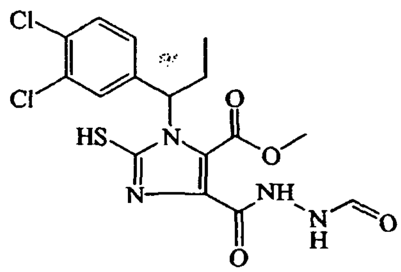

A solution of intermediate 18 (0.000257 mol) in THF,p.a., dried on molecular sieves, (10 ml) was stirred under $N_2$ and then hydrazinecarboxaldehyde (0.0125 mol) was added. The reaction mixture was stirred at room temperature for 1 hour and the solvent was evaporated. The residue was stirred in $H_2O$ (10 ml) and $CH_2Cl_2/CH_3OH$ (15 ml, 95/5). The organic layer was separated, washed with HCl (1N), dried ($MgSO_4$), filtered off and the solvent was evaporated, then co-evaporated with toluene. Yield: 800 mg of intermediate 19.

Example A7

a-1. Preparation of intermediate 24

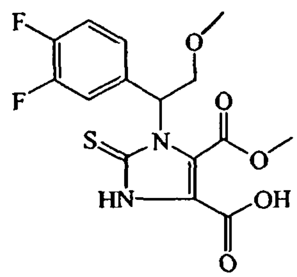

A mixture of

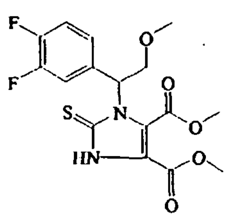

(intermediate 23 prepared according to A6.a) (0.0015 mol) and 1N NaOH (0.030 mol) in $H_2O$ (20 ml) and methanol (50 ml) was stirred for 20 hours at room temperature, then extra 1N NaOH (10 ml) was added and the reaction mixture was stirred for 5 hours at room temperature. Half of the solvent was evaporated and 1N HCl (40 ml) was added and the precipitated product was extracted with $CH_2Cl_2$. The organic layer was separated, dried, filtered off and the solvent was evaporated. Yield: 5.6 g of intermediate 24.

a-2. Preparation of intermediate 28

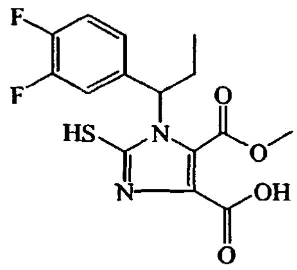

A mixture of

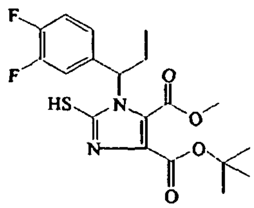

(intermediate 27 prepared according to A6.a) (0.0012 mol) in TFA (2 ml) and $CH_2Cl_2$ (70 ml) was stirred for 1 day at room temperature and then the reaction mixture was concentrated, yielding intermediate 28.

b. Preparation of intermediate 25

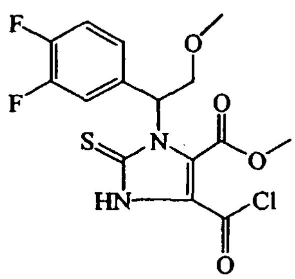

A mixture of intermediate 24 (prepared according to A7.a-1) (0.0075 mol) and $SOCl_2$ (0.015 mol) in $CH_2Cl_2$ (50 ml) was stirred and refluxed on an oil bath for 3 hours and then the solvent was evaporated. The residue was dissolved in toluene and the solvent was evaporated again. Yield: 2.9 g of intermediate 25.

c. Preparation of intermediate 26

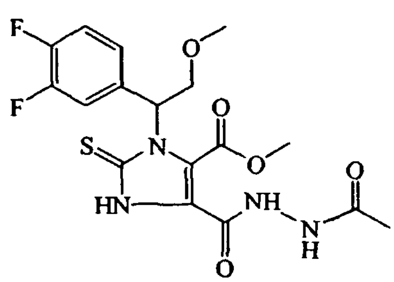

A mixture of hydrazide acetic acid (0.011 mol) and $Et_3N$ (0.015 mol) in THF (30 ml) was stirred and cooled on an ice bath at 0-5°C and then a mixture of intermediate 25 (prepared according to A7.b) (0.0075 mol) in THF (20 ml)

was added dropwise over 30 minutes at 0-5˚C. The reaction mixture was stirred for 1 hour at 0-5˚C and then $H_2O$ was added and the mixture was extracted with $CH_2Cl_2$. The organic layer was separated, dried, filtered off and the solvent was evaporated. The obtained residue was filtered over silica gel (eluent: $CH_2Cl_2/CH_3OH$ 97/3), then the product fractions were collected and the solvent was evaporated. Yield: 1.25 g of intermediate 26.

Example A8

a. Preparation of intermediate 30

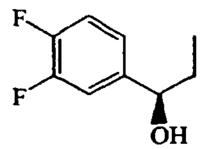

(R)

A solution of *N,N'*-(1S,2S)-1,2-cyclohexanediylbis[1,1,1-trifluoromethanesulfonamide] (catalytic quantity) and tetrakis(2-propanolato)titanium (0.415 mol) in toluene (p.a) (500 ml) was degassed under Argon and then stirred for 20 minutes at 1 40 ˚C. This mixture was cooled on a 2-propanol/$CO_2$ bath to -78 ˚C and diethylzinc (0.415 mol) was added dropwise over 15 minutes. The resulting mixture was stirred for 15 minutes at -78 ˚C and then a solution of 3,4-difluorobenzaldehyde (0.345 mol) in toluene (p.a.) (q.s.) was added dropwise over 20 minutes. The reaction mixture was stirred further for 30 minutes at -78 ˚C and was then allowed to slowly reach 0 ˚C. The mixture was quenched with 1N HCl and extracted with $CH_2Cl_2$. Both organic layer and aqueous layer were filtered over dicalite and the organic layer in the obtained filtrate was separated, then washed with $H_2O$, dried ($MgSO_4$) and filtered off. Finally, the solvent was evaporated. Yield: 57.7 g of intermediate 30.

b. Preparation of intermediate 31

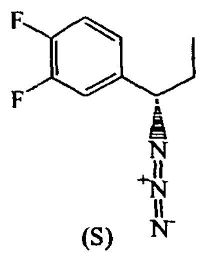

(S)

A solution of intermediate 30 (0.331 mol) and diphenylphosphoryl azide (0.4 mol) in toluene (p.a.) (500 ml) was stirred under $N_2$ on an ice bath at 0 ˚C, then DBU (0.4 mol) was added dropwise and the reaction mixture was stirred for 1 hour at 0 ˚C. The mixture was allowed to reach room temperature and was then stirred for 1 hour at 45 ˚C and overnight at room temperature. The resulting mixture was poured out into $H_2O$ (500 ml) and extracted with $Et_2O$ (500 ml). The organic layer was separated, dried ($MgSO_4$), filtered off and the solvent was evaporated. The residue was filtered over silica gel (eluent: $CH_2Cl_2$ 100 %). The product fractions were collected and then the solvent was evaporated and co-evaporated with Toluene. Yield: 57.2 g of intermediate 31.

c. Preparation of intermediate 32

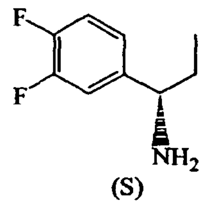

(S)

A mixture of intermediate 31 (0.29 mol) in $CH_3OH$ (600 ml) was hydrogenated with Pd/C 10% (2 g) as a catalyst. After uptake of $H_2$ (1 equivalent), the catalyst was filtered off and the filtrate was evaporated. The residue was dissolved in $CH_2Cl_2$ (300 ml) and then 1N HCl was slowly added while the solution was stirred vigorously on an ice bath. After this extraction, the aqueous layer was alkalised with a 50 % NaOH solution to pH > 10 and the resulting mixture was extracted with $CH_2Cl_2$. The organic layer was separated, dried ($MgSO_4$), filtered off and the solvent was evaporated. Yield: 40.0 g of intermediate 32.

d. Preparation of intermediate 33

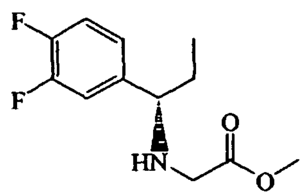

(S)

Brome acetic acid methyl ester (0.28 mol) was added to a stirring solution of intermediate 32 (0.233 mol) and *N*-ethyl-*N*-(1-methylethyl)-2-propanamine (0.466 mol) in THF (350 ml) and the reaction mixture was stirred for 5 days at room temperature. The resulting precipitate was filtered off, washed with THF and then the filtrate was evaporated. The obtained residue was stirred in $CH_2Cl_2$ and then washed with a half saturated aqueous $NaHCO_3$ solution and with water. The organic layer was separated, dried ($MgSO_4$), filtered off and then the solvent was evaporated and co-evaporated with toluene. Yield: intermediate 33.

e. Preparation of intermediate 34

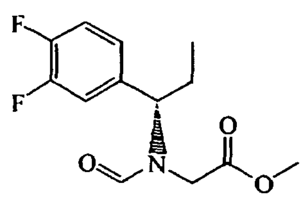

(S)

Formic acid (23 ml) was added to a stirring solution of intermediate 33 (0.23 mol) in xylene (p.a) (400 ml), then the reaction mixture was stirred and refluxed for 4 hours with a Dean-Stark apparatus. The mixture was allowed to reach room temperature and the solvent was evaporated. The residue was filtered over silica gel (eluent: $CH_2Cl_2/CH_3OH$ 99/1). The product fractions with a purity > 95 % were collected and then the solvent was evaporated and co-evaporated with toluene. Yield: 15.2 g of intermediate 34.

Example A9

a. Preparation of intermediate 35

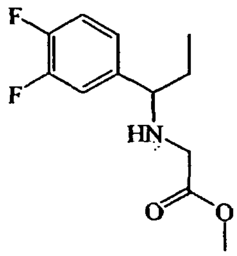

Following procedure was done 3 x. [A mixture of 1-(3,4-difluorophenyl)-1-propanone (0.43 mol), glycine methyl ester, hydrochloride (0.5 mol) and KF (0.43 mol) in $CH_3OH$ (700 ml) was hydrogenated at 50 °C (in Parr apparatus) with Pd/C 10% (5 g) as a catalyst in the presence of thiophene solution (2 ml). After uptake of $H_2$ (1 equivalent) was complete, the catalyst was filtered off and the filtrate was evaporated. The residue was stirred in water, then treated with $NaHCO_3$ (q.s.) and the product was extracted with $CH_2Cl_2$. The organic layer was separated, dried, filtered and the solvent evaporated. The residue was dissolved in 2-propanol (1000 ml) and converted into the hydrochloric acid salt with 6 N HCl/2-propanol. The precipitate was filtered off, washed with DIPE (to remove the 2-propanol), then stirred in water. $CH_2Cl_2$ was added and the mixture was treated with $K_2CO_3$ (q.s.). The layers were separated. The organic layer was dried, filtered and the solvent evaporated.] Yield: 221 g of intermediate 35.

b. Preparation of intermediate 36

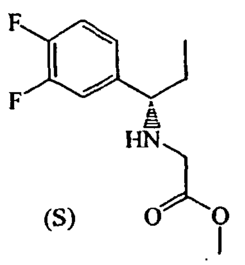

(S)

.[S-(R*,R*)]-2,3-bis[(4-methylbenzoyl)oxy] butanedioic acid

A mixture of intermediate 35 (0.9 mol) in 2-propanol (1500 ml) was stirred at room temperature. [S-(R*,R*)]-2,3-bis[(4-methylbenzoyl)oxy]-butanedioic acid (0.9 mol) was added in one portion. The reaction mixture was stirred for 2 hours at room temperature. The precipitate was filtered off and dissolved in refluxing 2-propanol (1500 ml). Then the mixture was stirred at room temperature, allowing the compound to precipitate again. The precipitate was filtered off, then re-dissolved in boiling 2-propanol. The mixture was allowed to cool to room temperature and the resulting precipitate was filtered off and dried. Yield: 231.4 g of intermediate 36 (96% (S)).

c. Preparation of intermediate 37

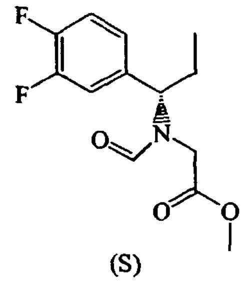

(S)

Intermediate 36 (370 g, 0.588 mol) was converted into the free base upon addition of $NH_4OH/CH_2Cl_2$. The product was extracted to give free base residue A*. A mixture of A* (132 g, 0.543 mol) in formic acid (150 ml) and xylene (750 ml) was stirred and refluxed for 5 hours at 120 °C. The solvent was evaporated. The residue was stirred in water, treated with $NaHCO_3$ and then the product was extracted with $CH_2Cl_2$. The organic layer was separated, dried, filtered and the solvent evaporated. Yield: 146 g of intermediate 37 used in next reaction step, without further purification).

Table 1 lists intermediates of formula (IX) which can be prepared according to one of the above examples (Ex.No.).

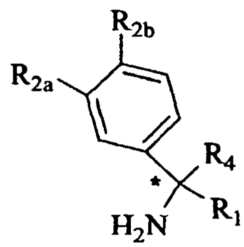

| Interm. | Ex.No. | $R_{2a}$ | $R_{2b}$ | $R_1$ | $R_4$ | Stereochem descriptor * |
|---|---|---|---|---|---|---|
| 1.1 | A1b | Cl | Cl | H | H | |
| 1.2 | A1b | Cl | Cl | $CH_3$ | H | (RS) |
| 1.3 | A5c-2) | Cl | Cl | $CH_3$ | H | (S) |
| 3 | A1b | Cl | Cl | $CH_2CH_3$ | H | (RS) |
| 22 | A5c-2) | Cl | Cl | $CH_2CH_3$ | H | (S) |
| 1.4 | A1b | Cl | Cl | $CH_2CH_2CH_3$ | H | (RS) |
| 1.5 | A5c-2) | Cl | Cl | $CH_2CH_2CH_3$ | H | (S) |
| 1.6 | A1b | Cl | Cl | phenyl | H | (RS) |
| 1.7 | A1b | Cl | Cl | 3,4-dichlorophenyl | H | (RS) |
| 1.8 | A1b | Cl | Cl | cyclohexyl | H | (RS) |
| 1.9 | A1b | Cl | Cl | cyclopropyl | H | (RS) |
| 1.10 | A1b | Cl | Cl | $CH_2$-$N(CH_3)_2$ | H | (RS) |
| 1.11 | A1b | Cl | Cl | $CH_2$-O-$CH_3$ | H | (RS) |
| 1.12 | A1b | Cl | Cl | 2-thienyl | H | (RS) |
| 2.1 | A1b | Cl | H | H | H | |
| 2.2 | A1b | Cl | H | $CH_3$ | H | (RS) |
| 2.3 | A5c-2) | Cl | H | $CH_3$ | H | (S) |
| 2.4 | A1b | Cl | H | $CH_2CH_3$ | H | (RS) |

(continued)

| Interm. | Ex.No. | $R_{2a}$ | $R_{2b}$ | $R_1$ | $R_4$ | Stereochem descriptor * |
|---|---|---|---|---|---|---|
| 2.5 | A5c-2) | Cl | H | $CH_2CH_3$ | H | (S) |
| 2.6 | A1b | Cl | H | $CH_2CH_2CH_3$ | H | (RS) |
| 2.7 | A5c-2) | Cl | H | $CH_2CH_2CH_3$ | H | (S) |
| 2.8 | A1b | Cl | H | phenyl | H | (RS) |
| 2.9 | A1b | Cl | H | 3,4-dichlorophenyl | H | (RS) |
| 2.10 | A1b | Cl | H | cyclohexyl | H | (RS) |
| 2.11 | A1b | Cl | H | cyclopropyl | H | (RS) |
| 2.12 | A1b | Cl | H | $CH_2$-$N(CH_3)_2$ | H | (RS) |
| 2.13 | A1b | Cl | H | $CH_2$-O-$CH_3$ | H | (RS) |
| 2.14 | A1b | Cl | H | 2-thienyl | H | (RS) |
| 3.1 | A1b | H | Cl | H | H | |
| 3.2 | A1b | H | Cl | $CH_3$ | H | (RS) |
| 3.3 | A5c-2) | H | Cl | $CH_3$ | H | (S) |
| 3.4 | A1b | H | Cl | $CH_2CH_3$ | H | (RS) |
| 3.5 | A5c-2) | H | Cl | $CH_2CH_3$ | H | (S) |
| 3.6 | Alb | H | Cl | $CH_2CH_2CH_3$ | H | (RS) |
| 3.7 | A5c-2) | H | Cl | $CH_2CH_2CH_3$ | H | (S) |
| 3.8 | A1b | H | Cl | phenyl | H | (RS) |
| 3.9 | A1b | H | Cl | 3,4-dichlorophenyl | H | (RS) |
| 3.10 | A1b | H | Cl | cyclohexyl | H | (RS) |
| 3.11 | A1b | H | Cl | cyclopropyl | H | (RS) |
| 3.12 | A1b | H | Cl | $CH_2$-$N(CH_3)_2$ | H | (RS) |
| 3.13 | A1b | H | Cl | $CH_2$-O-$CH_3$ | H | (RS) |
| 3.14 | A1b | H | Cl | 2-thienyl | H | (RS) |
| 4.1 | A1b | $CH_3$ | $CH_3$ | H | H | |
| 4.2 | A1b | $CH_3$ | $CH_3$ | $CH_3$ | H | (RS) |
| 4.3 | A5c-2) | $CH_3$ | $CH_3$ | $CH_3$ | H | (S) |
| 4.4 | A1b | $CH_3$ | $CH_3$ | $CH_2CH_3$ | H | (RS) |
| 4.5 | A5c-2) | $CH_3$ | $CH_3$ | $CH_2CH_3$ | H | (S) |
| 4.6 | A1b | $CH_3$ | $CH_3$ | $CH_2CH_2CH_3$ | H | (RS) |
| 4.7 | A5c-2) | $CH_3$ | $CH_3$ | $CH_2CH_2CH_3$ | H | (S) |
| 4.8 | A1b | $CH_3$ | $CH_3$ | phenyl | H | (RS) |
| 4.9 | A1b | $CH_3$ | $CH_3$ | 3,4-dichlorophenyl | H | (RS) |
| 4.10 | A1b | $CH_3$ | $CH_3$ | cyclohexyl | H | (RS) |
| 4.11 | A1b | $CH_3$ | $CH_3$ | cyclopropyl | H | (RS) |
| 4.12 | A1b | $CH_3$ | $CH_3$ | $CH_2$-$N(CH_3)_2$ | H | (RS) |
| 4.13 | A1b | $CH_3$ | $CH_3$ | $CH_2$-O-$CH_3$ | H | (RS) |

(continued)

| Interm. | Ex.No. | $R_{2a}$ | $R_{2b}$ | $R_1$ | $R_4$ | Stereochem descriptor * |
|---|---|---|---|---|---|---|
| 4.14 | A1b | $CH_3$ | $CH_3$ | 2-thienyl | H | (RS) |
| 5.1 | A1b | $CH_3O$ | $CH_3O$ | H | H | |
| 5.2 | A1b | $CH_3O$ | $CH_3O$ | $CH_3$ | H | (RS) |
| 5.3 | A5c-2) | $CH_3O$ | $CH_3O$ | $CH_3$ | H | (S) |
| 5.4 | A1b | $CH_3O$ | $CH_3O$ | $CH_2CH_3$ | H | (RS) |
| 5.5 | A5c-2) | $CH_3O$ | $CH_3O$ | $CH_2CH_3$ | H | (S) |
| 5.6 | A1b | $CH_3O$ | $CH_3O$ | $CH_2CH_2CH_3$ | H | (RS) |
| 5.7 | A5c-2) | $CH_3O$ | $CH_3O$ | $CH_2CH_2CH_3$ | H | (S) |
| 5.8 | A1b | $CH_3O$ | $CH_3O$ | phenyl | H | (RS) |
| 5.9 | A1b | $CH_3O$ | $CH_3O$ | 3,4-dichlorophenyl | H | (RS) |
| 5.10 | A1b | $CH_3O$ | $CH_3O$ | cyclohexyl | H | (RS) |
| 5.11 | A1b | $CH_3O$ | $CH_3O$ | cyclopropyl | H | (RS) |
| 5.12 | A1b | $CH_3O$ | $CH_3O$ | $CH_2$-$N(CH_3)_2$ | H | (RS) |
| 5.13 | A1b | $CH_3O$ | $CH_3O$ | $CH_2$-O-$CH_3$ | H | (RS) |
| 5.14 | A1b | $CH_3O$ | $CH_3O$ | 2-thienyl | H | (RS) |
| 6.1 | A1b | F | F | H | H | |
| 6.2 | A1b | F | F | $CH_3$ | H | (RS) |
| 6.3 | A5c-2) | F | F | $CH_3$ | H | (S) |
| 6.4 | A1b | F | F | $CH_2CH_3$ | H | (RS) |
| 6.5 | A5c-2)/A8c | F | F | $CH_2CH_3$ | H | (S) |
| 6.6 | A1b | F | F | $CH_2CH_2CH_3$ | H | (RS) |
| 6.7 | A5c-2) | F | F | $CH_2CH_2CH_3$ | H | (S) |
| 6.8 | A1b | F | F | phenyl | H | (RS) |
| 6.9 | A1b | F | F | 3,4-dichlorophenyl | H | (RS) |
| 6.10 | A1b | F | F | cyclohexyl | H | (RS) |
| 6.11 | A1b | F | F | cyclopropyl | H | (RS) |
| 6.12 | A1b | F | F | $CH_2$-$N(CH_3)_2$ | H | (RS) |
| 6.13 | A1b | F | F | $CH_2$-O-$CH_3$ | H | (RS) |
| 6.14 | A1b | F | F | 2-thienyl | H | (RS) |
| 7.1 | A1b | Br | Br | H | H | |
| 7.2 | A1b | Br | Br | $CH_3$ | H | (RS) |
| 7.3 | A5c-2) | Br | Br | $CH_3$ | H | (S) |
| 7.4 | A1b | Br | Br | $CH_2CH_3$ | H | (RS) |
| 7.5 | A5c-2) | Br | Br | $CH_2CH_3$ | H | (S) |
| 7.6 | A1b | Br | Br | $CH_2CH_2CH_3$ | H | (RS) |
| 7.7 | A5c-2) | Br | Br | $CH_2CH_2CH_3$ | H | (S) |
| 7.8 | A1b | Br | Br | phenyl | H | (RS) |

(continued)

| Interm. | Ex.No. | $R_{2a}$ | $R_{2b}$ | $R_1$ | $R_4$ | Stereochem descriptor * |
|---|---|---|---|---|---|---|
| 7.9 | A1b | Br | Br | 3,4-dichlorophenyl | H | (RS) |
| 7.10 | A1b | Br | Br | cyclohexyl | H | (RS) |
| 7.11 | A1b | Br | Br | cyclopropyl | H | (RS) |
| 7.12 | A1b | Br | Br | $CH_2$-$N(CH_3)_2$ | H | (RS) |
| 7.13 | A1b | Br | Br | $CH_2$-O-$CH_3$ | H | (RS) |
| 7.14 | A1b | Br | Br | 2-thienyl | H | (RS) |
| 8.1 | A1b | F | $CF_3$ | H | H | |
| 8.2 | A1b | F | $CF_3$ | $CH_3$ | H | (RS) |
| 8.3 | A5c-2) | F | $CF_3$ | $CH_3$ | H | (S) |
| 8.4 | A1b | F | $CF_3$ | $CH_2CH_3$ | H | (RS) |
| 8.5 | A5c-2) | F | $CF_3$ | $CH_2CH_3$ | H | (S) |
| 8.6 | A1b | F | $CF_3$ | $CH_2CH_2CH_3$ | H | (RS) |
| 8.7 | A5c-2) | F | $CF_3$ | $CH_2CH_2CH_3$ | H | (S) |
| 8.8 | A1b | F | $CF_3$ | phenyl | H | (RS) |
| 8.9 | A1b | F | $CF_3$ | 3,4-dichlorophenyl | H | (RS) |
| 8.10 | A1b | F | $CF_3$ | cyclohexyl | H | (RS) |
| 8.11 | A1b | F | $CF_3$ | cyclopropyl | H | (RS) |
| 8.12 | A1b | F | $CF_3$ | $CH_2$-$N(CH_3)_2$ | H | (RS) |
| 8.13 | A1b | F | $CF_3$ | $CH_2$-O-$CH_3$ | H | (RS) |
| 8.14 | A1b | F | $CF_3$ | 2-thienyl | H | (RS) |
| 9.1 | A1b | $CF_3$ | F | H | H | |
| 9.2 | A1b | $CF_3$ | F | $CH_3$ | H | (RS) |
| 9.3 | A5c-2) | $CF_3$ | F | $CH_3$ | H | (S) |
| 9.4 | A1b | $CF_3$ | F | $CH_2CH_3$ | H | (RS) |
| 9.5 | A5c-2) | $CF_3$ | F | $CH_2CH_3$ | H | (S) |
| 9.6 | A1b | $CF_3$ | F | $CH_2CH_2CH_3$ | H | (RS) |
| 9.7 | A5c-2) | $CF_3$ | F | $CH_2CH_2CH_3$ | H | (S) |
| 9.8 | A1b | $CF_3$ | F | phenyl | H | (RS) |
| 9.9 | A1b | $CF_3$ | F | 3,4-dichlorophenyl | H | (RS) |
| 9.10 | A1b | $CF_3$ | F | cyclohexyl | H | (RS) |
| 9.11 | A1b | $CF_3$ | F | cyclopropyl | H | (RS) |
| 9.12 | A1b | $CF_3$ | F | $CH_2$-$N(CH_3)_2$ | H | (RS) |
| 9.13 | A1b | $CF_3$ | F | $CH_2$-O-$CH_3$ | H | (RS) |
| 9.14 | A1b | $CF_3$ | F | 2-thienyl | H | (RS) |

B. Preparation of the final compounds

Example B1

Preparation of compound 1

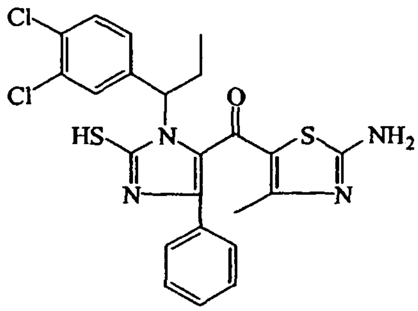

1N NaOH (0.006 mol) was added to a solution of intermediate 4 (prepared according to A2.a) (0.0026 mol) in ethanol, p.a. (10 ml), then the solution was stirred at 50°C for 1 hour and was allowed to reach room temperature. The reaction mixture was poured out into $H_2O$ (30 ml) and 1N HCl (7 ml) was slowly added. The resulting precipitate was filtered off, washed with $H_2O$ and dried (vacuum) at 60°C. A part (0.68 g of 0.78 g) of the crude product was purified by high-performance liquid chromatography (eluent: (0.5 % $NH_4OAc$ in $H_2O$)/$CH_3CN$ 90/10). The product fractions were collected and the organic solvent was evaporated. The aqueous concentrate was extracted with $CH_2Cl_2$, then the organic layer was separated, dried ($MgSO_4$) and filtered off. Finally, the solvent was evaporated. Yield: 0.24 g of compound 1.

Example B2

a. Preparation of compound 2

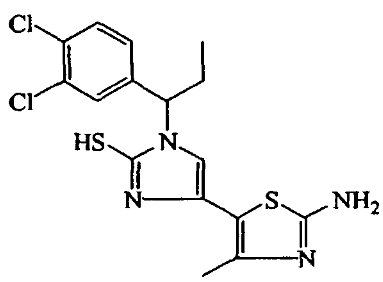

.HCl

A solution of potassium thiocyanic acid salt (0.00285 mol) in $H_2O$ (10 ml) was added to a stirring solution of intermediate 5 (prepared according to A2.b) (0.00095 mol) in methanol (10 ml), then 36% HCl, p.a. (0.00475 mol) was added and the reaction mixture was stirred for 4 hours at 55°C. Extra potassium thiocyanic acid salt (0.1 g) was added and the mixture was stirred for 18 hours at 55°C. The resulting mixture was allowed to reach room temperature and was left to stand for 1 hour. The formed precipitate was filtered off, washed with $CH_3OH/H_2O$ (1/1) and dried (vacuum) at 60°C. Yield: 0.16 g of compound 2 (m.p.: 301.8-303.3°C)

b-1. Preparation of compound 3

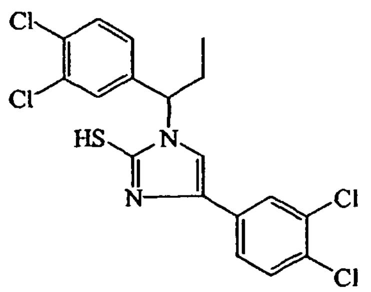

$H_2O$ (2.5 ml), followed by potassium thiocyanic acid salt (0.00328 mol) and then concentrated HCl (0.5 ml) were added to a solution of intermediate 8 (prepared according to A3.c) (0.0013 mol) in methanol (15 ml) and the reaction mixture was stirred at room temperature for 20 hours, then the mixture was stirred at 55°C for 48 hours and extra concentrated HCl (0.75 ml) was added. The resulting mixture was stirred at 60°C for 24 hours, $H_2O$ was added and the mixture was extracted with $CH_2Cl_2$. The organic layer was separated, dried ($MgSO_4$), filtered off and the solvent was evaporated. The residue was purified by reversed phase high-performance liquid chromatography ($NH_4OAc$ 10 % $CH_3CN$). The product fractions were collected and half of the solvent was evaporated. The resulting precipitate was washed with $H_2O$ and dried (vacuum) at 50°C. Yield: 0.061 g of compound 3.

b-2. Preparation of compound 4

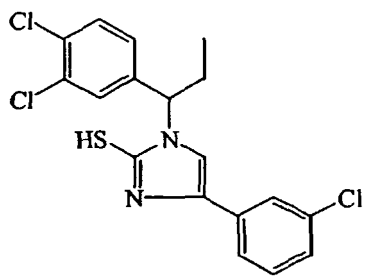

Potassium thiocyanic acid salt (0.000314 mol) and then HCl, 1N (0.00086 mol) were added to a solution of

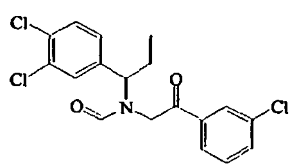

(prepared according to A3.c) (0.000286 mol) in methanol, p.a. (3 ml) and the reaction mixture was stirred at room temperature for 4 hours. HCl (0.5 ml, concentrated) was added and the mixture was stirred at 55°C for 18 hours, then extra potassium thiocyanic acid salt (0.000515 mol) was added and the reaction mixture was stirred at 55°C for 7 hours. The mixture was left to stand for 18 hours, $H_2O$ (15 ml) was added and the product was extracted with $CH_2Cl_2$. The organic layer was separated and evaporated. The residue was purified by Fast high-performance liquid chromatography using the Reversed Phase-method. The product fractions were collected and the organic volatiles were evaporated. The product was extracted with $CH_2Cl_2$ and the separated organic layer was evaporated. Yield: 0.005 g of compound 4.

Example B3

a. Preparation of compound 5

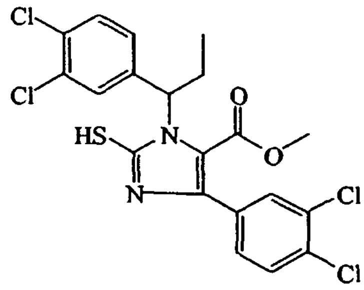

Intermediate 10 (prepared according to A4.b) (0.002 mol) was added at 0˚C to a mixture of *N*-(1-methylethyl)-2-propanamine lithium salt (0.0025 mol) in THF (40 ml) for 30 minutes and 3,4-dichlorobenzoyl chloride (0.0033 mol) was added, then the reaction mixture was reacted for 1 hour and quenched with water. The mixture was extracted with EtOAc, dried and the solvent was evaporated. The residue was diluted with $CH_3OH$ and then concentrated HCl (q.s.) and potassium thiocyanic acid salt (1 g) were added. The resulting mixture was heated at 70˚C for 48 hours and partitioned between water and $CH_2Cl_2$. The organic layer was separated, dried and the solvent was evaporated. The residue was purified by high-performance liquid chromatography.
The product fractions were collected and the solvent was evaporated. Yield: 0.180 g of compound 5.

<u>b. Preparation of compound 6</u>

<u>Method 1</u>

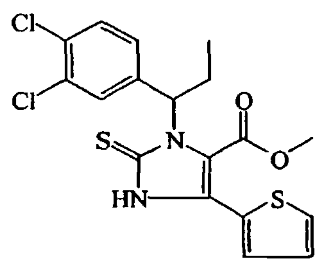

Intermediate 10 (prepared according to A4.b) (0.003 mol, in 10 ml THF) was added dropwise to a cold (-78˚C) solution of *N*-(1-methylethyl)-2-propanamine lithium salt (0.0036 mol, 2M in THF) in THF (5 ml). After 30 minutes 2-thiophenecarbonyl chloride (0.0031 mol) was added and the reaction mixture stood for 1 hour. The mixture was quenched with $NH_4Cl$ and extracted with EtOAc. The organic layer was separated, dried ($MgSO_4$), filtered off and the solvent was evaporated. The residue was diluted with methanol (25 ml), then potassium thiocyanic acid salt (1 g) and concentrated HCl (1 ml) were added. The resulting mixture was heated overnight at 70˚C and the solvent was evaporated. The residue was partitioned between EtOAc and water, then the separated organic layer was dried and the solvent was evaporated. The residue was purified by high-performance liquid chromatography; the product fractions were collected and the solvent was evaporated. Yield: 0.040 g of compound 6.

<u>c. Preparation of compound 6</u>

<u>Method 2</u>

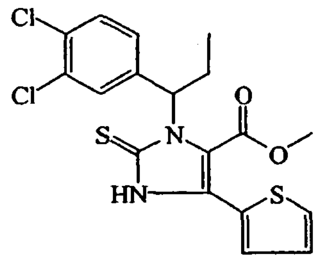

t-BuONa (0.150 g) was added at 0°C to a mixture of intermediate 10 (prepared according to A4.b) (0.00082 mol) in THF (5 ml), then 2-thiophenecarbonyl chloride (0.041 mol) was added and after 30 minutes potassium thiocyanic acid salt (0.250 g) and concentrated HCl (1 ml) were added. The reaction mixture was heated at 70°C for 18 hours and treated with water/$CH_2Cl_2$. The organic layer was separated, dried ($MgSO_4$), filtered off and the solvent was evaporated. The residue was purified by high-performance liquid chromatography, then the product fractions were collected and the solvent was evaporated. The residue was further purified by Flash column chromatography and then by normal phase high-performance liquid chromatography. The product fractions were collected and the solvent was evaporated. Yield: 45 mg of compound 6.

d-1. Preparation of compound 7

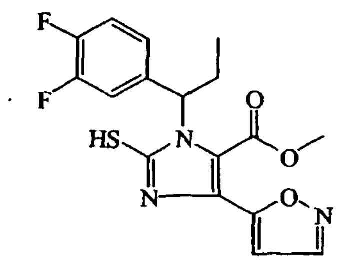

*N*-(1-methylethyl)-2-propanamine lithium salt (0.0184 mol, 2M in THF) was added dropwise at -78°C under $N_2$ to a cold mixture of intermediate 29

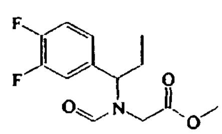

(prepared according to A4.b) (0.00924 mol) in THF (q.s.). After 30 minutes 5-isoxazolecarbonyl chloride (0.0110 mol) was added and then the reaction mixture was allowed to slowly reach room temperature. The mixture was quenched with $NH_4Cl$ and the solvent was evaporated. The residue was diluted with $CH_3OH$, with $H_2O$ and then potassium thiocyanic acid salt (3 g) and concentrated HCl were added. The reaction mixture was heated overnight at 80°C, then cooled, quenched with $K_2CO_3$ and extracted with $CH_2Cl_2$. The organic layer was separated, dried and the solvent was evaporated. The residue was purified by column chromatography over silica gel (gradient eluent: $CH_2Cl_2/CH_3OH$ 100/0 -> 90/10). Two product fractions were collected and the solvent was evaporated, to give Residue (I) and Residue (II). Residue (I) was purified by reversed phase high-performance liquid chromatography. The product fractions were collected and the solvent was evaporated. The residue was diluted with $CH_2Cl_2$ and washed 3 times with 1N HCl. The organic layer was separated, dried and the solvent was evaporated. Yield: 0.290 g of compound 7.

d-2. Preparation of compound 43

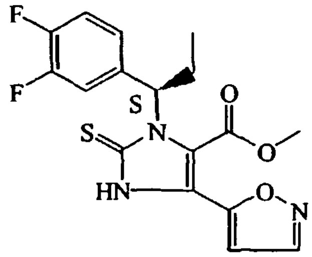

A solution of intermediate 34 (prepared according to A8.e) (0.0169 mol) in THF, p.a., dried on molecular sieves (80 ml) was stirred under $N_2$ on a 2-propanol/$CO_2$ cooling bath, then *N*-(1-methylethyl)-2-propanamine lithium salt (0.0179 mol; 2 M in THF/Heptane) was slowly added dropwise at -78 ˚C and the mixture was further stirred for 5 minutes. A solution of 5-isoxazolecarbonylchloride (0.02 mol) in THF, p.a., dried on molecular sieves (5 ml) was slowly added dropwise and the resulting mixture was stirred for 90 minutes at -78 ˚C and was then allowed to reach 10 ˚C. A solution of KSCN (0.051 mol) in $H_2O$ (30 ml) was added, followed by addition of $CH_3OH$ (40 ml) and then concentrated HCl (14 ml). The reaction mixture was stirred for 4 hours at 60 ˚C and for 18 hours at 70 ˚C, then the mixture was allowed to reach room temperature. The mixture was poured out into $H_2O$ and was extracted with $CH_2Cl_2$. The organic layer was separated, dried ($MgSO_4$), filtered off and the solvent was evaporated. The residue was purified by high-performance liquid chromatography (Reversed Phase-method "$NH_4OAc$ 10 % $CH_3CN$ - $CH_3OH$"). The product fractions were collected, then the organic solvent was evaporated and the aqueous concentrate was extracted with $CH_2Cl_2$. The organic layer was separated, dried ($MgSO_4$), filtered off and the solvent was evaporated. Yield: 0.269 g of final compound 43.

d-3. Preparation of compound 53

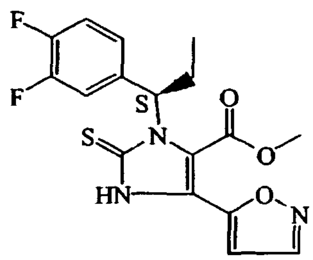

A mixture of intermediate 37 (prepared according to A9.c) (0.54 mol) in THF (1000 ml) was stirred and cooled to -78 ˚C on a $CO_2$/2-propanol-bath. 5-Isoxazolecarbonyl chloride (0.75 mol) was added. Then, 1,1,1-trimethyl-*N*-(trimethylsilyl)-silanamine lithium salt, 1M/THF (0.8 mol, 800 ml) was added over a 30 minutes period. The mixture was stirred for 4 hours at -78 ˚C. Then, the reaction temperature was raised to -20 ˚C. A solution of HCl, concentrated (66 ml) in water (400 ml) was added dropwise over 15 minutes. The organic solvent (THF) was evaporated. Water (400 ml) was added. Then, HCl, concentrated (280 ml) was added, followed by methanol (800 ml), then KSCN (0.8 mol). The reaction mixture was stirred for 22 hours at 60 ˚C. Water was added. The mixture was cooled to room temperature, then treated with $K_2CO_3$ and $NaHCO_3$. This mixture was extracted with $CH_2Cl_2$. The separated organic layer was dried, filtered and the solvent evaporated. The residue (± 200 g) was purified by high-performance liquid chromatography. The product fractions were collected and the solvent was evaporated. The residue was crystallized from DIPE (2 x). The precipitate was filtered off and dried. Yield: 45.5 g of final compound 53 (mp: 128.5 ˚C).

Example B4

Preparation of compound 8

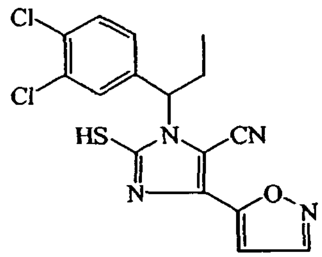

A solution of intermediate 15 prepared according to A5.e) (0.00369 mol) in THF, p.a., dried on molecular sieves, (20 ml) was stirred on a 2-propanol/$CO_2$ cooling-bath and then *N*-(1-methylethyl)-2-propanamine lithium salt (0.0074 mol) was slowly added at -78°C. The resulting mixture was stirred for 15 minutes and 5-isoxazolecarbonyl chloride (0.0037 mol) was slowly added. The mixture was stirred for 10 minutes at -78°C and was then allowed to reach room temperature. A solution of potassium thiocyanic acid salt (0.011 mol) in $H_2O$ (15 ml) was added, followed by $CH_3OH$ (10 ml) and then 36% HCl (1.3 ml). The reaction mixture was stirred for 4 hours at 60°C, poured out into ice-water and extracted with $CH_2Cl_2/CH_3OH$ (98/2). The organic layer was separated, dried ($MgSO_4$), filtered off and the solvent was evaporated. The residue was purified by reversed phase high-performance liquid chromatography (eluent: (0.5 % $NH_4OAc$ in $H_2O$)/$CH_3CN$ 10/90). The product fractions were collected and the organic solvent was evaporated. The aqueous residue was purified by Flash column chromatography on flash tubes (eluent: $CH_2Cl_2$/THF 92/8). The product fractions were collected, stirred in $CH_2Cl_2/CH_3OH$ (98/2) and filtered to remove the silica. Finally, the filtrate was evaporated. Yield: 0.020 g of compound 8.

Example B5

Preparation of compound 9

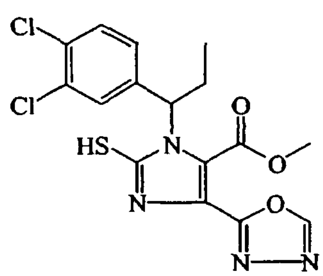

A solution of intermediate 19 (prepared according to A6.c) (0.00025 mol) in phosphorus oxychloride (5 ml) was stirred at 60°C for 18 hours and then the solvent was evaporated. The residue was stirred in ice-water and extracted with $CH_2Cl_2$. The organic layer was separated, dried ($MgSO_4$), filtered off and the solvent was evaporated. The residue was stirred in 2-propanone (5 ml) and the mixture was treated with $SO_2$-gas for 15 minutes. The solvent was evaporated and the residue was purified by high-performance liquid chromatography. The product fractions were collected and the organic solvent was evaporated. The aqueous concentrate was extracted with $CH_2Cl_2$; the organic layer was separated, dried ($MgSO_4$), filtered off and the solvent was evaporated. Yield: 0.010 g of compound 9.

Example B6

Preparation of compound 10

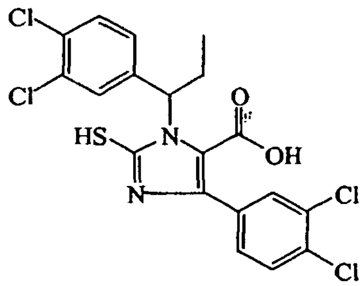

A mixture of compound 5 (prepared according to B3.a) (0.00025 mol) in 1N NaOH (1 ml) and methanol (3 ml) was reacted overnight at 70˚C and then the solvent was evaporated. The residue was treated with HCl and the mixture was filtered, then the desired product was dried (vacuum oven). Yield: 0.070 g of compound 10.

Example B7

a. Preparation of compound 11

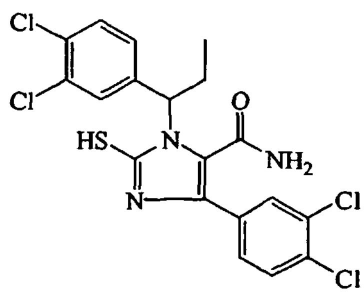

A mixture of compound 10 (prepared according to B6) (0.00015 mol) in $SOCl_2$ (5 ml) was heated at 70˚C for 4 hours, then the reaction mixture was cooled and the solvent was evaporated. The residue was diluted in THF and aqueous $NH_4OH$ (3 ml) was added in one portion. The reaction mixture was stirred for 15 minutes and the solvent was evaporated. The residue was extracted with $CH_2Cl_2$ and then the organic layer was dried and the solvent was evaporated. The residue was taken up in 2-propanone and $SO_2$ (gas) was bubbled through the mixture for 10 minutes. The solvent was evaporated and the residue was purified by flash column chromatography (eluent: $CH_2Cl_2/CH_3CN$ 95/5). The product fractions were collected and the solvent was evaporated. Yield: 15 mg of compound 11.

b. Preparation of compound 13

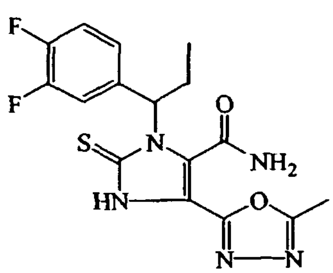

A mixture of compound 12 (prepared according to B6) (0.0012 mol), *N'*-(ethylcarbonimidoyl)-*N,N*-dimethyl-1,3-propanediamine (0.0013 mol) and 1-hydroxy-*1H*-benzotriazole (0.0013 mol) in DMF (8 ml) was stirred for 30 minutes at room temperature, then $NH_3$ (gas) was passed through the solution for 15 minutes and the reaction mixture was stirred for 1 hour. The solvent was evaporated and the obtained residue was stirred in $H_2O$, then the mixture was extracted with $CH_2Cl_2$. The organic layer was separated, dried, filtered off and the solvent was evaporated. The residue was purified by column chromatography over silica gel (eluent: $CH_2Cl_2/CH_3OH$ 96/4). The pure product fractions were collected and the solvent was evaporated. The residue was stirred in DIPE and the resulting solids were collected. Yield: 0.190 g. This fraction was purified by reversed-phase high-performance liquid chromatography, then the pure product fractions were collected and the solvent was evaporated. The obtained residue was stirred in DIPE and then the desired product was filtered off and dried. Yield: 0.091 g of compound 13 (m.p.: 228-229˚C).

Example B8

Preparation of compound 14

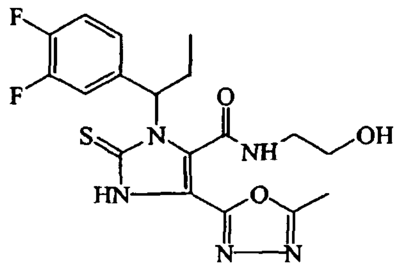

A mixture of compound 12 (prepared according to B6) (0.0012 mol), *N'*-(ethylcarbonimidoyl)-*N,N*-dimethyl-1,3-propanediamine (0.0012 mol) and 1-hydroxy-*1H*-benzotriazole (0.0012 mol) in DMF (8 ml) was stirred for 30 minutes at room temperature, then 2-aminoethanol (0.0024 mol) was added and the reaction mixture was stirred for 3 hours at room temperature. The solvent was evaporated and the obtained residue was purified by column chromatography over silica gel (eluent: $CH_2Cl_2/CH_3OH$ 96/4). The pure product fractions were collected and the solvent was evaporated. The residue was stirred in DIPE and then the resulting solids were filtered off and dried. Yield: 0.155 g of compound 14 (m.p.: 165-166˚C).

Example B9

Preparation of compound 16

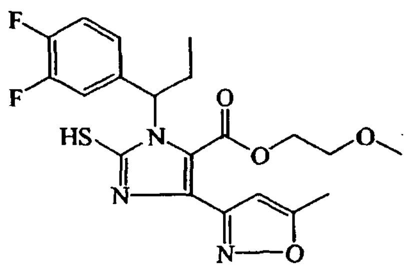

A mixture of compound 15 (prepared according to B3) (0.00029 mol) and $NaBH_4$ (0.00211 mol) in 2-methoxyethanol (5 ml) was heated for 24 hours at 100˚C and then the solvent was evaporated. The residue was purified by reversed-phase high-performance liquid chromatography. The product fractions were collected and the solvent was evaporated. Yield: 0.020 g of compound 16.

Example B10

Preparation of compound 50

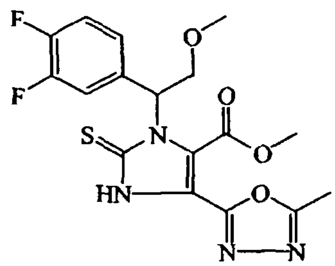

A mixture of intermediate 26 (prepared according to A7.c) (0.00125 mol) and Burgess'reagent (0.00375 mol) in THF (10 ml) was stirred for 3 hours at room temperature, then $CH_2Cl_2$ was added and the mixture was washed with $H_2O$. The organic layer was separated, dried, filtered off and the solvent was evaporated. The obtained residue was filtered over silica gel (eluent: $CH_2Cl_2/CH_3OH$ 98/2), then the product fractions were collected and the solvent was evaporated. The residue was stirred in DIPE and the desired product was filtered off and dried. Yield: 0.082 g of final compound 50 (m.p.: 102.8-102.9˚C).

Tables 2 and 3 list the compounds of formula (I) which were prepared according to one of the above samples (Ex. No.)

Table 2

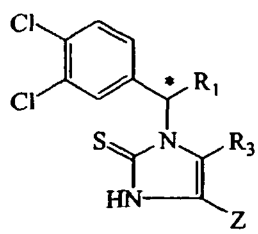

| Comp. No. | Exp. No. | R1 | R3 | Z | Properties |
|---|---|---|---|---|---|
| 17 | B2.b-1 | $-CH_2CH_3$ | H | 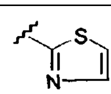 | HCl (1:1) |
| 2 | B2.a | $-CH_2CH_3$ | H | 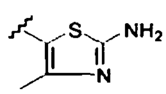 | HCl (1:1) |
| 18 | B2.a | $-CH_2CH_3$ | H | 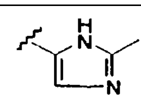 | |
| 4 | B2.b-2 | $-CH_2CH_3$ | H | 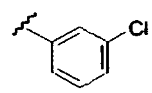 | |
| 3 | B2.b-1 | $-CH_2CH_3$ | H | 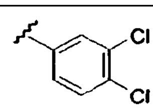 | |

(continued)

| Comp. No. | Exp. No. | R1 | R3 | Z | Properties |
|---|---|---|---|---|---|
| 19 | B2.b-1 | $-CH_2CH_3$ | H | 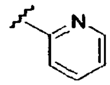 | HCl(1:1) |
| 20 | B2.a | $-CH_2CH_3$ | H | 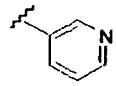 | HCl (1:1) |
| 21 | B4 | $-CH_2CH_3$ | 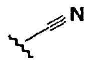 | 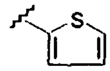 | |
| 8 | B4 | $-CH_2CH_3$ | 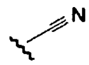 | 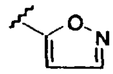 | |
| 38 | B4 | $-CH_2CH_3$ | 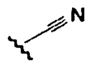 | 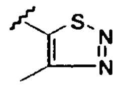 | |
| 39 | B6 | $-CH_2CH_3$ | 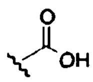 | 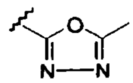 | |
| 22 | B6 | $-CH_2CH_3$ | 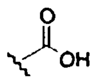 | 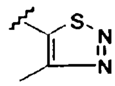 | |
| 10 | B6 | $-CH_2CH_3$ | 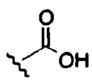 | 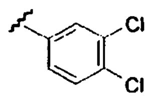 | |
| 23 | B6 | $-CH_2CH_3$ | 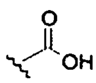 | 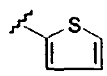 | |
| 6 | B3.b/c | $-CH_2CH_3$ | 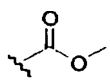 | 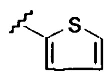 | |
| 24 | B3.c | $-CH_2CH_3$ | 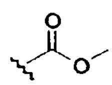 | 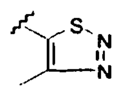 | |
| 25 | B3.abc | $-CH_2CH_3$ | 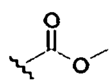 | 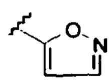 | |
| 26 | B3.abc | $-CH_2CH_3$ | 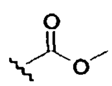 | 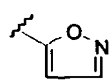 | m.p. 165-166˚C; *(S) |
| 9 | B5 | $-CH_2CH_3$ | 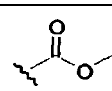 | 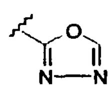 | |
| 27 | B5 | $-CH_2CH_3$ | 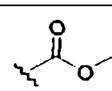 | 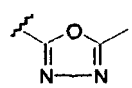 | |
| 28 | B3.abc | $-CH_2CH_3$ | 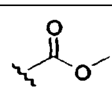 | 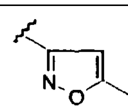 | mp. 163-164˚C |
| 29 | B3.abc | $-CH_2CH_3$ | 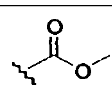 | 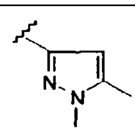 | mp. 107.8-108.3˚C |

(continued)

| Comp. No. | Exp. No. | R1 | R3 | Z | Properties |
|---|---|---|---|---|---|
| 30 | B3.abc | $-CH_2CH_3$ | 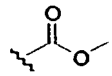 | 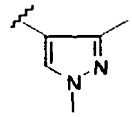 | m.p. 147.9-149.3°C |
| 31 | B3.abc | $-CH_2CH_3$ | 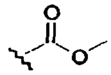 | 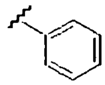 | |
| 32 | B3.abc | $-CH_2CH_3$ | 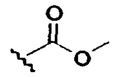 | 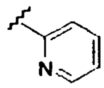 | m.p. 187.6-189.1°C |
| 33 | B3.abc | $-CH_2CH_3$ | 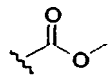 | 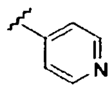 | m.p. 168.9-171.5°C |
| 34 | B3.abc | $-CH_2CH_3$ | 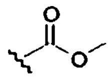 | 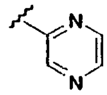 | m.p. 147.9-149.3°C |
| 5 | B3.a | $-CH_2CH_3$ | 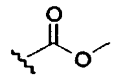 | 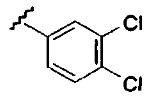 | |
| 35 | B3.abc | $-CH_2CH_3$ | 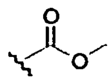 | 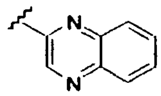 | |
| 36 | B7.b | $-CH_2CH_3$ | 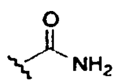 | 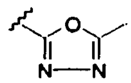 | m.p. 236-237°C |
| 11 | B7.a | $-CH_2CH_3$ | 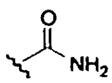 | 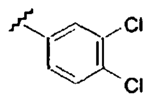 | |
| 37 | B8 | $-CH_2CH_3$ | 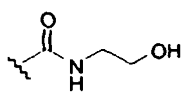 | 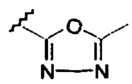 | m.p. 204-206°C |
| 1 | B1 | $-CH_2CH_3$ | 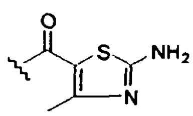 | 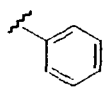 | |
| 40 | B5 | $-CH_2CH_2CH_3$ | 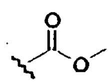 | 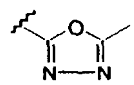 | |
| 51 | B3abc | $-CH_2CH_3$ | 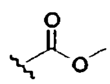 | 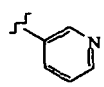 | .HCl(1:1) |
| 52 | B5 | $-CH_2CH_3$ | 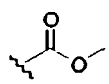 | 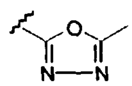 | * (S) mp.154.2-154.4°C |

Table 3

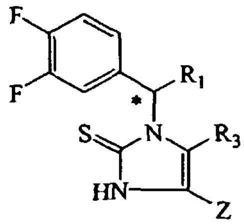

| Comp. No. | Exp. No | R1 | R3 | Z | Properties |
|---|---|---|---|---|---|
| 41 | B3.abc | $-CH_3$ | 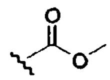 | 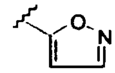 | |
| 42 | B5 | $-CH_3$ | 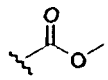 | 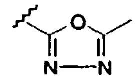 | m.p.192-194°C |
| 12 | B6 | $-CH_2CH_3$ | 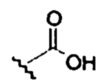 | 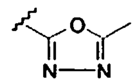 | |
| 7 | B3.d-1 | $-CH_2CH_3$ | 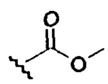 | 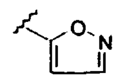 | |
| 43 | B3.d-2 | $-CH_2CH_3$ | 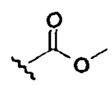 | 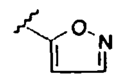 | * (S); rot[1]. -140.6°; m.p. 111.5°C |
| 15 | B3 | $-CH_2CH_3$ | 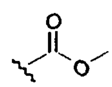 | 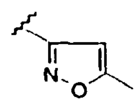 | |
| 44 | B5 | $-CH_2CH_3$ | 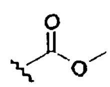 | 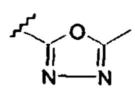 | |
| 45 | B3.abc | $-CH_2CH_3$ | 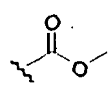 | 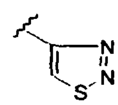 | |
| 46 | B3.abc | $-CH_2CH_3$ | 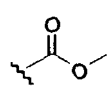 | 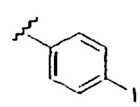 | |
| 47 | B7.b | $-CH_2CH_3$ | 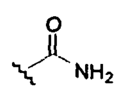 | 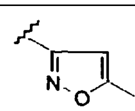 | |
| 13 | B7.b | $-CH_2CH_3$ | 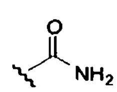 | 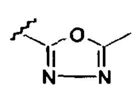 | m.p. 228-229°C |
| 14 | B8 | $-CH_2CH_3$ | 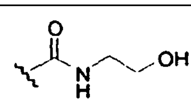 | 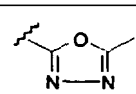 | m.p. 165-166°C |
| 16 | B9 | $-CH_2CH_3$ | 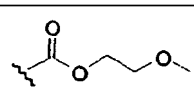 | 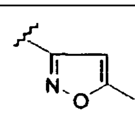 | |

(continued)

| Comp. No. | Exp. No | R1 | R3 | Z | Properties |
|---|---|---|---|---|---|
| 48 | B3.abc | $-CH_2CH_2CH_3$ | 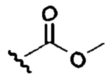 | 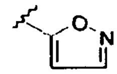 | |
| 49 | B3.abc | $-CH_2CH_2CH_3$ | 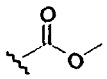 | 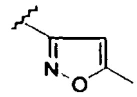 | m.p. 141˚C |
| 50 | B10 | $-CH_2OCH_3$ | 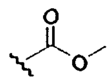 | 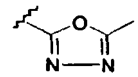 | m.p. 102.8-102.9˚C |
| 53 | B3.d-3 | $-CH_2CH_3$ | 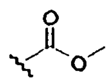 | 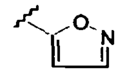 | (S); rot[2]. -172˚; m.p.128.5˚C |
| 1 : $[\alpha]^{D}_{20}$ at concentration of 0.030 g/100 ml in $CHCl_3$;<br>2 : $[\alpha]^{D}_{20}$ at concentration of 0.5 g/100 g in $CHCl_3$; | | | | | |

C. Analytical Part

*LCMS conditions 1*

The HPLC gradient was supplied by a Waters Alliance HT 2790 system with a columnheater set at 40˚C. Flow from the column was split to a Waters 996 photodiode array (PDA) detector and a Waters-Micromass ZQ mass spectrometer with an electrospray ionization source operated in positive and negative ionization mode. Reversed phase HPLC was carried out on a Xterra MS C18 column (3.5 μm, 4.6 x 100 mm) (12 minutes column) with a flow rate of 1.6 ml/minutes. Three mobile phases (mobile phase A : 95% 25mM ammoniumacetate + 5% acetonitrile; mobile phase B: acetonitrile; mobile phase C: methanol) were employed to run a gradient condition from 100 % A to 50% B and 50% C in 6.5 minutes, to 100 % B in 1 minute, 100% B for 1 minute and reequilibrate with 100 % A for 1.5 minute. An injection volume of 10 μL was used.
Mass spectra were acquired by scanning from 100 to 1000 in 1 s using a dwell time of 0.1 s. The capillary needle voltage was 3kV and the source temperature was maintained at 140˚C . Nitrogen was used as the nebulizer gas. Cone voltage was 10 V for positive ionzation mode and 20 V for negative ionization mode. Data acquisition was performed with a Waters-Micromass MassLynx-Openlynx data system

*LCMS conditions 2 Speed analysis*

The HPLC gradient was supplied by a Waters Alliance 2690 system with a columnheater set at 50˚C. Flow from the column was split to a Waters 996 photodiode array (PDA) detector and a Waters-Micromass ZQ mass spectrometer with an electrospray ionization source operated in positive and negative ionization mode. Reversed phase HPLC was carried out on a Xterra MS C18 column (2.5 μm, 4.6 x 20 mm) with a flow rate of 3 ml/min. Three mobile phases (mobile phase A 95% 25mM ammoniumacetate + 5% acetonitrile; mobile phase B: acetonitrile; mobile phase C: methanol) were employed to run a gradient condition from 100 % A to 50% B and 50% C in 0.9 min., to 100 % B in 0.37 min, 100% B for 0.18 min. and reequilibrate with 100 % A for 0.2 min. An injection volume of 2 μL was used.
Mass spectra were acquired by scanning from 100 to 1000 in Is using a dwell time of 0.1 s. The capillary needle voltage was 3kV and the source temperature was maintained at 140˚C . Nitrogen was used a the nebulizer gas. Cone voltage was 10 V for positive ionzation mode and 20 V for negative ionization mode. Data acquisition was performed with a Waters-Micromass MassLynx-Openlynx data system

Table 4 : LCMS parent peak ([$M^+$] defines the mass of the compound) and retention time (minutes)

| Compound no. | [MH+] | Retention time | LCMS condition |
|---|---|---|---|
| 31 | 421 | 6.24 | 1 |
| 6 | 427 | 6.24 | 1 |
| 27 | 427 | 5.02 | 1 |
| 5 | 488 | 6.35 | 1 |
| 9 | 413 | 4.82 | 1 |
| 11 | 473 | 5.45 | 1 |
| 4 | 397 | 6.54 | 1 |
| 19 | 364* | 5.54 | 1 |
| 17 | 370* | 6.20 | 1 |
| 3 | 433 | 4.76 | 1 |
| 24 | 443 | 5.34 | 1 |
| 40 | 441 | 5.12 | 1 |
| 35 | 473 | 5.91 | 1 |
| 1 | 503 | 5.65 | 1 |
| 20 | 364* | 4.57 | 1 |
| 18 | 367 | 5.24 | 1 |
| 25 | 412 | 5.60 | 1 |
| 2 | 399* | 5.51 | 1 |
| 21 | 394 | 4.81 | 1 |
| 10 | 475 | 4.40 | 1 |
| 8 | 377(-) | 5.34 | 1 |
| 7 | 378 | 5.59 | 1 |
| 38 | 410 | 4.95 | 1 |
| 15 | 393 | 5.61 | 1 |
| 45 | 396 | 5.74 | 1 |
| 47 | 378 | 3.57 | 1 |
| 46 | 514 | 6.62 | 1 |
| 16 | 437 | 5.33 | 1 |
| 51 | 422 | 1.02 | 2 |

[$M^+$] defines the mass of the compound
* mass of the corresponding base

D. Pharmacological example

*Inhibition of MCP-1 induced Ca-flux in human THP-1 cells*

MCP-1 binding to the CCR2 receptor induces a rapid and transient intracellular release of $Ca^{2+}$ (secondary messenger) in several cell lines (Charo et al, PNAS 1994). Free $Ca^{2+}$ levels can be measured using a $Ca^{2+}$ sensitive dye. When the CCR2 receptor is blocked with a CCR2 receptor antagonist, the MCP-1 induced release of $Ca^{2+}$ is inhibited.

Human THP-1 cells (monocytic cell line, ATCC TIB-202) were cultured in RPMI 1640 medium supplemented with 10 % fetal calf serum (FCS), 1% L-Glutamine, penicillin (50 U/ml) and streptomycin (50 $\mu$g/ml) (all GIBCO BRL,

Gent). After centrifugation, cells were loaded for 30 minutes with the $Ca^{2+}$ sensitive fluorescent dye Fluo-3 AM (Molecular Probes, Leiden, Netherlands) (2 million cells/ml in RPMI medium containing 4 μM Fluo-3 AM, 20 mM HEPES, 0.1 % Bovine Serum Albumin (BSA) and 5 mM probenecid). Excess dye was removed by 3-fold washing with buffer (5 mM HEPES, 140 mM NaCl, 1 mM $MgCl_2$, 5 mM KCl, 10 mM glucose, 2.5 mM probenecid, 1.25 mM $CaCl_2$, 0.1 % BSA; all further incubations were done in this buffer). Cells were plated at a density of 150 000 cells/well in dark-wall 96-well plates (Costar, Cambridge, MA) and sedimented by centrifugation (1 minute). The cells were pre-incubated for 20 minutes with test compound. Then, $10^{-7}$ M hMCP-1 (Bachem, Bubendorf, Switserland) was added. Changes in intracellular free $Ca^{2+}$ concentration were measured using the Fluorescent Imaging Plate Reader (FLIPR, Molecular Devices, Munchen, Germany). Fluorescence was recorded every second from 10 seconds before the addition of the MCP-1 till 2 minutes after the addition (first minute: 60 records with 1 second intervals, second minute 20 records with 3 second intervals). The maximal fluorescence obtained during this time frame was used for further calculations.

Table 5 reports $pIC_{50}$ values obtained in the above-described test for compounds of formula (I). $pIC_{50}$ defines $-\log IC_{50}$ wherein $IC_{50}$ is the molar concentration of the test compound which inhibits 50 % of specific MCP-1 induced $Ca^{2+}$ flux.

Table 5

| Comp. No. | $pIC_{50}$ |
|---|---|
| 31 | 7.12 |
| 24 | 7.14 |
| 6 | 7.26 |
| 40 | 7.36 |
| 36 | 7.4 |
| 51 | 7.4 |
| 9 | 7.45 |
| 27 | 7.48 |
| 45 | 7.5 |
| 16 | 7.5 |
| 49 | 7.6 |
| 32 | 7.6 |
| 34 | 7.6 |
| 28 | 7.8 |
| 25 | 7.88 |
| 48 | 7.9 |
| 33 | 7.9 |
| 7 | 7.93 |
| 26 | 8.1 |
| 43/53 | 8.1 |

*Radioligand binding assay*.

$^{125}$I-MCP-1 binding assays were performed in 96-well plates with 40 μg of protein per well. Compounds were dissolved and diluted in DMSO to 100X dilutions. A 10X concentration range of compounds was prepared in binding buffer (10% DSMO). Competition binding assays contained the following components in a total volume of 250 μl: 25 μl of the appropriate compound dilution (final concentration of 1% DMSO), 200 μl membranes from CCR2B- transfected CHO cells dissolved in binding buffer and 25 μl $^{125}$I-MCP-1 (Bolton and Hunter labeled, Amersham, specific activity = 2000 Ci/mmol, 0.15 nM final). Binding buffer was composed of 25 mM HEPES, 5 mM $MgCl_2$, 1 mM $CaCl_2$, 0.5% protease-free bovine serum albumin, pH 7.4. After 90 minutes incubation at 25 °C, membranes were harvested on GF/B filters - presoaked in 0.5% polyethylenimine, followed by washing with buffer containing 25 mM HEPES, 5 mM $MgCl_2$, 1 mM

$CaCl_2$, 5 mM NaCl, pH 7.4. Filter bound radioactivity was determined by liquid scintillation counting. $EC_{50}$ values (μM) and $K_i$ values (μM) were calculated. The $EC_{50}$ value indicates the concentration of the test compound that competes with MCP-1 for half of the specific binding sites; the $K_i$ value indicates the equilibrium dissociation constant, i.e. the concentration of the test compound that will bind to half of the binding sites at equilibrium in the absence of radioligand or other competitors. $EC_{50}$ values and $K_i$ values were calculated using non-linear regression in Graphpad Prism. Prism calculates the $K_i$ or affinity of the receptor for the competing drug using the equation of Cheng and Prusoff (Biochem. Pharmacol. 1973, 22: 3099-3108). A low $K_i$ indicates a high affinity of the receptor for the test compound.

$$K_i = \frac{EC_{50}}{1 + \frac{[radioligand]}{K_d}}$$

wherein $K_d$ describes the affinity of the radioligand for the receptor, i.e. the concentration of the radioligand that will bind to half of the binding sites at equilibrium in the absence of competitors.

Table 6 lists $K_i$ values (μM) obtained in the above-described test for compounds of formula (I).

Table 6 :

| Comp.No. | $K_i$ values |
|---|---|
| 13 | 0.30 |
| 15 | 0.35 |
| 26 | 0.05 |
| 28 | 0.01 |
| 31 | 0.07 |
| 36 | 0.07 |
| 43/53 | 0.03 |

*Chemotactic response*

The CCR2 antagonistic activity of the compounds of the present invention can also be determined by measuring the effect of the compounds on the chemotactic response of cells in the presence of a chemokine, such as for example MCP-1.

Mononuclear cells from human heparinized peripheral blood (PBMC) were isolated using Ficoll-Paque gradient centrifugation (Amersham Biosciences). Assays of chemotactic responsiveness were performed using disposable 96-well chemotaxis chambers (ChemoTx, Neuro Probe) with 5-μm pore size polycarbonate (PVP-free) filter membranes. Mononuclear cells were fluorescently labeled with 5 μg/ml Calcein-AM (Molecular Probes, Eugene, OR) at 37°C for 30 minutes. Labeled cells were washed twice and resuspended at $5x10^6$ cells/ml in Hanks' Balanced Salt Solution (Gibco BRL) supplemented with 0.2% bovine serum albumin. Subsequently, cells were pre-incubated for 10 minutes at room temperature with serial dilutions of the compounds in DMSO (dimethylsulfoxide) (final DMSO concentration of 0.2%). Bottom wells of the chemotaxis chamber were loaded with 28 μl medium containing 30 ng/ml recombinant hMCP-1 (R&D) or buffer only. Pre-treated cells (100.000 cells) were added in triplicate to the topside of the filter (20 μl) and incubated at 37°C in humidified air containing 5% $CO_2$. After 105 minutes incubation, the non-migrated cells were removed from the top of the filter by gently wiping the filter with a tissue. The migrated cells were measured using a fluorescent plate reader ($\lambda_{excitation}$ = 485 nm; $\lambda_{emission}$ = 538 nm). The chemotactic response can be expressed as chemotactic index (C.I.), being the ratio of the means of migrated cells in the presence of MCP-1 and the means of migrated cells in the absence of chemokine. Percentage inhibition was calculated using the formula:

$$\%inhibition = (1 - \frac{F_{sample} - F_{buffer}}{F_{MCP-1} - F_{buffer}})x100$$

with $F_{sample}$, the fluorescence of the cells pre-incubated with 10, 1, 0.1, 0.01 or 0.001 μM compound and migrated to

30 ng/ml MCP-1 in the bottom wells; $F_{MCP-1}$, the fluorescence of the cells pre-incubated with buffer-0.2% DMSO and migrated to 30 ng/ml MCP-1 and $F_{buffer}$, the fluorescence of cells pre-incubated with buffer-0.2% DMSO and spontaneous migrated to buffer in the bottom wells. Table 7 lists the $IC_{50}$ values (μM) obtained in the above-described test for compounds of formula (I). Assays were run in triplicate and repeated 2-7 times.

Table 7 :

| Compound no. | IC50 (μM) |
|---|---|
| 23 | 0.024 |
| 53 | 0.108 |
| 31 | 0.214 |
| 36 | 0.244 |
| 28 | 0.367 |
| 51 | 0.474 |
| 15 | 0.798 |

## Claims

**1.** A compound of formula

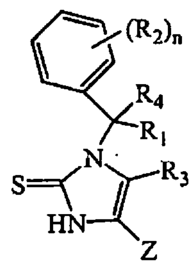

(I)

,

a *N*-oxide, a pharmaceutically acceptable addition salt, a quaternary amine, a polymorphic form or a stereochemically isomeric form thereof, wherein

$R_1$ represents $C_{1-6}$alkyl or $C_{1-6}$alkyloxy$C_{1-6}$alkyl;
each $R_2$ independently represents halo;
$R_3$ represents hydrogen, cyano, $C_{1-6}$alkyl optionally substituted with hydroxy or $C_{1-6}$alkyloxy, C(=O)-O-$R_5$, C(=O)-$NR_{6a}R_{6b}$, C(=S)-$NR_{6a}R_{6b}$, $S(=O)_2$-$NR_{6a}R_{6b}$ or C(=O)-$R_7$;
$R_4$ represents hydrogen;
$R_5$ represents hydrogen, $C_{1-6}$alkyl, hydroxy$C_{1-6}$alkyl, $C_{2-6}$alkenyl, $C_{2-6}$alkynyl, polyhalo$C_{1-6}$alkyl, $C_{1-6}$alkyloxy$C_{1-6}$alkyl, amino$C_{1-6}$alkyl, mono-or di($C_{1-4}$alkyl)amino$C_{1-6}$alkyl, aminocarbonyl$C_{1-6}$alkyl, mono-or di($C_{1-4}$alkyl)aminocarbonyl$C_{1-6}$alkyl or aryl;
$R_{6a}$ and $R_{6b}$ each independently represent hydrogen, $C_{1-6}$alkyl, amino, mono-or di($C_{1-4}$alkyl)amino, arylNH-, amino$C_{1-6}$alkyl, mono-or di($C_{1-4}$alkyl)amino-$C_{1-6}$alkyl, $C_{1-6}$alkylcarbonylamino, aminocarbonylamino, $C_{1-6}$alkyloxy, carbonylamino or hydroxy$C_{1-6}$alkyl; or
$R_{6a}$ and $R_{6b}$ taken together with the nitrogen to which they are attached form pyrrolidinyl, imidazolidinyl, pyrazolidinyl, piperidinyl, piperazinyl, morpholinyl, thiomorpholinyl or piperazinyl substituted with $C_{1-6}$alkyl;
$R_7$ represents hydrogen, $C_{1-6}$alkyl, hydroxy$C_{1-6}$alkyl, $C_{2-6}$alkenyl, $C_{2-6}$alkynyl, polyhalo$C_{1-6}$alkyl, $C_{1-6}$alkyloxy$C_{1-6}$alkyl, amino$C_{1-6}$alkyl, mono-or di($C_{1-4}$alkyl)amino$C_{1-6}$alkyl, aminocarbonyl$C_{1-6}$alkyl, mono-or di($C_{1-4}$alkyl)aminocarbonyl$C_{1-6}$alkyl, aryl or heteroaryl;
Z represents a cyclic ring system selected from

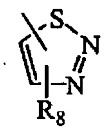 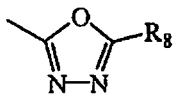 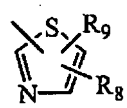 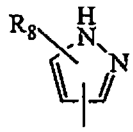 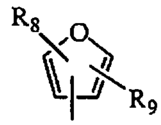 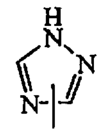

(a-1) (a-2) (a-3) (a-4) (a-5) (a-6)

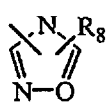 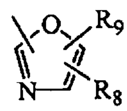 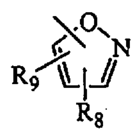 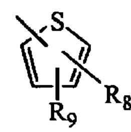 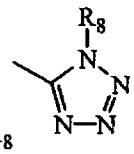 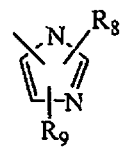

(a-7) (a-8) (a-9) (a-10) (a-11) (a-12)

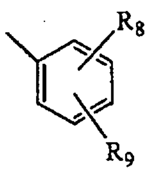 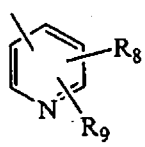 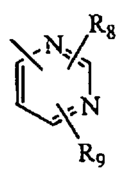 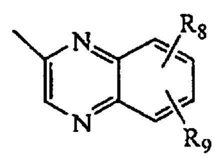 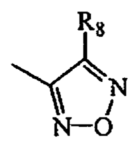

(a-13) (a-14) (a-15) (a-16) (a-17)

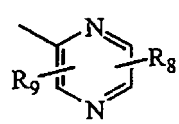

(a-18)

each $R_8$ independently represents hydrogen, halo, $C_{1-6}$alkyl, $C_{1-6}$alkyloxy, polyhalo$C_{1-6}$alkyl, polyhalo$C_{1-6}$alkyloxy, cyano, aminocarbonyl, mono-or di($C_{1-4}$alkyl)aminocarbonyl, amino, mono-or di($C_{1-4}$alkyl) amino, hydroxy$C_{1-6}$alkylamino, aryl, aryloxy, piperidinyl, piperidinylamino, morpholinyl, piperazinyl or nitro;
each $R_9$ independently represents hydrogen, halo or $C_{1-6}$alkyl;
n is 1, 2, 3, 4 or 5;
aryl represents phenyl or phenyl substituted with one, two, three, four or five substituents each independently selected from halo, $C_{1-6}$alkyl, $C_{1-6}$alkyloxy, polyhalo$C_{1-6}$alkyl, polyhalo$C_{1-6}$alkyloxy, cyano, aminocarbonyl, mono-or di($C_{1-4}$alkyl)aminocarbonyl, amino, mono-or di($C_{1-4}$alkyl)amino, phenyloxy or nitro;
heteroaryl represents furanyl, thienyl, pyrrolyl, oxazolyl, thiazolyl, imidazolyl, pyrazolyl, isoxazolyl, isothiazolyl, oxadiazolyl, triazolyl, thiadiazolyl, pyridyl, pyridazinyl, pyrimidinyl, pyrazinyl, each of said heterocycles optionally being substituted with one or two substituents each independently selected from halo, $C_{1-6}$alkyl, $C_{1-6}$alkyloxy, polyhalo$C_{1-6}$alkyl, polyhalo$C_{1-6}$alkyloxy, cyano, aminocarbonyl, mono-or di($C_{1-4}$alkyl)aminocarbonyl, amino, mono-or di($C_{1-4}$alkyl)amino or nitro.

**2.** A compound according to claim 1 wherein $R_2$ represents fluoro.

**3.** A compound according to claim 1 or 2 wherein n is 2 or 3.

**4.** A compound according to claim 3 wherein n is 2.

**5.** A compound according to claim 4 wherein n is 2 and said two $R^2$ substituents are placed in meta and para postion.

**6.** A compound according to any of one of the preceding claims wherein Z represents a radical of formula (a-1), (a-2), (a-3), (a-9), (a-10), (a-12), (a-13), (a-14) or (a-16).

**7.** A compound according to claim 6 wherein Z represents a radical of formula (a-1), (a-2), (a-9), (a-10) or (a-13).

**8.** A compound according to claim 7 wherein Z represents a radical of formula (a-9).

**9.** A compound according to any one of the preceding claims wherein $R_3$ represents hydrogen, cyano, C(=O)-O-$R_5$, C(=O)-$NR_{6a}R_{6b}$, C(=S)-$N_{6a}R_{6b}$, $S(=O)_2$-$NR_{6a}R_{6b}$ or C(=O)-$R_7$.

**10.** A compound according to claim 9 wherein $R_3$ represents cyano, C(=O)-O-$R_5$, C(=O)-$NR_{6a}R_{6b}$, C(=S)-$NR_{6a}R_{6b}$, S $(=O)_2$-$NR_{6a}R_{6b}$ or C(=O)-$R_7$.

**11.** A compound according to claim 10 wherein $R_3$ represents C(=O)-O-$R_5$.

**12.** A compound according to claim 11 wherein $R_3$ represents methoxycarbonyl.

**13.** A compound according to any one of the preceding claims wherein $R_1$ represents $C_{1-6}$alkyl.

**14.** A compound according to claim 13 wherein $R_1$ represents ethyl.

**15.** A compound according to claim 1 wherein $R_1$ represents $C_{1-6}$alkyl or $C_{1-6}$alkyloxyalkyl; $R_2$ represents halo; $R_3$ represents hydrogen, cyano, C(=O)-O-$R_5$, C(=O)-$NR_{6a}R_{6b}$, C(=O)-$R_7$; Z represents a ring system selected from (a-1), (a-2), (a-3), (a-9), (a-10), (a-12), (a-13), (a-14) or (a-16); $R_4$ represents hydrogen; n is 2.

**16.** A compound according to any one of the preceding claims wherein the compound is stereochemically pure.

**17.** A compound according to any one of the preceding claims wherein the compound has the following formula

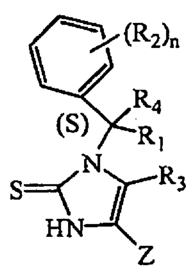

(I')

**18.** A compound according to claim 1 wherein the compound has the following formula

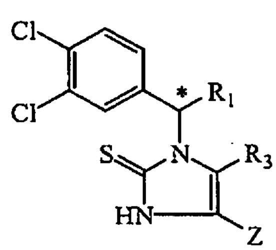

| $R_1$ | $R_3$ | Z | |
|---|---|---|---|
| $-CH_2CH_3$ | 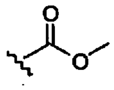 | 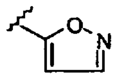 | * (S) |
| $-CH_2CH_3$ | 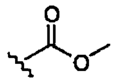 | 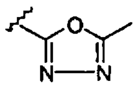 | * (S) |

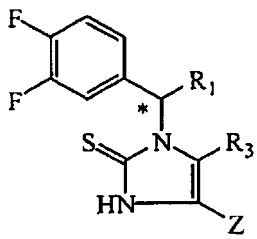

| $R_1$ | $R_3$ | Z | |
|---|---|---|---|
| $-CH_2CH_2CH_3$ | 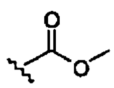 | 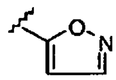 | |
| $-CH_2CH_3$ | 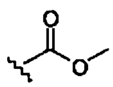 | 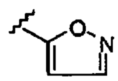 | * (S); m.p. 111.5˚C |
| $-CH_2CH_3$ | 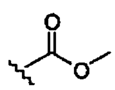 | 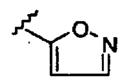 | * (S); m.p. 128.5˚C |

a *N*-oxide, a pharmaceutically acceptable addition salt, a quaternary amine or a polymorphic form thereof.

**19.** A compound according to claim 1 wherein the compound is (S)-3-[1-(3,4-difluorophenyl)-propyl]-5-isoxazol-5-yl-2-thioxo-2,3-dihydro-1*H*-imidazole-4-carboxylic acid methyl ester, a *N*-oxide, a pharmaceutically acceptable addition salt, a quaternary amine or a polymorphic form thereof.

**20.** A compound according to claim 19 wherein the compound is (S)-3-[1-(3,4-difluoro-phenyl)-propyl]-5-isoxazol-5-yl-2-thioxo-2,3-dihydro-1*H*-imidazole-4-carboxylic acid methyl ester or a *N*-oxide thereof.

**21.** A compound according to claim 19 wherein the compound is (S)-3-[1-(3,4-difluoro-phenyl)-propyl]-5-isoxazol-5-yl-2-thioxo-2,3-dihydro-1*H*-imidazole-4-carboxylic acid methyl ester or a pharmaceutically acceptable addition salt thereof.

**22.** A compound according to claim 19 wherein the compound is (S)-3-[1-(3,4-difluoro-phenyl)-propyl]-5-isoxazol-5-yl-2-thioxo-2,3-dihydro-1*H*-imidazole-4-carboxylic acid methyl ester or a quaternary amine thereof.

**23.** A compound according to claim 19 wherein the compound is (S)-3-[1-(3,4-difluoro-phenyl)-propyl]-5-isoxazol-5-yl-2-thioxo-2,3-dihydro-1*H*-imidazole-4-carboxylic acid methyl ester.

**24.** A compound according to claim 19 wherein the compound is (S)-3-[1-(3,4-difluoro-phenyl)-propyl]-5-isoxazol-5-yl-2-thioxo-2,3-dihydro-1*H*-imidazole-4-carboxylic acid methyl ester with a melting point of 128.5˚C.

**25.** A compound as claimed in any one of the preceding claims for use as a medicine.

26. Use of a compound as claimed in any one of claims 1 to 24 for the manufacture of a medicament for preventing or treating diseases mediated through activation of the CCR2 receptor.

27. Use according to claim 26 wherein the disease is an inflammatory disease.

28. A pharmaceutical composition comprising a pharmaceutically acceptable carrier, and as active ingredient a therapeutically effective amount of a compound as claimed in any one of claims 1 to 24.

29. A process of preparing a composition as claimed in claim 28 **characterized in that** a pharmaceutically acceptable carrier is intimately mixed with a therapeutically effective amount of a compound as claimed in any one of claims 1 to 24.

30. A process of preparing a compound as defined in claim 1 **characterized by**

a) by reacting an intermediate of formula (II-a) or (II-b) with KSCN in the presence of a suitable acid and a suitable solvent,

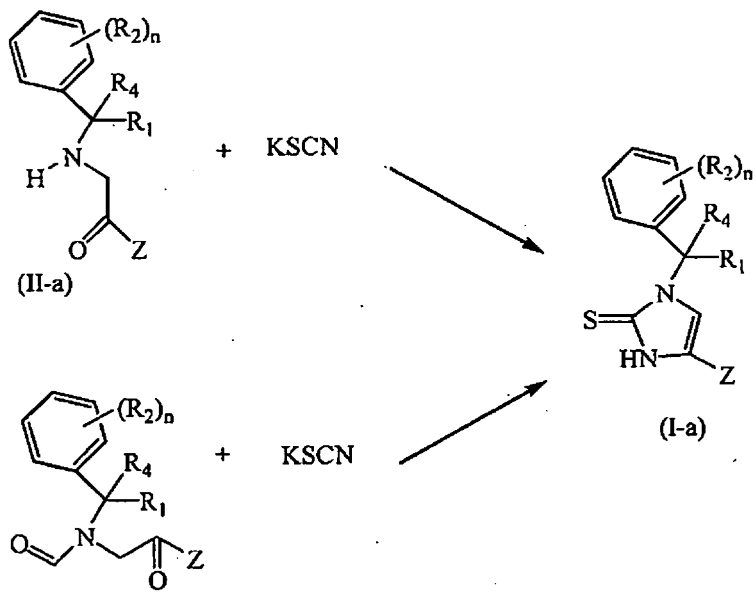


(II-a)

(I-a)

(II-b)

with $R_1$, $R_2$, $R_4$, Z and n as defined in claim 1;

b) reacting an intermediate of formula (III) with an intermediate of formula (IV) wherein $W_1$ represents a suitable leaving group, in the presence of KSCN, a suitable acid, a suitable solvent, and a suitable base,

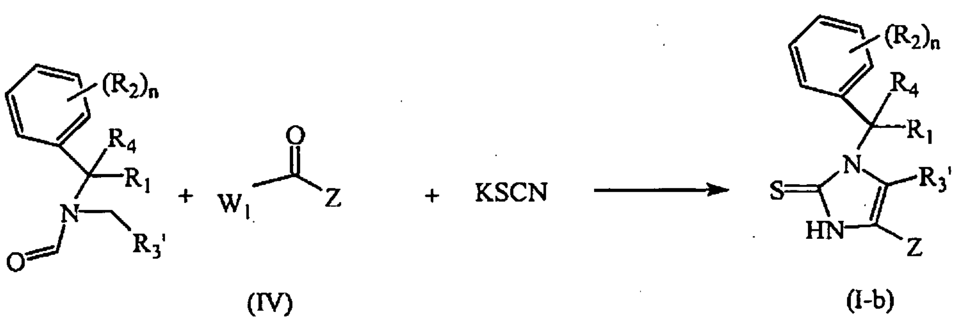


(III) (IV) (I-b)

with $R_1$, $R_2$, $R_4$, Z and n as defined in claim 1 and with $R_{3'}$ representing $R_3$ other than hydrogen;

c) reacting an intermediate of formula (V) with a suitable base in the presence of a suitable solvent

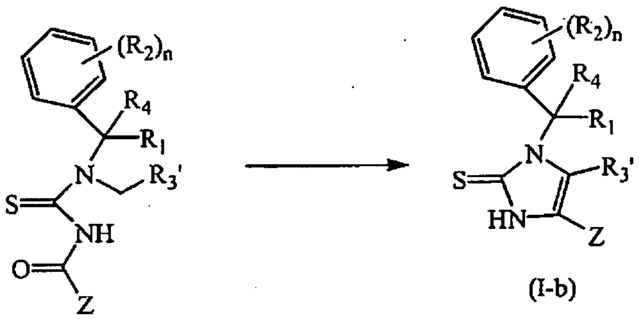

(I-b)

(V)

with $R_1$, $R_2$, $R_4$, Z and n as defined in claim 1 and with $R_{3'}$ representing $R_3$ other than hydrogen;

d) reacting an intermediate of formula (VI) with phosphoric trichloride or Burgess'reagent optionally in the presence of a suitable solvent

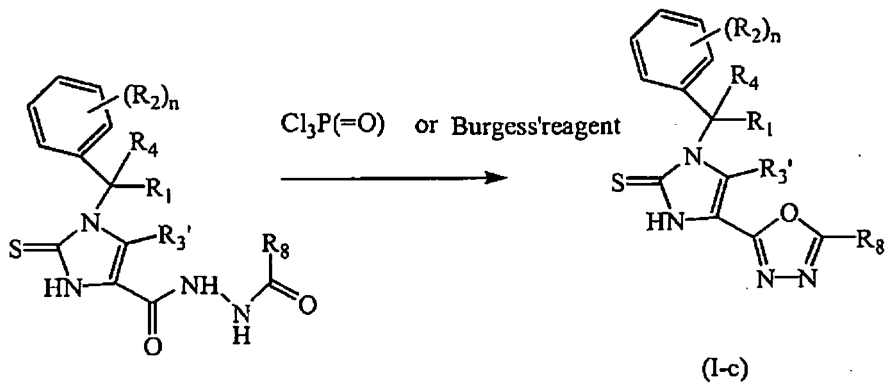


(I-c)

(VI)

with $R_1$, $R_2$, $R_3$, $R_4$, $R_8$ and n as defined in claim 1;

e) reacting an intermediate of formula (VII), wherein $W_2$ represents a suitable leaving group, with an appropriate alcohol of formula HO-$R_5$, wherein $R_5$, represents $C_{1-6}$alkyl or hydroxy$C_{1-6}$alkyl in the presence of a suitable solvent

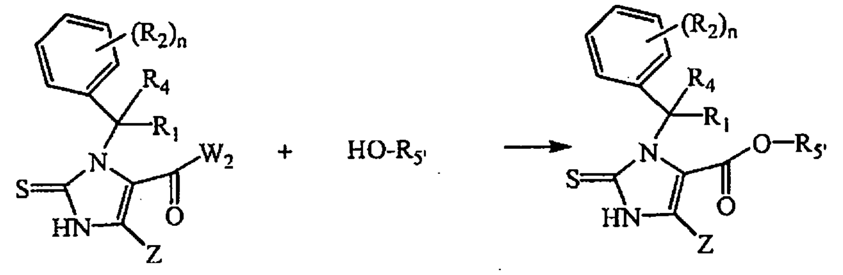


(VII)

(I-d)

with $R_1$, $R_2$, $R_4$, Z and n as defined in claim 1;

f) reacting an intermediate of formula (VII), wherein $W_2$ represents a suitable leaving group, with an intermediate of formula (VIII) in the presence of a suitable solvent

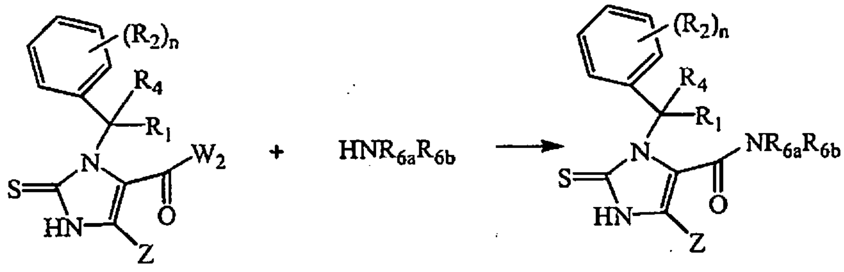

(VII) (VIII) (I-e)

with $R_1$, $R_2$, $R_4$, $R_{6a}$, $R_{6b}$, Z and n as defined in claim 1;

g) reacting an intermediate of formula (VII) with a suitable reducing agent in the presence of a suitable solvent

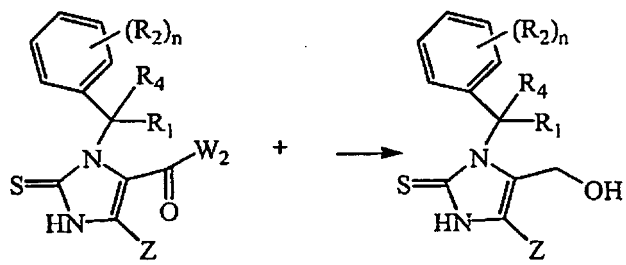

(VII) (I-f)

with $R_1$, $R_2$, $R_4$, Z and n as defined in claim 1;

or, if desired, converting compounds of formula (I) into each other following art-known transformations, and further, if desired, converting the compounds of formula (I), into a therapeutically active non-toxic acid addition salt by treatment with an acid, or into a therapeutically active non-toxic base addition salt by treatment with a base, or conversely, converting the acid addition salt form into the free base by treatment with alkali, or converting the base addition salt into the free acid by treatment with acid; and, if desired, preparing stereochemically isomeric forms, quaternary amines or *N*-oxide forms thereof.

**31.** A process of preparing a compound as defined in claim 17 **characterized by** performing the reactions according to claim 30 starting from an intermediate wherein the carbon atom carrying the $R_1$ and $R_4$ substituent has the (S) configuration.

**32.** A compound of formula (IX-b-1-1)

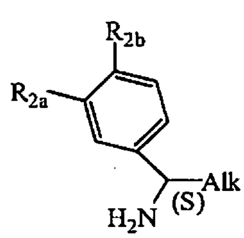

(IX-b-1-1)

a *N*-oxide, a pharmaceutically acceptable addition salt or a quaternary amine thereof, wherein Alk represents methyl, ethyl or n-propyl, and each $R_{2a}$ and $R_{2b}$ independently represents chloro or fluoro provided that when $R_{2a}$ and $R_{2b}$ are both chloro, then Alk is other than ethyl.

**33.** A compound according to claim 32 wherein the compound is

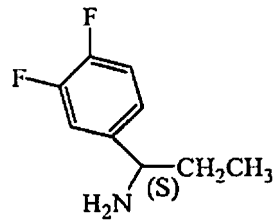

(IX-b-1-1-a)

**34.** A compound having the following formula

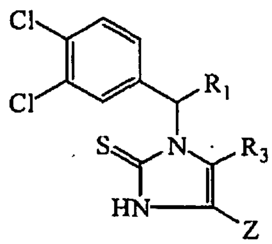

| R1 | R3 | Z |
|---|---|---|
| $-CH_2CH_3$ | 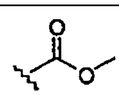 | 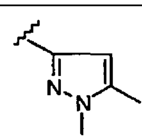 |
| $-CH_2CH_3$ | 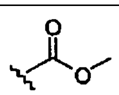 | 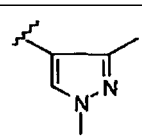 |

## Patentansprüche

**1.** Verbindung der Formel (I)

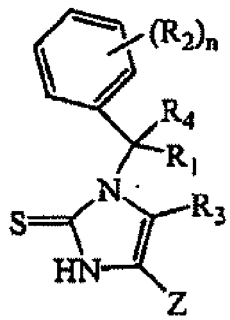

(I)

ein *N*-Oxid, ein pharmazeutisch annehmbares Additionssalz, ein quaternäres Amin, eine polymorphe Form oder eine stereochemisch isomere Form davon, wobei

$R_1$ für $C_{1-6}$-Alkyl oder $C_{1-6}$-Alkoxy-$C_{1-6}$-alkyl steht;
$R_2$ jeweils unabhängig voneinander für Halogen steht;
$R_3$ für Wasserstoff, Cyano, gegebenenfalls durch Hydroxy oder $C_{1-6}$-Alkyloxy substituiertes $C_{1-6}$-Alkyl, C(=O)-O-$R_5$, C(=O)-$NR_{6a}R_{6b}$, C(=S)-$NR_{6a}R_{6b}$, S $(=O)_2$-$NR_{6a}R_{6b}$ oder C(=O)-$R_7$ steht;
$R_4$ für Wasserstoff steht;
$R_5$ für Wasserstoff, $C_{1-6}$-Alkyl, Hydroxy-$C_{1-6}$-alkyl, $C_{2-6}$-Alkenyl, $C_{2-6}$-Alkinyl, Polyhalogen-$C_{1-6}$-alkyl, $C_{1-6}$-Alkyloxy-$C_{1-6}$-alkyl, Amino-$C_{1-6}$-alkyl, Mono- oder Di ($C_{1-4}$-alkyl)amino-$C_{1-6}$-alkyl, Aminocarbonyl-$C_{1-6}$-alkyl, Mono- oder Di($C_{1-4}$-alkyl)aminocarbonyl-$C_{1-6}$-alkyl oder Aryl steht;
$R_{6a}$ und $R_{6b}$ jeweils unabhängig voneinander für Wasserstoff, $C_{1-6}$-Alkyl, Amino, Mono- oder Di($C_{1-4}$-alkyl) amino, Aryl-NH-, Amino-$C_{1-6}$-alkyl, Mono- oder Di ($C_{1-4}$-alkyl) amino-$C_{1-6}$-alkyl, $C_{1-6}$-Alkylcarbonylamino, Aminocarbonylamino, $C_{1-6}$-Alkyloxy, Carbonylamino oder Hydroxy-$C_{1-6}$-alkyl stehen; oder
$R_{6a}$ und $R_{6b}$ zusammen mit dem Stickstoff, an den sie gebunden sind, Pyrrolidinyl, Imidazolidinyl, Pyrazolidinyl, Piperidinyl, Piperazinyl, Morpholinyl, Thiomorpholinyl oder Piperazinyl, substituiert durch $C_{1-6}$-Alkyl, bilden;
$R_7$ für Wasserstoff, $C_{1-6}$-Alkyl, Hydroxy-$C_{1-6}$-alkyl, $C_{2-6}$-Alkenyl, $C_{2-6}$-Alkinyl, Polyhalogen-$C_{1-6}$-alkyl, $C_{1-6}$-Alkyloxy-$C_{1-6}$-alkyl, Amino-$C_{1-6}$-alkyl, Mono- oder Di($C_{1-4}$-alkyl)amino-$C_{1-6}$-alkyl, Aminocarbonyl-$C_{1-6}$-alkyl, Mono- oder Di($C_{1-4}$-alkyl)aminocarbonyl-$C_{1-6}$-alkyl, Aryl oder Heteroaryl steht;
Z für ein unter

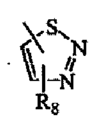
(a-1)

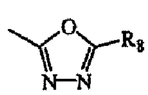
(a-2)

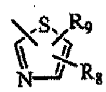
(a-3)

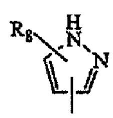
(a-4)

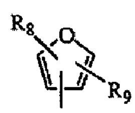
(a-5)

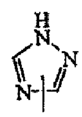
(a-6)

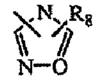
(a-7)

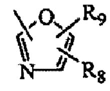
(a-8)

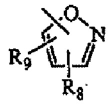
(a-9)

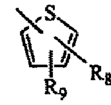
(a-10)

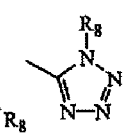
(a-11)

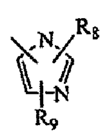
(a-12)

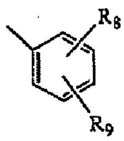 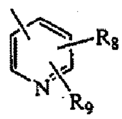 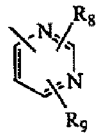 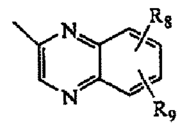 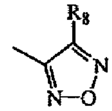

(a-13) (a-14) (a-15) (a-16) (a-17)

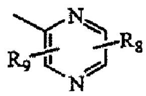

ausgewähltes cyclisches Ringsystem steht;

$R_8$ jeweils unabhängig voneinander für Wasserstoff, Halogen, $C_{1-6}$-Alkyl, $C_{1-6}$-Alkyloxy, Polyhalogen-$C_{1-6}$-alkyl, Polyhalogen-$C_{1-6}$-alkyloxy, Cyano, Aminocarbonyl, Mono- oder Di($C_{1-4}$-alkyl)aminocarbonyl, Amino, Mono- oder Di($C_{1-4}$-alkyl)amino, Hydroxy-$C_{1-6}$-alkylamino, Aryl, Aryloxy, Piperidinyl, Piperidinylamino, Morpholinyl, Piperazinyl oder Nitro steht;

$R_9$ jeweils unabhängig voneinander für Wasserstoff, Halogen oder $C_{1-6}$-Alkyl steht;

n für 1, 2, 3, 4 oder 5 steht;

Aryl für Phenyl oder durch einen, zwei, drei, vier oder fünf Substituenten, die jeweils unabhängig voneinander unter Halogen, $C_{1-6}$-Alkyl, $C_{1-6}$-Alkyloxy, Polyhalogen-$C_{1-6}$-alkyl, Polyhalogen-$C_{1-6}$-alkyloxy, Cyano, Aminocarbonyl, Mono- oder Di($C_{1-4}$-alkyl)aminocarbonyl, Amino, Mono- oder Di($C_{1-4}$-alkyl)amino, Phenyloxy oder Nitro ausgewählt sind, substituiertes Phenyl steht;

Heteroaryl für Furanyl, Thienyl, Pyrrolyl, Oxazolyl, Thiazolyl, Imidazolyl, Pyrazolyl, Isoxazolyl, Isothiazolyl, Oxadiazolyl, Triazolyl, Thiadiazolyl, Pyridyl, Pyridazinyl, Pyrimidinyl oder Pyrazinyl steht, wobei jeder der Heterocyclen gegebenenfalls durch einen oder zwei Substituenten, die jeweils unabhängig voneinander unter Halogen, $C_{1-6}$-Alkyl, $C_{1-6}$-Alkyloxy, Polyhalogen-$C_{1-6}$-alkyl, Polyhalogen-$C_{1-6}$-alkyloxy, Cyano, Aminocarbonyl, Mono- oder Di($C_{1-4}$-alkyl)aminocarbonyl, Amino, Mono- oder Di($C_{1-4}$-alkyl)amino oder Nitro ausgewählt sind, substituiert ist.

**2.** Verbindung nach Anspruch 1, in der $R_2$ für Fluor steht.

**3.** Verbindung nach Anspruch 1 oder 2, in der n für 2 oder 3 steht.

**4.** Verbindung nach Anspruch 3, in der n für 2 steht.

**5.** Verbindung nach Anspruch 4, in der n für 2 steht und die beiden $R^2$-Substituenten in meta- und para-Position stehen.

**6.** Verbindung nach einem der vorhergehenden Ansprüche, in der Z für einen unter (a-1), (a-2), (a-3), (a-9), (a-10), (a-12), (a-13), (a-14) oder (a-16) ausgewählten Rest steht.

**7.** Verbindung nach Anspruch 6, in der Z für einen unter (a-1), (a-2), (a-9), (a-10) oder (a-13) ausgewählten Rest steht.

**8.** Verbindung nach Anspruch 7, in der Z für einen Rest der Formel (a-9) steht.

**9.** Verbindung nach einem der vorhergehenden Ansprüche, in der $R_3$ für Wasserstoff, Cyano, C(=O)-O-$R_5$, C (=O)-$NR_{6a}R_{6b}$, C(=S)-$NR_{6a}R_{6b}$, S(=O)$_2$-$NR_{6a}P_{6b}$ oder C(=O)-$R_7$ steht.

**10.** Verbindung nach Anspruch 9, in der $R_3$ für Cyano, C(=0) -O-$R_5$, C (=O)-$NR_{6a}R_{6b}$, C (=S)-$NR_{6a}R_{6b}$, S (=O)$_2$-$NR_{6a}R_{6b}$ oder C(=O)-$R_7$ steht.

**11.** Verbindung nach Anspruch 10, in der $R_3$ für C(=O)-O-$R_5$ steht.

**12.** Verbindung nach Anspruch 11, in der $R_3$ für Methoxycarbonyl steht.

**13.** Verbindung nach einem der vorhergehenden Ansprüche, in der $R_1$ für $C_{1-6}$-Alkyl steht.

**14.** Verbindung nach Anspruch 13, in der $R_1$ für Ethyl steht.

**15.** Verbindung nach Anspruch 1, in der $R_1$ für $C_{1-6}$-Alkyl oder $C_{1-6}$-Alkyloxyalkyl steht; $R_2$ für Halogen steht; $R_3$ für Wasserstoff, Cyano, C(=O)-O-$R_5$, C(=O)-$NR_{6a}R_{6b}$ oder C((=O)-$R_7$ steht; Z für ein unter (a-1), (a-2), (a-3), (a-9), (a-10), (a-12), (a-13), (a-14) oder (a-16) ausgewähltes Ringsystem steht; $R_4$ für Wasserstoff steht und n für 2 steht.

**16.** Verbindung nach einem der vorhergehenden Ansprüche, die stereochemisch rein ist.

**17.** Verbindung nach einem der vorhergehenden Ansprüche mit der folgenden Formel

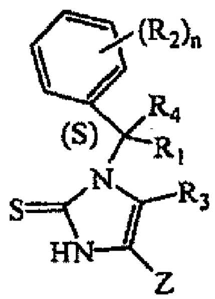

(I')

**18.** Verbindung nach Anspruch 1 mit der folgenden Formel

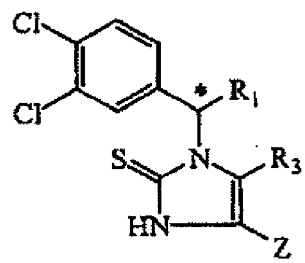

| $R_1$ | $R_3$ | Z | |
|---|---|---|---|
| $-CH_2CH_3$ | 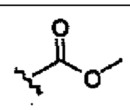 | 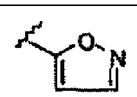 | * (S) |
| $-CH_2CH_3$ | 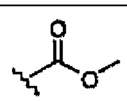 | 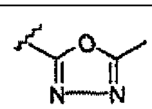 | * (S) |

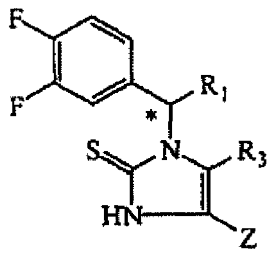

| $R_1$ | $R_3$ | Z | |
|---|---|---|---|
| $-CH_2CH_2CH_3$ | 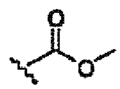 | 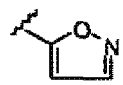 | |
| $-CH_2CH_3$ | 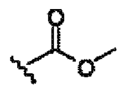 | 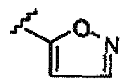 | * (S); m.p. 111,5˚C |
| $-CH_2CH_3$ | 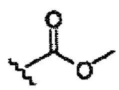 | 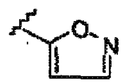 | * (S); m.p. 128,5˚C |

ein N-Oxid, ein pharmazeutisch annehmbares Additionssalz, ein quaternäres Amin oder eine polymorphe Form davon.

**19.** Verbindung nach Anspruch 1, bei der es sich um (S)-3-[1-(3,4-Difluorphenyl)propyl]-5-isoxazol-5-yl-2-thioxo-2,3-dihydro-1*H*-imidazol-4-carbonsäuremethylester, ein N-Oxid, ein pharmazeutisch annehmbares Additionssalz, ein quaternäres Amin oder eine polymorphe Form davon handelt.

**20.** Verbindung nach Anspruch 19, bei der es sich um (S)-3-[1-(3,4-Difluorphenyl)propyl]-5-isoxazol-5-yl-2-thioxo-2,3-dihydro-1*H*-imidazol-4-carbonsäuremethylester oder ein N-Oxid davon handelt.

**21.** Verbindung nach Anspruch 19, bei der es sich um (S)-3-[1-(3,4-Difluorphenyl)propyl]-5-isoxazol-5-yl-2-thioxo-2,3-dihydro-1*H*-imidazol-4-carbonsäuremethylester oder ein pharmazeutisch annehmbares Additionssalz davon handelt.

**22.** Verbindung nach Anspruch 19, bei der es sich um (S)-3-[1-(3,4-Difluorphenyl)propyl]-5-isoxazol-5-yl-2-thioxo-2,3-dihydro-1*H*-imidazol-4-carbonsäuremethylester oder ein quaternäres Amin davon handelt.

**23.** Verbindung nach Anspruch 19, bei der es sich um (S)-3-[1-(3,4-Difluorphenyl)propyl]-5-isoxazol-5-yl-2-thioxo-2,3-dihydro-1*H*-imidazol-4-carbonsäuremethylester handelt.

**24.** Verbindung nach Anspruch 19, bei der es sich um (S)-3-[1-(3,4-Difluorphenyl)propyl]-5-isoxazol-5-yl-2-thioxo-2,3-dihydro-1*H*-imidazol-4-carbonsäuremethylester mit einem Schmelzpunkt von 128,5˚C handelt.

**25.** Verbindung nach einem der vorhergehenden Ansprüche zur Verwendung als Medizin.

**26.** Verwendung einer Verbindung nach einem der Ansprüche 1 bis 24 zur Herstellung eines Arzneimittels zur Prävention oder Behandlung von Krankheiten, die durch Aktivierung des CCR2-Rezeptors vermittelt werden.

**27.** Verwendung nach Anspruch 26, bei der es sich bei der Krankheit um eine Entzündungskrankheit handelt.

**28.** Pharmazeutische Zusammensetzung, enthaltend einen pharmazeutisch annehmbaren Träger und als Wirkstoff eine therapeutisch wirksame Menge einer Verbindung nach einem der Ansprüche 1 bis 24.

**29.** Verfahren zur Herstellung einer Zusammensetzung nach Anspruch 28, **dadurch gekennzeichnet, daß** man einen

pharmazeutisch annehmbaren Träger innig mit einer therapeutisch wirksamen Menge einer Verbindung nach einem der Ansprüche 1 bis 24 vermischt.

**30.** Verfahren zur Herstellung einer Verbindung gemäß Anspruch 1, **dadurch gekennzeichnet, daß** man

a) ein Zwischenprodukt der Formel (II-a) oder (IIb) in Gegenwart einer geeigneten Säure und eines geeigneten Lösungsmittels mit KSCN umsetzt

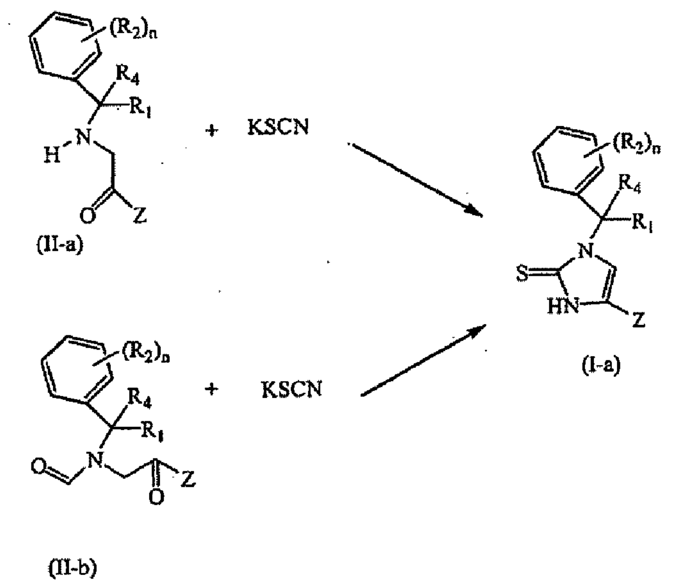

(II-a) (II-b) (I-a)

wobei $R_1$, $R_2$, $R_4$, Z und n die in Anspruch 1 angegebene Bedeutung besitzen;

b) ein Zwischenprodukt der Formel (III) in Gegenwart von KSCN, einer geeigneten Säure, eines geeigneten Lösungsmittels und einer geeigneten Base mit einem Zwischenprodukt der Formel (IV), worin $W_1$ für eine geeignete Abgangsgruppe steht, umsetzt

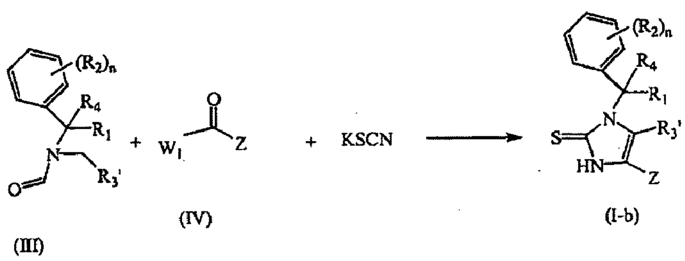

(III) (IV) (I-b)

wobei $R_1$, $R_2$, $R_4$, Z und n die in Anspruch 1 angegebene Bedeutung besitzen und $R_3$, für von Wasserstoff verschiedenes $R_3$ steht;

c) ein Zwischenprodukt der Formel (V) in Gegenwart eines geeigneten Lösungsmittels mit einer geeigneten Base umsetzt

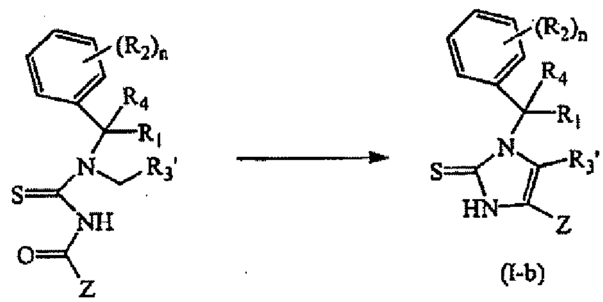

(I-b)

wobei $R_1$, $R_2$, $R_4$, Z und n die in Anspruch 1 angegebene Bedeutung besitzen und $R_{3'}$ für von Wasserstoff verschiedenes $R_3$ steht;

d) ein Zwischenprodukt der Formel (VI) mit Phosphoryltrichlorid oder Burgess-Reagens umsetzt, gegebenenfalls in Gegenwart eines geeigneten Lösungsmittels

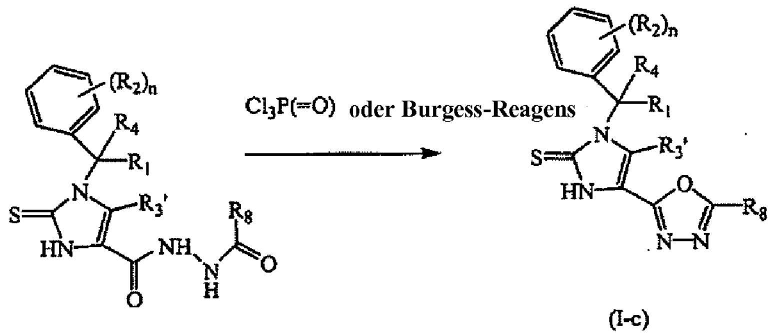


(I-c)

(VI)

wobei $R_1$, $R_2$, $R_3$, $R_4$, $R_8$ und n die in Anspruch 1 angegebene Bedeutung besitzen;

e) ein Zwischenprodukt der Formel (VII), worin $W_2$ für eine geeignete Abgangsgruppe steht, in Gegenwart eines geeigneten Lösungsmittels mit einem entsprechenden Alkohol der Formel HO-$R_{5'}$, worin $R_{5'}$ für $C_{1-6}$-Alkyl oder Hydroxy-$C_{1-6}$-alkyl steht, umsetzt

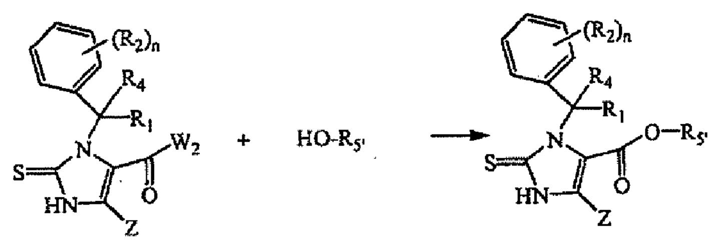

(I-d)

(VII)

wobei $R_1$, $R_2$, $R_4$, Z und n die in Anspruch 1 angegebene Bedeutung besitzen;

f) ein Zwischenprodukt der Formel (VII), worin $W_2$ für eine geeignete Abgangsgruppe steht, in Gegenwart eines geeigneten Lösungsmittels mit einem Zwischenprodukt der Formel (VIII) umsetzt

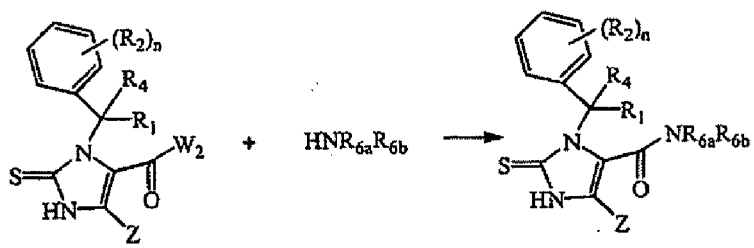

(VII) (VIII) (I-e)

wobei $R_1$, $R_2$, $R_4$, $R_{6a}$, $R_{6b}$, Z und n die in Anspruch 1 angegebene Bedeutung besitzen;
g) ein Zwischenprodukt der Formel (VII) in Gegenwart eines geeigneten Lösungsmittels mit einem geeigneten Reduktionsmittel umsetzt

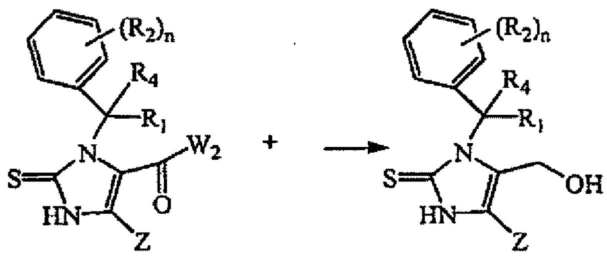

(VII) (I-f)

wobei $R_1$, $R_2$, $R_4$, Z und n die in Anspruch 1 angegebene Bedeutung besitzen;

oder gegebenenfalls Verbindungen der Formel (I) durch an sich bekannte Transformationen ineinander umwandelt und weiterhin gegebenenfalls die Verbindungen der Formel (I) durch Behandlung mit einer Säure in ein therapeutisch wirksames nichttoxisches Säureadditionssalz oder durch Behandlung mit einer Base in ein therapeutisch wirksames nichttoxisches Basenadditionssalz umwandelt oder umgekehrt die Säureadditionssalzform durch Behandlung mit Alkali in die freie Base oder das Basenadditionssalz durch Behandlung mit Säure in die freie Säure umwandelt und gegebenenfalls stereochemisch isomere Formen, quaternäre Amine oder N-Oxidformen davon herstellt.

**31.** Verfahren zur Herstellung einer Verbindung gemäß Anspruch 17, **dadurch gekennzeichnet, daß** man die Reaktionen nach Anspruch 30 ausgehend von einem Zwischenprodukt, in dem das den $R_1$- und $R_4$-Substituenten tragende Kohlenstoffatom die (S)-Konfiguration aufweist, durchführt.

**32.** Verbindung der Formel (IX-b-1-1)

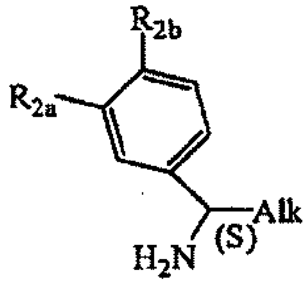

(IX-b-1-1)

ein N-Oxid, ein pharmazeutisch annehmbares Additionssalz oder ein quaternäres Amin davon, wobei Alk für Methyl, Ethyl oder n-Propyl steht und $R_{2a}$ und $R_{2b}$ jeweils unabhängig voneinander für Chlor oder Fluor stehen, mit der Maßgabe, daß Alk nicht für Ethyl steht, wenn $R_{2a}$ und $R_{2b}$ beide für Chlor stehen.

**33.** Verbindung nach Anspruch 32, bei der es sich um

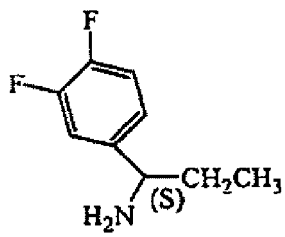

(IX-b-1-1-a)

handelt.

**34.** Verbindung mit der folgenden Formel

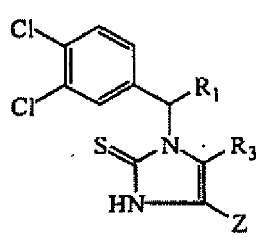

| R1 | R3 | Z |
|---|---|---|
| $-CH_2CH_3$ | 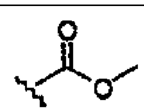 | 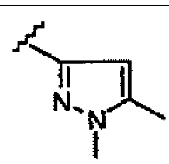 |

(fortgesetzt)

| R1 | R3 | Z |
|---|---|---|
| $-CH_2CH_3$ | 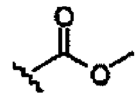 | 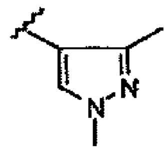 |

**Revendications**

1. Composé de formule (I)

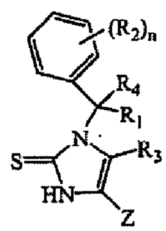

(I),

un *N*-oxyde, un sel d'addition pharmaceutiquement acceptable, une amine quaternaire, une forme polymorphe ou une forme stéréochimiquement isomère de celui-ci, dans laquelle

$R_1$ représente $C_{1-6}$-alkyle ou $C_{1-6}$-alkyloxy-$C_{1-6}$-alkyle ;
chaque $R_2$ représente indépendamment halogéno ;
$R_3$ représente hydrogène, cyano, $C_{1-6}$-alkyle éventuellement substitué par hydroxy ou $C_{1-6}$-alkyloxy, C(=O)-O-$R_5$, C(=O)-$NR_{6a}R_{6b}$, C(=S)-$NR_{6a}R_{6b}$, $S(=O)_2$-$NR_{6a}R_{6b}$ ou C(=O)-$R_7$ ;
$R_4$ représente hydrogène ;
$R_5$ représente hydrogène, $C_{1-6}$-alkyle, hydroxy-$C_{1-6}$-alkyle, $C_{2-6}$-alcényle, $C_{2-6}$-alcynyle, polyhalogéno-$C_{1-6}$-alkyle, $C_{1-6}$-alkyloxy-$C_{1-6}$-alkyle, amino-$C_{1-6}$-alkyle, mono- ou di ($C_{1-4}$-alkyl) amino-$C_{1-6}$-alkyle, aminocarbonyl-$C_{1-6}$-alkyle, mono- ou di($C_{1-4}$-alkyl)aminocarbonyl-$C_{1-6}$-alkyle ou aryle ;
$R_{6a}$ et $R_{6b}$ représentent chacun indépendamment hydrogène, $C_{1-6}$-alkyle, amino, mono- ou di($C_{1-4}$-alkyl)amino, arylNH-, amino-$C_{1-6}$-alkyle, mono- ou di($C_{1-4}$-alkyl)amino-$C_{1-6}$-alkyle, $C_{1-6}$-alkylcarbonylamino, aminocarbonylamino, $C_{1-6}$-alkyloxy, carbonylamino ou hydroxy-$C_{1-6}$-alkyle ; ou $R_{6a}$ et $R_{6b}$ pris ensemble avec l'azote auquel ils sont liés forment pyrrolidinyle, imidazolidinyle, pyrazolidinyle, pipéridinyle, pipérazinyle, morpholinyle, thiomorpholinyle ou pipérazinyle, substitué par $C_{1-6}$-alkyle ;
$R_7$ représente hydrogène, $C_{1-6}$-alkyle, hydroxy-$C_{1-6}$-alkyle, $C_{2-6}$-alcényle, $C_{2-6}$-alcynyle, polyhalogéno-$C_{1-6}$-alkyle, $C_{1-6}$-alkyloxy-$C_{1-6}$-alkyle, amino-$C_{1-6}$-alkyle, mono- ou di ($C_{1-4}$-alkyl) amino-$C_{1-6}$-alkyle, aminocarbonyl-$C_{1-6}$-alkyle, mono- ou di($C_{1-4}$-alkyl)aminocarbonyl-$C_{1-6}$-alkyle, aryle ou hétéroaryle ;
Z représente un système de cycles choisi parmi

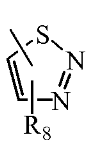
(a-1)

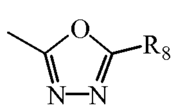
(a-2)

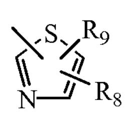
(a-3)

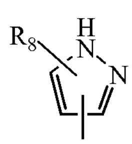
(a-4)

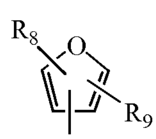
(a-5)

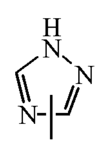
(a-6)

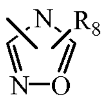
(a-7)

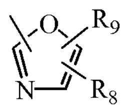
(a-8)

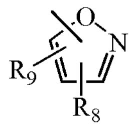
(a-9)

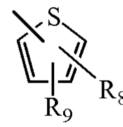
(a-10)

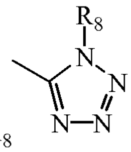
(a-11)

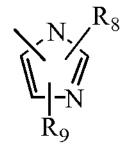
(a-12)

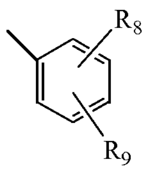
(a-13)

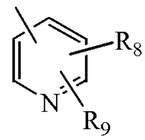
(a-14)

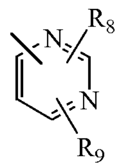
(a-15)

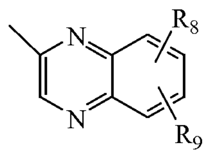
(a-16)

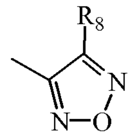
(a-17)

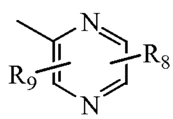
(a-18)

;

chaque $R_8$ représente indépendamment hydrogène, halogéno, $C_{1-6}$-alkyle, $C_{1-6}$-alkyloxy, polyhalogéno-$C_{1-6}$-alkyle, polyhalogéno-$C_{1-6}$-alkyloxy, cyano, aminocarbonyle, mono- ou di($C_{1-4}$-alkyl)aminocarbonyle, amino, mono- ou di($C_{1-4}$alkyl)amino, hydroxy-$C_{1-6}$-alkylamino, aryle, aryloxy, pipéridinyle, pipéridinylamino, morpholinyle, pipérazinyle ou nitro ;

chaque $R_9$ représente indépendamment hydrogène, halogéno ou $C_{1-6}$-alkyle ;

n est 1, 2, 3, 4 ou 5 ;

Aryle représente phényle ou phényle substitué par un, deux, trois, quatre ou cinq substituants choisis chacun indépendamment parmi halogéno, $C_{1-6}$-alkyle, $C_{1-6}$-alkyloxy, polyhalogéno-$C_{1-6}$-alkyle, polyhalogéno-$C_{1-6}$-alkyloxy, cyano, aminocarbonyle, mono- ou di($C_{1-4}$-alkyl)aminocarbonyle, amino, mono- ou di($C_{1-4}$-alkyl)amino, phényloxy ou nitro ;

hétéroaryle représente furanyle, thiényle, pyrrolyle, oxazolyle, thiazolyle, imidazolyle, pyrazolyle, isoxazolyle, isothiazolyle, oxadiazolyle, triazolyle, thiadiazolyle, pyridyle, pyridazinyle, pyrimidinyle ou pyrazinyle, chacun desdits hétérocycles étant éventuellement substitué par un ou deux substituants choisis chacun indépendamment parmi halogéno, $C_{1-6}$-alkyle, $C_{1-6}$-alkyloxy, polyhalogéno-$C_{1-6}$-alkyle, polyhalogéno-$C_{1-6}$-alkyloxy, cyano, aminocarbonyle, mono- ou di($C_{1-4}$-alkyl)aminocarbonyle, amino, mono- ou di($C_{1-4}$-alkyl)amino ou nitro.

2. Composé selon la revendication 1, **caractérisé en ce que** $R_2$ représente fluoro.

3. Composé selon la revendication 1 ou 2, **caractérisé en ce que** n est 2 ou 3.

4. Composé selon la revendication 3, **caractérisé en ce que** n est 2.

5. Composé selon la revendication 4, **caractérisé en ce que** n est 2 et lesdits deux substituants $R^2$ sont placés en position méta et para.

6. Composé selon l'une quelconque des revendications précédentes, **caractérisé en que** Z représente un radical de formule (a-1), (a-2), (a-3), (a-9), (a-10), (a-12), (a-13), (a-14) ou (a-16).

**7.** Composé selon la revendication 6, **caractérisé en ce que** Z représente un radical de formule (a-1), (a-2), (a-9), (a-10) ou (a-13).

**8.** Composé selon la revendication 7, **caractérisé en ce que** Z représente un radical de formule (a-9).

**9.** Composé selon l'une quelconque des revendications précédentes, **caractérisé en ce que** $R_3$ représente hydrogène, cyano, C(=O)-O-$R_5$, C(=O)-$NR_{6a}R_{6b}$, C(=S)-$NR_{6a}R_{6b}$, S (=O)$_2$-$NR_{6a}R_{6b}$ ou C (=O)-$R_7$.

**10.** Composé selon la revendication 9, **caractérisé en que** $R_3$ représente cyano, C(=O)-O-$R_5$, C(=O)-$NR_{6a}R_{6b}$, C (=S)-$NR_{6a}R_{6b}$, S(=O)$_2$-$NR_{6a}R_{6b}$ ou C (=O)-$R_7$.

**11.** Composé selon la revendication 10, **caractérisé en ce que** $R_3$ représente C(=O)-O-$R_5$.

**12.** Composé selon la revendication 11, **caractérisé en ce que** $R_3$ représente méthoxycarbonyle.

**13.** Composé selon l'une quelconque des revendications précédentes, **caractérisé en ce que** $R_1$ représente $C_{1-6}$-alkyle.

**14.** Composé selon la revendication 13, **caractérisé en ce que** $R_1$ représente éthyle.

**15.** Composé selon la revendication 1, **caractérisé en ce que** $R_1$ représente $C_{1-6}$-alkyle ou $C_{1-6}$-alkyloxyalkyle ; $R_2$ représente halogéno ; $R_3$ représente hydrogène, cyano, C(=O)-O-$R_5$, C(=O)-$NR_{6a}R_{6b}$ ou C(=O)-$R_7$ ; Z représente un système de cycles choisi parmi (a-1), (a-2), (a-3), (a-9), (a-10), (a-12), (a-13), (a-14) ou (a-16) ; $R_4$ représente hydrogène et n est 2.

**16.** Composé selon l'une quelconque des revendications précédentes, **caractérisé en ce que** le composé est stéréochimiquement pur.

**17.** Composé selon l'une quelconque des revendications précédentes, **caractérisé en ce que** le composé a la formule suivante

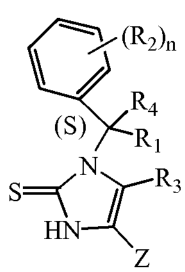

(I')

**18.** Composé selon la revendication 1, **caractérisé en ce que** le composé a la formule suivante

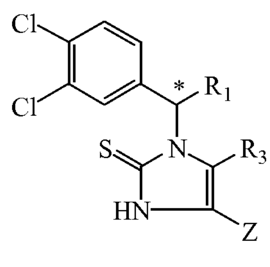

| $R_1$ | $R_3$ | Z | |
|---|---|---|---|
| $-CH_2CH_3$ | 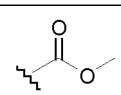 | 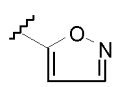 | * (S) |

(suite)

| $R_1$ | $R_3$ | Z | |
|---|---|---|---|
| $-CH_2CH_3$ | 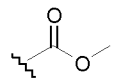 | 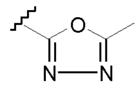 | * (S) |

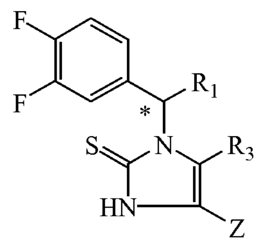

| $R_1$ | $R_3$ | Z | |
|---|---|---|---|
| $-CH_2CH_2CH_3$ | 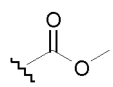 | 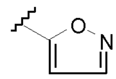 | |
| $-CH_2CH_3$ | 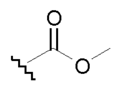 | 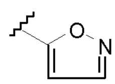 | * (S); F 111,5˚C |
| $-CH_2CH_3$ | 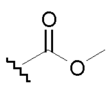 | 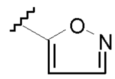 | * (S); F 128,5˚C |

un N-oxyde, un sel d'addition pharmaceutiquement acceptable, une amine quaternaire ou une forme polymorphe de celui-ci.

**19.** Composé selon la revendication 1, **caractérisé en ce que** le composé est l'ester méthylique d'acide (S)-3-[1-(3,4-difluorophényl)propyl]-5-isoxazol-5-yl-2-thioxo-2,3-dihydro-1*H*-imidazol-4-carboxylique, un N-oxyde, un sel d'addition pharmaceutiquement acceptable, une amine quaternaire ou une forme polymorphe de celui-ci.

**20.** Composé selon la revendication 19, **caractérisé en ce que** le composé est l'ester méthylique d'acide (S)-3-[1-(3,4-difluorophényl)propyl]-5-isoxazol-5-yl-2-thioxo-2,3-dihydro-1*H*-imidazol-4-carboxylique ou un N-oxyde de celui-ci.

**21.** Composé selon la revendication 19, **caractérisé en ce que** le composé est l'ester méthylique d'acide (S)-3-[1-(3,4-difluorophényl)propyl]-5-isoxazol-5-yl-2-thioxo-2,3-dihydro-1*H*-imidazol-4-carboxylique ou un sel d'addition pharmaceutiquement acceptable de celui-ci.

**22.** Composé selon la revendication 19, **caractérisé en ce que** le composé est l'ester méthylique d'acide (S)-3-[1-(3,4-difluorophényl)propyl]-5-isoxazol-5-yl-2-thioxo-2,3-dihydro-1*H*-imidazol-4-carboxylique ou une amine quaternaire de celui-ci.

**23.** Composé selon la revendication 19, **caractérisé en ce que** le composé est l'ester méthylique d'acide (S)-3-[1-(3,4-difluorophényl)propyl]-5-isoxazol-5-yl-2-thioxo-2,3-dihydro-1*H*-imidazol-4-carboxylique.

**24.** Composé selon la revendication 19, **caractérisé en ce que** le composé est l'ester méthylique d'acide (S)-3-[1-(3,4-difluorophényl)propyl]-5-isoxazol-5-yl-2-thioxo-2,3-dihydro-1*H*-imidazol-4-carboxylique ayant un point de fusion de 128,5˚ C.

**25.** Composé selon l'une quelconque des revendications précédentes, destiné à être utilisé en médecine.

**26.** Utilisation d'un composé selon l'une quelconque des revendications 1 à 24, pour la fabrication d'un médicament

destiné à la prévention ou au traitement de maladies médiées par l'activation du récepteur CCR2.

**27.** Utilisation selon la revendication 26, **caractérisée en ce que** la maladie est une maladie inflammatoire.

**28.** Composition pharmaceutique comprenant un support pharmaceutiquement acceptable et, à titre de principe actif, une quantité thérapeutiquement efficace d'un composé selon l'une quelconque des revendications 1 à 24.

**29.** Procédé de préparation d'une composition selon la revendication 28, **caractérisé en ce qu'** un support pharmaceutiquement acceptable est mélangé intimement avec une quantité thérapeutiquement efficace d'un composé selon l'une quelconque des revendications 1 à 24.

**30.** Procédé de préparation d'un composé tel que défini dans la revendication 1, **caractérisé par**

a) la réaction d'un intermédiaire de formule (II-a) ou (II-b) avec du KSCN en présence d'un acide convenable et d'un solvant convenable,

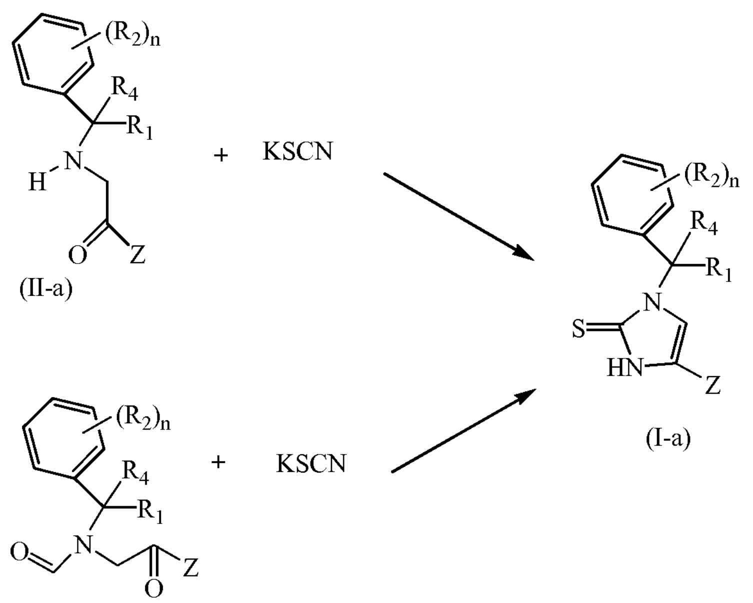


(II-a)

(I-a)

(II-b)

$R_1$, $R_2$, $R_4$, Z et n étant tels que définis dans la revendication 1 ;
b) la réaction d'un intermédiaire de formule (III) avec un intermédiaire de formule (IV) dans laquelle $W_1$ représente un groupement partant convenable, en présence de KSCN, d'un acide convenable, d'un solvant convenable, et d'une base convenable,

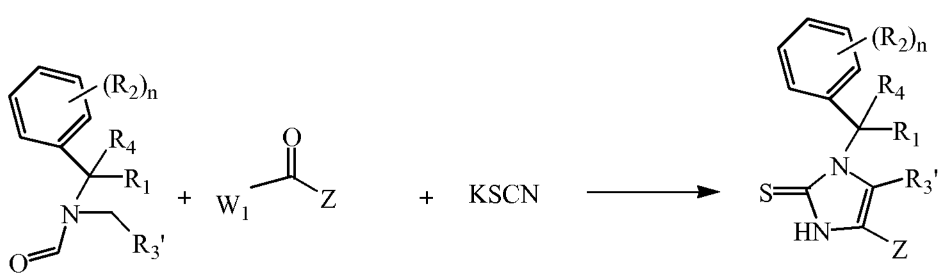


(IV)

(I-b)

(III)

$R_1$, $R_2$, $R_4$, Z et n étant tels que définis dans la revendication 1 et $R_3$, représentant $R_3$ autre que l'hydrogène ; c) la réaction d'un intermédiaire de formule (V) avec une base convenable en présence d'un solvant convenable

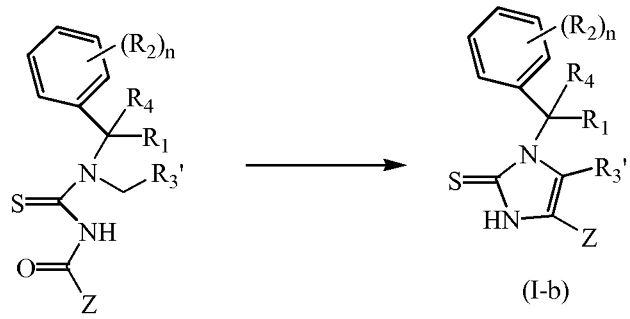

(I-b)

(V)

$R_1$, $R_2$, $R_4$, Z et n étant tels que définis dans la revendication 1 et $R_3$, représentant $R_3$ autre que l'hydrogène ; d) la réaction d'un intermédiaire de formule (VI) avec du trichlorure phosphorique ou du réactif de Burgess éventuellement en présence d'un solvant convenable

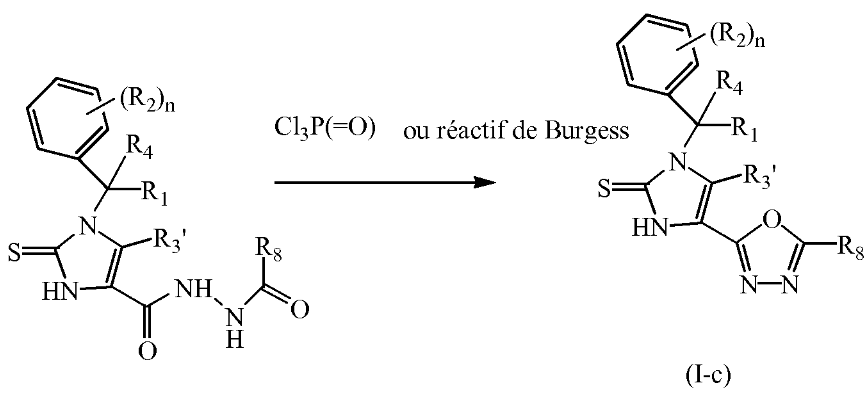


(I-c)

(VI)

$R_1$, $R_2$, $R_3$, $R_4$, $R_8$ et n étant tels que définis dans la revendication 1 ;
e) la réaction d'un intermédiaire de formule (VII), dans laquelle $W_2$ représente un groupement partant convenable, avec un alcool approprié de formule HO-$R_{5'}$, dans laquelle $R_{5'}$ représente $C_{1-6}$-alkyle ou hydroxy-$C_{1-6}$-alkyle en présence d'un solvant convenable

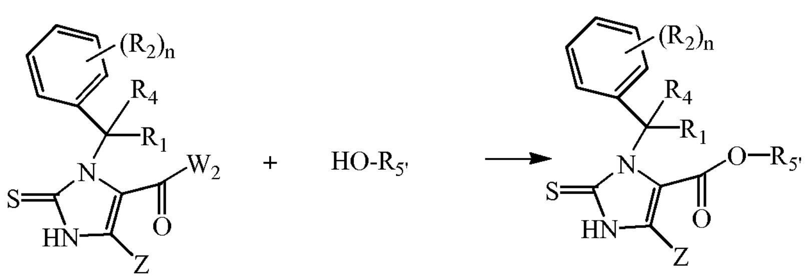

(I-d)

(VII)

$R_1$, $R_2$, $R_4$, Z et n étant tels que définis dans la revendication 1 ;
f) la réaction d'un intermédiaire de formule (VII), dans laquelle $W_2$ représente un groupement partant convenable, avec un intermédiaire de formule (VIII) en présence d'un solvant convenable

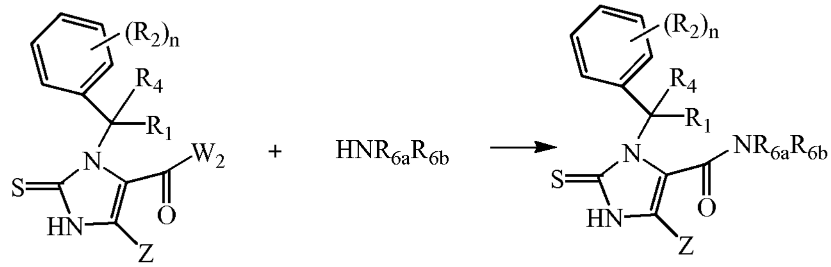

(VII) (VIII) (I-e)

$R_1$, $R_2$, $R_4$, $R_{6a}$, $R_{6b}$, Z et n étant tels que définis dans la revendication 1 ;

g) la réaction d'un intermédiaire de formule (VII) avec un agent de réduction convenable en présence d'un solvant convenable

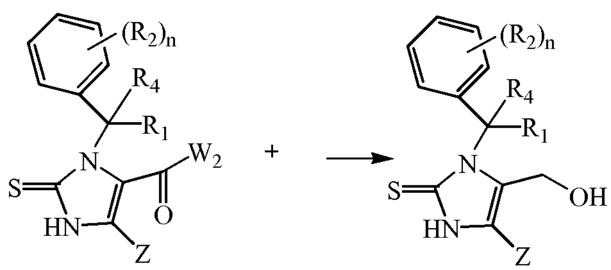

(VII) (I-f)

$R_1$, $R_2$, $R_4$, Z et n étant tels que définis dans la revendication 1 ;

ou, si on le souhaite, la transformation des composés de formule (I) les uns en les autres en suivant des transformations connues dans l'art, et en outre, si on le souhaite, la transformation des composés de formule (I) en un sel d'addition d'acide thérapeutiquement actif non toxique par traitement avec un acide, ou en un sel d'addition de base thérapeutiquement actif non toxique par traitement avec une base, ou inversement, la transformation de la forme sel d'addition d'acide en la base libre par traitement avec un alcali, ou la transformation du sel d'addition de base en l'acide libre par traitement avec un acide ; et, si on le souhaite, la préparation de formes stéréochimiquement isomères, d'amines quaternaires, ou de formes N-oxydes de ceux-ci.

**31.** Procédé de préparation d'un composé tel que défini dans la revendication 17, **caractérisé par** la mise en oeuvre des réactions selon la revendication 30 en partant d'un intermédiaire dans lequel l'atome de carbone portant les substituants $R_1$ et $R_4$ a la configuration (S).

**32.** Composé de formule (IX-b-1-1)

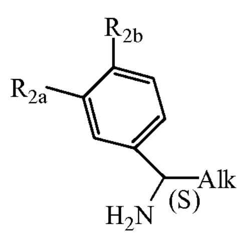

(IX-b-1-1)

un N-oxyde, un sel d'addition pharmaceutiquement acceptable ou une amine quaternaire de celui-ci, dans laquelle Alk représente méthyle, éthyle ou n-propyle, et chaque $R_{2a}$ et $R_{2b}$ représente indépendamment chloro ou fluoro à

condition que lorsque $R_{2a}$ et $R_{2b}$ sont tous deux chloro, alors Alk soit autre qu' éthyle.

**33.** Composé selon la revendication 32, **caractérisé en ce que** le composé est

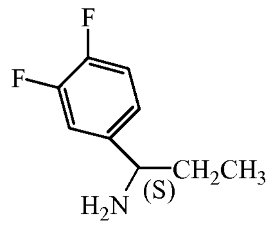

(IX-b-1-1-a)

**34.** Composé ayant la formule suivante

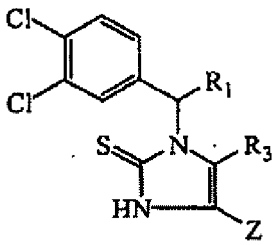

| $R_1$ | $R_3$ | Z |
|---|---|---|
| $-CH_2CH_3$ | 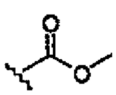 | 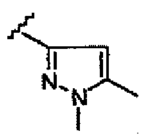 |
| $-CH_2CH_3$ | 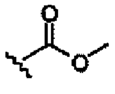 | 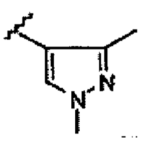 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 02066458 A
- FR 1487326
- FR 6751 M
- US 3850944 A
- WO 2004069809 A
- WO 2004069810 A


### Non-patent literature cited in the description


- *Bull. Soc. Chim.Belg.,* 1964, vol. 73, 181-188
- *Archiv der Pharmazie,* 1972, vol. 305 (12), 891-901
- *Annales pharmaceutiques francaises,* 1971, vol. 29 (1), 63-70
- **CHARO et al.** *PNAS,* 1994
- **CHENG ; PRUSOFF.** *Biochem. Pharmacol.,* 1973, vol. 22, 3099-3108